# EUROPEAN PATENT SPECIFICATION

(11) **EP 4 438 584 B1**
(45) Date of publication and mention of the grant of the patent: **12.11.2025**
(21) Application number: 23165042.5
(22) Date of filing: 29.03.2023
(51) Int. Cl.: C07C 13/00, H10K 50/17, C07D 221/00, C07B 59/00, C07D 213/26, C07D 213/84, C07D 213/85, C07D 239/26, C07D 239/28, C07D 401/10, C07D 403/10

(54) **COMPOUND OF FORMULA (I) AND THEIR USE IN AN ORGANIC ELECTRONIC DEVICE**
VERBINDUNG DER FORMEL (I) UND DEREN VERWENDUNG IN EINER ORGANISCHEN ELEKTRONISCHEN VORRICHTUNG
COMPOSÉ DE FORMULE (I) ET SON UTILISATION DANS UN DISPOSITIF ÉLECTRONIQUE ORGANIQUE

(43) Date of publication of application: 02.10.2024
(73) Proprietor: Novaled GmbH, 01099 Dresden (DE)
(72) Inventor: LUSCHTINETZ, Regina, 01099 Dresden (DE); NÜLLEN, Max Peter, 01099 Dresden (DE); SCHULZE, Benjamin, 01099 Dresden (DE); WUDARCZYK, Jacob Jacek, 01099 Dresden (DE)
(74) Representative: Michalski Hüttermann & Partner Patentanwälte mbB

(56) References cited:
- CN-A- 110 372 540
- CN-A- 114 516 821
- US-A1- 2015 155 490

## Description

### Technical Field

The present invention relates to a compound of formula (I) and their use in organic electronic devices, an organic semiconductor layer comprising the compound of formula (I), an organic electronic device comprising the organic semiconductor layer, and a display device comprising the organic electronic device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

There remains a need to improve performance of organic semiconductor materials, semiconductor layers, as well as organic electronic devices thereof, in particular to achieve improved operating voltage, improved efficiency, improved lifetime and/or improved voltage stability over time through improving the characteristics of the compounds comprised therein. In addition, there remains a need to improve LUMO energy, improve the dipole moment and/or improve the thermal properties compared to comparative examples, in particular to improve thermal stability and/or processing properties at elevated temperatures.

Compounds having structures related to those of the invention are known from CN 110 372 540 A and US 2015/155490 A1.

### DISCLOSURE

An aspect of the present invention provides a compound of formula (I): 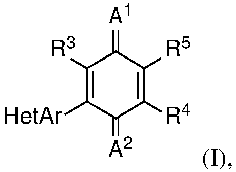 wherein
HetAr is selected from formula (II): 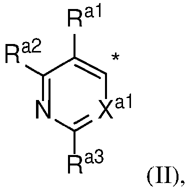
A¹ is selected from formula (III): 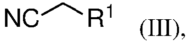
A² is selected from formula (IV) 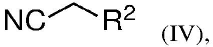
wherein the asterisk "*" denotes the binding position,
   - R¹ and R²: are independently selected from CN, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, SF₅,
   wherein the one or more substituents on R¹ and R² are independently selected from D, partially fluorinated C₁ to C₈ alkyl, perfluorinated C₁ to C₈ alkyl, CF₃, halogen, F, SF₅ or CN;
   - R³ and R⁴: are independently selected from H, D, halogen, F, CN, partially fluorinated C₁ to C₄ alkyl, perfluorinated C₁ to C₄ alkyl, SF₅ or CF₃;
   - R⁵: is independently selected from H, D, halogen, F, CN, partially fluorinated C₁ to C₈ alkyl, perfluorinated C₁ to C₈ alkyl, CF₃, substituted or unsubstituted C₆ to C₃₀ aryl or substituted or unsubstituted C₂ to C₃₀ heteroaryl, SF₅,
   wherein the one or more substituents on R⁵ are independently selected from D, partially fluorinated C₁ to C₈ alkyl or perfluorinated C₁ to C₈ alkyl, CF₃, halogen, F, CN, SF₅;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN, SF₅,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN, SF₅;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, CN or SF₅.

It should be noted that throughout the application and the claims any R¹, R², R³, R⁴, R⁵, R^{a1}, R^{a2}, R^{a3} and R^{a4}, etc. always refer to the same moieties, unless otherwise noted.

In the present specification, when a definition is not otherwise provided, "partially fluorinated" refers to a C₁ to C₈ alkyl group in which only part of the hydrogen atoms are replaced by fluorine atoms.

In the present specification, when a definition is not otherwise provided, "perfluorinated" refers to a C₁ to C₈ alkyl group in which all hydrogen atoms are replaced by fluorine atoms.

In the present specification, when a definition is not otherwise provided, "substituted" refers to one substituted with a deuterium, D, halogen, F, CN, partially fluorinated C₁ to C₄ alkyl, perfluorinated C₁ to C₄ alkyl, or CF.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₈ alkyl group. More specifically, the alkyl group may be a C₁ to C₆ alkyl group or a C₁ to C₄ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms may be selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms.

The term "six-member ring" is understood to mean a ring formed by 6 atoms. The ring-forming atoms of the "six-member ring" may be bonded to further atoms outside the ring, for example hydrogen atoms.

The term "five-member ring" is understood to mean a ring formed by 5 atoms. The ring-forming atoms of the "five-member ring "may be bonded to further atoms outside the ring, for example hydrogen atoms.

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "p-type charge generation layer", "p-CGL" and "hole generation layer" are used synonymously.

The terms "n-type charge generation layer", "n-CGL" and "electron generation layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The asterisk "*" denotes the binding position.

### Advantageous Effects

Surprisingly, it was found that the organic compound of the present invention according to formula (I) solves the problem underlying the present invention by enabling devices in various aspects superior over the organic electroluminescent devices known in the art, with respect to operating voltage, efficiency and/or lifetime, and in particular with respect to operating voltage, efficiency, and voltage stability over time. It has been further surprisingly found that compounds of formula (I) may have improved LUMO energy, improved dipole moment and/or improved thermal properties compared to comparative examples, in particular improved thermal stability and/or processing properties at elevated temperatures.

The compounds of formula (I) exhibiting a good thermal property such a evaporation temperature for the good manufacturing of an OLED device, and when it is used as a p-dopant in a OLED device, it exhibits a good performance such as a low operational voltage, a low increase of the operational over time, and efficiency and a high life time. Moreover, the compounds of formula (I) exhibiting a low mass loss during heating in order to avoid tool contamination during the manufacturing of an organic electronic device.

The thermal stability of compounds according to formula (I), as determined by TGA5%, and volatility, as determined by the rate onset temperature, may be in the range required for mass production.

Surprisingly, it was found that the LUMO energy may be in the range required for efficient hole injection and/or hole generation.

### Compound of formula (I)

According to one embodiment of the present invention, the compound of formula (I) is represented by: 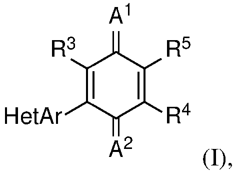 wherein
HetAr is selected from formula (II): 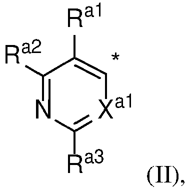
A¹ is selected from formula (III): 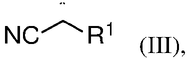
A² is selected from formula (IV) 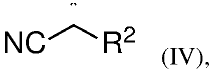
wherein the asterisk "*" denotes the binding position,
   - R¹ and R²: are independently selected from CN, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl,
   wherein the one or more substituents on R¹ and R² are independently selected from D, partially fluorinated C₁ to C₈ alkyl, perfluorinated C₁ to C₈ alkyl, CF₃, halogen, F, or CN;
   - R³ and R⁴: are independently selected from H, D, halogen, F, CN, partially fluorinated C₁ to C₄ alkyl, perfluorinated C₁ to C₄ alkyl, or CF₃;
   - R⁵: is independently selected from H, D, halogen, F, CN, partially fluorinated C₁ to C₈ alkyl, perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, halogen, F, CN;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN, SF₅,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN, SF₅;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, CN, or SF₅.

According to one embodiment, wherein for the compounds of formula (I) a SF₅ group may be excluded.

According to one embodiment, wherein compounds of formula (I) comprising more than one pyridin group may be excluded.

According to one embodiment, wherein compounds of formula (I) comprising more than two pyridin groups may be excluded.

According to one embodiment, wherein compounds of formula (I) comprising more than one aromatic diazine group are excluded.

According to one embodiment, wherein compounds of formula (I) comprising more than one aromatic triazine group may be excluded.

According to one embodiment, wherein compounds of formula (I) comprising more than one aromatic diazine group, comprising an aromatic triazine group and comprising more than one pyridin group, may be excluded.

According to one embodiment, wherein R^{a2} and R^{a3} of compounds of formula (I) is not selected CN.

According to one embodiment, wherein R^{a2} of compounds of formula (I) is not selected CN.

According to one embodiment of the present invention, the compound of formula (I) is represented by: 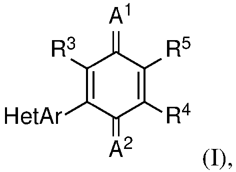 wherein
HetAr is selected from formula (II): 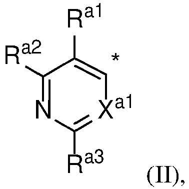
A¹ is selected from formula (III): 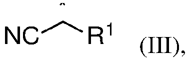
A² is selected from formula (IV) 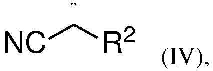
wherein the asterisk "*" denotes the binding position,
   - R¹ and R²: are independently selected from from CN, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl,
   wherein the one or more substituents on R¹ and R² are independently selected from D, partially fluorinated C₁ to C₄ alkyl, perfluorinated C₁ to C₄ alkyl, CF₃, or CN;
   - R³ and R⁴: are independently selected from H, D, CN, CF₃;
   - R⁵: is independently selected from H, D, CN, substituted or unsubstituted C₆ aryl or substituted or unsubstituted C₄ to C₅ heteroaryl,
   wherein the one or more substituents on Rindependently selected from D, partially fluorinated C₁ to C₈ alkyl or perfluorinated C₁ to C₈ alkyl, CF₃, halogen, F, CN;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, or CN; and
optional R^{a2} and R^{a3} of compounds of formula (I) is not selected CN.

According to one embodiment of the present invention, the compound of formula (I) is represented by: 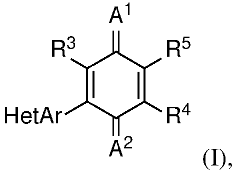 wherein
HetAr is selected from formula (II): 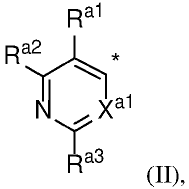
A¹ is selected from formula (III): 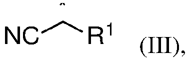
A² is selected from formula (IV) 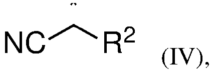
wherein the asterisk "*" denotes the binding position,
   - R¹ and R²: are independently selected from from CN, partially fluorinated C₁ to C₄ alkyl, perfluorinated C₁ to C₄ alkyl, CF₃, substituted or unsubstituted C₆ aryl, substituted or unsubstituted C₄ to C₅ heteroaryl,
   wherein the one or more substituents on R¹ and R² are independently selected from D, partially fluorinated C₁ to C₄ alkyl, perfluorinated C₁ to C₄ alkyl, CF₃, or CN;
   - R³ and R⁴: are independently selected from H, D;
   - R⁵: is independently selected from substituted or unsubstituted C₆ aryl or substituted or unsubstituted C₄ to C₅ heteroaryl,
   wherein the one or more substituents on Rindependently selected from D, partially fluorinated C₁ to C₈ alkyl or perfluorinated C₁ to C₈ alkyl, CF₃, halogen, F, CN;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, or CN; and
optional R^{a2} and R^{a3} of compounds of formula (I) is not selected CN.

According to one embodiment, there is provided a compound of formula (I): 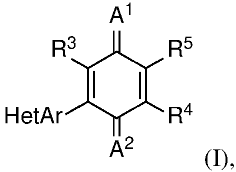 wherein
HetAr is selected from formula (II): 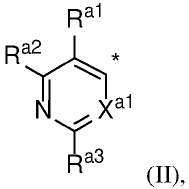
A¹ is selected from formula (III): 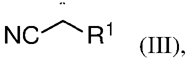
A² is selected from formula (IV) 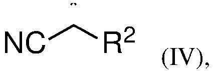
wherein the asterisk "*" denotes the binding position,
   - R¹ and R²: are independently selected from CN, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl,
   wherein the one or more substituents on R¹ and R² are independently selected from D, partially fluorinated C₁ to C₈ alkyl, perfluorinated C₁ to C₈ alkyl, CF₃, halogen, F, or CN;
   - R³ and R⁴: are independently selected from H, D, halogen, F, CN, partially fluorinated C₁ to C₄ alkyl, perfluorinated C₁ to C₄ alkyl, or CF₃;
   - R⁵: is independently selected from H, D, halogen, F, CN, partially fluorinated C₁ to C₈ alkyl, perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, halogen, F, CN;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, or CN; and wherein HetAr and R⁵ is selected the same.

According to one embodiment of formula (I), wherein a CN group is not bound to an aromatic carbon atom when said aromatic carbon is directly bound to a sp2-hybridized ring nitrogen atom.

According to one embodiment, wherein the compound of formula (I) is represented: 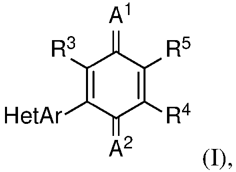 wherein
HetAr is selected from formula (II): 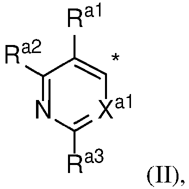
A¹ is selected from formula (III): 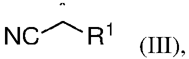
A² is selected from formula (IV) 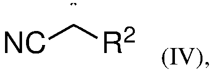
wherein the asterisk "*" denotes the binding position,
   - R¹ and R²: are independently selected from CN, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl,
   wherein the one or more substituents on R¹ and R² are independently selected from D, partially fluorinated C₁ to C₈ alkyl, perfluorinated C₁ to C₈ alkyl, CF₃, halogen, F, or CN;
   - R³ and R⁴: are independently selected from H, D, halogen, F, CN, partially fluorinated C₁ to C₄ alkyl, perfluorinated C₁ to C₄ alkyl, or CF₃;
   - R⁵: is independently selected from H, D, halogen, F, CN, partially fluorinated C₁ to C₈ alkyl, perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, halogen, F, CN;
   - X^{a1}: is selected from N or CR^{a4};
   - R^{a1}, R^{a2}, R^{a3} and R^{a4}: are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN;

   wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, or CN; and
   wherein a CN group is not bound to an aromatic carbon atom when said aromatic carbon is directly bound to a sp2-hybridized ring nitrogen atom; and optional R^{a2} is not selected from CN.

According to one embodiment of the present invention, the compound of formula (I) comprises less than nine CN groups, preferably less than eight CN groups, further preferred more than one CN group and less than 6 CN groups, more than two CN group and less than 5 CN groups.

According to one embodiment of the present invention, the compound of formula (I) comprises ≥ 6 F and ≤ 24 F groups, preferably ≥ 8 F and ≤ 18 F atoms.

According to one embodiment of the present invention, the calculated LUMO of the compound of Formula (I) is in the range of ≤ -5.00 eV to ≥ -5.75 eV, when calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). by applying the hybrid functional B3LYP with a 6-31G* basis set in the gas phase, preferably ≤ -5.05 eV to ≥ -5.75 eV; even more preferred ≤ -5.10 eV to ≥ -5.70 eV, and most preferred ≤ -5.15 eV to ≥ -5.70 eV.

### R¹ and R²

According to an embodiment, wherein R¹ and R² may be independently selected from CN, partially fluorinated C₁ to C₄ alkyl or perfluorinated C₁ to C₄ alkyl, CF₃, substituted or unsubstituted C₆ to C₂₄ aryl, substituted or unsubstituted C₃ to C₂₄ heteroaryl.

According to an embodiment, wherein R¹ and R² may be independently selected from CN, partially fluorinated C₁ to C₄ alkyl or perfluorinated C₁ to C₄ alkyl, CF₃, substituted or unsubstituted C₆ to C₁₈ aryl, substituted or unsubstituted C₃ to C₁₈ heteroaryl.

According to an embodiment, wherein R¹ and R² may be independently selected from CN, partially fluorinated C₁ to C₄ alkyl or perfluorinated C₁ to C₄ alkyl, CF₃, substituted or unsubstituted C₆ to C₁₂ aryl, substituted or unsubstituted C₃ to C₁₂ heteroaryl.

According to one embodiment, the substituents R¹ and R² may be independently selected from CN, partially fluorinated C₁ to C₄ alkyl, perfluorinated C₁ to C₄ alkyl, CF₃, substituted or unsubstituted C₆ to C₁₀ aryl, or substituted or unsubstituted C₃ to C₉ heteroaryl.

According to one embodiment, the substituents R¹ and R² may be independently selected from CN, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl.

According to one embodiment, the substituents R¹ and R² may be independently selected from CN, partially fluorinated C₁ to C₄ alkyl, perfluorinated C₁ to C₄ alkyl, CF₃, substituted or unsubstituted C₆ aryl, substituted or unsubstituted C₄ to C₅ heteroaryl.

According to one embodiment, wherein the one or more substituents on R¹ and R² may be independently selected from D, partially fluorinated C₁ to C₈ alkyl, perfluorinated C₁ to C₈ alkyl, CF₃, or CN.

According to one embodiment, wherein the one or more substituents on R¹ and R² may be independently selected from D, partially fluorinated C₁ to C₄ alkyl, perfluorinated C₁ to C₄ alkyl, CF₃, or CN.

According to one embodiment, wherein the one or more substituents on R¹ and R² may be independently selected from D, CF₃, CN.

### Substituents on R¹ and R²

According to an embodiment, the one or more substituents on R¹ and R² are independently selected from D, partially fluorinated C₁ to C₄ alkyl or perfluorinated C₁ to C₄ alkyl, CF₃, halogen, F, CN.

According to an embodiment, the one or more substituents on R¹ and R² are independently selected from D, CF₃, halogen, F, CN.

According to an embodiment, the one or more substituents on R¹ and R² are independently selected from D, partially fluorinated C₁ to C₈ alkyl or perfluorinated C₁ to C₈ alkyl, CF₃, CN.

According to an embodiment, the one or more substituents on R¹ and R² are independently selected from D, partially fluorinated C₁ to C₄ alkyl or perfluorinated C₁ to C₄ alkyl, CF₃, CN.

According to an embodiment, the one or more substituents on R¹ and R² are independently selected from D, CF₃, CN.

### R³ and R⁴

According to one embodiment, the substituents R³ and R⁴ may be independently selected from H, D, CN, partially fluorinated C₁ to C₄ alkyl, perfluorinated C₁ to C₄ alkyl, or CF₃.

According to one embodiment, the substituents R³ and R⁴ may be independently preferably selected from H, D, CN, CF₃.

According to one embodiment, the substituents R³ and R⁴ may be independently further preferred selected from H or D.

### R⁵

According to an embodiment, wherein R⁵ is selected from H, D, halogen, F, CN, partially fluorinated C₁ to C₄ alkyl or perfluorinated C₁ to C₄ alkyl, CF₃, substituted or unsubstituted C₆ to C₂₄ aryl or substituted or unsubstituted C₃ to C₂₄ heteroaryl,

According to an embodiment, wherein R⁵ is selected from H, D, halogen, F, CN, partially fluorinated C₁ to C₄ alkyl or perfluorinated C₁ to C₄ alkyl, CF₃, substituted or unsubstituted C₆ to C₁₈ aryl or substituted or unsubstituted C₃ to C₁₈ heteroaryl,

According to an embodiment, wherein R⁵ is selected from H, D, halogen, F, CN, partially fluorinated C₁ to C₄ alkyl or perfluorinated C₁ to C₄ alkyl, CF₃, substituted or unsubstituted C₆ to C₁₂ aryl or substituted or unsubstituted C₃ to C₁₂ heteroaryl,

According to an embodiment, wherein R⁵ is selected from H, D, halogen, F, CN, partially fluorinated C₁ to C₄ alkyl or perfluorinated C₁ to C₄ alkyl, CF₃, substituted or unsubstituted C₆ to C₁₀ aryl or substituted or unsubstituted C₃ to C₉ heteroaryl,

According to an embodiment, wherein R⁵ is selected from H, D, halogen, F, CN, partially fluorinated C₁ to C₄ alkyl or perfluorinated C₁ to C₄ alkyl, CF₃, substituted or unsubstituted C₆ aryl or substituted or unsubstituted C₃ to C₅ heteroaryl.

According to one embodiment, the substituent R⁵ may be independently selected from H, D, CN, CF₃, substituted or unsubstituted C₆ aryl, substituted or unsubstituted C₄ to C₅ heteroaryl.

According to one embodiment, the substituent R⁵ may be independently preferably selected from H, D, CN, substituted or unsubstituted C₆ aryl or substituted or unsubstituted C₄ to C₅ heteroaryl.

According to one embodiment, the substituent R⁵ may be independently further preferred selected from substituted or unsubstituted C₆ aryl or substituted or unsubstituted C₄ to C₅ heteroaryl.

According to one embodiment, the one or more substituents on R⁵ are independently selected from D, partially fluorinated C₁ to C₄ alkyl, perfluorinated C₁ to C₄ alkyl, CF₃, halogen, F, or CN.

According to one embodiment, the one or more substituents on R⁵ are independently preferably selected from D, partially fluorinated C₁ to C₄ alkyl, perfluorinated C₁ to C₄ alkyl, CF₃, or CN.

According to one embodiment, the one or more substituents on R⁵ are independently further preferred selected from D, CF₃, or CN.

### Substituents on R⁵

According to an embodiment, wherein the one or more substituents on R⁵ are independently selected from D, partially fluorinated C₁ to C₄ alkyl or perfluorinated C₁ to C₄ alkyl, CF₃, halogen, F, CN.

According to an embodiment, wherein the one or more substituents on R⁵ are independently selected from D, partially fluorinated C₁ to C₄ alkyl or perfluorinated C₁ to C₄ alkyl, CF₃, CN.

According to an embodiment, wherein the one or more substituents on R⁵ are independently selected from D, CF₃, CN.

According to an embodiment, wherein the one or more substituents CN on R⁵ is not selected bound to an aromatic carbon when said aromatic carbon is directly bound to a sp2-hybridized ring nitrogen atom. The stability of the compound of formula (I) or (VII) can be further increased.

### Formula (VIa)

According to one embodiment, wherein the substituent R⁵ may be selected from formula (VIa): 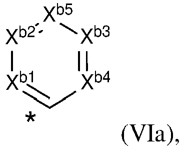 wherein the "*" denotes the binding position, and
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
   - R^{b1} to R^{b5}: are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, or CN,
   wherein the one or more substituents on R^{b1} to R^{b5} are independently selected from D, partially fluorinated C₁ to C₈ alkyl, perfluorinated C₁ to C₈ alkyl, CF₃, halogen, F, or CN.

According to one embodiment, wherein the substituent R⁵ may be selected from formula (VIa): 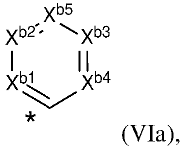 wherein the "*" denotes the binding position, and
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
   - R^{b1} to R^{b5}: are independently preferably selected from partially fluorinated or perfluorinated C₁ to C₈ alkyl, CF₃, halogen, F, or CN.

According to one embodiment, wherein the substituent R⁵ may be selected from formula (VIa): 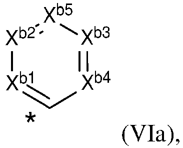 wherein the "*" denotes the binding position, and
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
   - R^{b1} to R^{b5}: are independently more preferred selected from partially fluorinated or perfluorinated C₁ to C₈ alkyl, CF₃, or CN.

According to one embodiment of formula (VIa), wherein when R^{bn} is N with n=1 to 5 then the neighbored R^{bm} with m=1 to 5 is not a CN group.

According to one embodiment, wherein the substituent R⁵ may be selected from formula (VIa): 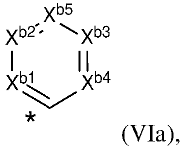 wherein the "*" denotes the binding position, and
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
   - R^{b1} to R^{b5}: are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, or CN,
   wherein the one or more substituents on R^{b1} to R^{b5} are independently selected from D, partially fluorinated C₁ to C₈ alkyl, perfluorinated C₁ to C₈ alkyl, CF₃, halogen, F, or CN;
wherein when R^{bn} is N with n=1 to 5 then the neighbored R^{bm} with m=1 to 5 is not a CN group.

### Formula (VIb)

According to one embodiment, wherein the substituent R⁵ may be selected from formula (VIb): 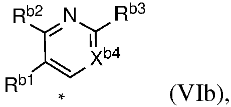 wherein the "*" denotes the binding position, and
wherein
- X^{b4}: is selected from N or CR^{b4},
- R^{b1} to R^{b3}: are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} to R^{b3} are independently selected from D, partially fluorinated C₁ to C₈ alkyl, perfluorinated C₁ to C₈ alkyl, CF₃, halogen, F, CN.

According to one embodiment according to formula (VIb), wherein preferably at least one of R^{b1} to R^{b3} is selected from partially fluorinated or perfluorinated C₁ to C₈ alkyl, CF₃, halogen, F, or CN.

According to one embodiment according to formula (VIb), wherein more preferably at least one of R^{b1} to R^{b3} is selected from partially fluorinated or perfluorinated C₁ to C₈ alkyl, CF₃, or CN.

### Formula (VIc)

According to one embodiment, wherein the substituent R⁵ may be selected from formula (VIc): 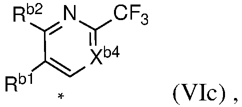 wherein the "*" denotes the binding position, and
wherein
- X^{b4}: is selected from N or CR^{b4},
- R^{b1} and R^{b2}: are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} and R^{b2} are independently selected from D, partially fluorinated C₁ to C₈ alkyl, perfluorinated C₁ to C₈ alkyl, CF₃, halogen, F, CN.

According to one embodiment according to formula (VIc), wherein preferably at least one of R^{b1} and R^{b2} is selected from partially fluorinated or perfluorinated C₁ to C₈ alkyl, CF₃, halogen, F, or CN.

According to one embodiment according to formula (VIc), wherein more preferably at least one of R^{b1} and R^{b2} is selected from partially fluorinated or perfluorinated C₁ to C₈ alkyl, CF₃, or CN.

### Formula (VId)

According to one embodiment, wherein the substituent R⁵ may be selected from formula (VId): 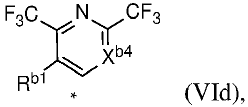 wherein the "*" denotes the binding position, and
wherein
- X^{b4}: is selected from N or CR^{b4},
- R^{b1}: is independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} is independently selected from D, partially fluorinated C₁ to C₈ alkyl, perfluorinated C₁ to C₈ alkyl, CF₃, halogen, F, CN.

According to one embodiment according to formula (VId), wherein preferably R^{b1} is selected from partially fluorinated or perfluorinated C₁ to C₈ alkyl, CF₃, halogen, F, or CN.

According to one embodiment according to formula (VId), wherein more preferably R^{b1} is selected from partially fluorinated or perfluorinated C₁ to C₈ alkyl, CF₃, or CN.

### Formula (VIe)

According to one embodiment, wherein the substituent R⁵ may be selected from formula (VIe): 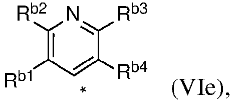 wherein the "*" denotes the binding position, and
wherein
- R^{b1} to R^{b4}: are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} to R^{b4} are independently selected from D, partially fluorinated C₁ to C₈ alkyl, perfluorinated C₁ to C₈ alkyl, CF₃, halogen, F, CN.

According to one embodiment according to formula (VIe), wherein preferably at least one of R^{b1} to R^{b4} are selected from partially fluorinated or perfluorinated C₁ to C₈ alkyl, CF₃, halogen, F, or CN.

According to one embodiment according to formula (VIe), wherein more preferably at least one of R^{b1} to R^{b4} are selected from partially fluorinated or perfluorinated C₁ to C₈ alkyl, CF₃, or CN.

### Formula (VIf)

According to one embodiment, wherein the substituent R⁵ may be selected from formula (VIf): 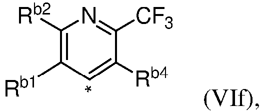 wherein the "*" denotes the binding position, and
wherein
- R^{b1}, R^{b2} and R^{b4}: are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1}, R^{b2} and R^{b4} are independently selected from D, partially fluorinated C₁ to C₈ alkyl, perfluorinated C₁ to C₈ alkyl, CF₃, halogen, F, CN.

According to one embodiment according to formula (VIf), wherein preferably at least one of R^{b1}, R^{b2} and R^{b4} are selected from partially fluorinated or perfluorinated C₁ to C₈ alkyl, CF₃, halogen, F, or CN.

According to one embodiment according to formula (VIf), wherein more preferably at least one of R^{b1} to R^{b4} are selected from partially fluorinated or perfluorinated C₁ to C₈ alkyl, CF₃, or CN.

### Formula (VIg)

According to one embodiment, wherein the substituent R⁵ may be selected from formula (VIg): 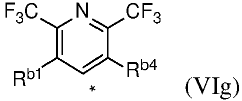 wherein the "*" denotes the binding position, and
wherein
- R^{b1} and R^{b4}: are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} and R^{b4} are independently selected from D, partially fluorinated C₁ to C₈ alkyl, perfluorinated C₁ to C₈ alkyl, CF₃, halogen, F, CN.

According to one embodiment according to formula (VIg), wherein preferably at least one of R^{b1} and R^{b4} are selected from partially fluorinated or perfluorinated C₁ to C₈ alkyl, CF₃, halogen, F, or CN.

According to one embodiment according to formula (VIg), wherein more preferably at least one of R^{b1} and R^{b4} are selected from partially fluorinated or perfluorinated C₁ to C₈ alkyl, CF₃, or CN.

### R^{a1} to R^{a4}

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₈ alkyl, partially fluorinated C₁ to C₈ alkyl or perfluorinated C₁ to C₈ alkyl, CF₃, substituted or unsubstituted C₆ to C₃₀ aryl, substituted or unsubstituted C₃ to C₃₀ heteroaryl, halogen, F, CN; and wherein at least one of R^{a1} to R^{a4} is selected from partially fluorinated C₁ to C₈ alkyl or perfluorinated C₁ to C₈ alkyl, CF₃, halogen, F, CN.

According to an embodiment, wherein R^{a1} to R^{a4} in formula (I), (VII), (II), (IIa), (IIb), (IIc), (IId), (IIe), (IIf), if present, if present are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₂₄ aryl, substituted or unsubstituted C₃ to C₂₄ heteroaryl, halogen, F, CN; and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₈ aryl, substituted or unsubstituted C₃ to C₁₈ heteroaryl, halogen, F, CN; and wherein at least one of R^{a1} to R^{a4} is selected from partially fluorinated CF₃, halogen, F, CN; and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₂ aryl, substituted or unsubstituted C₃ to C₁₂ heteroaryl, halogen, F, CN and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₀ aryl, substituted or unsubstituted C₃ to C₉ heteroaryl, halogen, F, CN; and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId) , (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ aryl, substituted or unsubstituted C₃ to C₅ heteroaryl, halogen, F, CN; and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId), if present, are independently selected from H, D, partially fluorinated C₁ to C₄ alkyl or perfluorinated C₁ to C₄ alkyl, CF₃, substituted or unsubstituted C₆ aryl, substituted or unsubstituted C₃ to C₅ heteroaryl, halogen, F, CN; and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId) if present are independently selected from H, D, partially fluorinated C₁ to C₄ alkyl or perfluorinated C₁ to C₄ alkyl, CF₃, halogen, F, CN. and wherein at least one of R^{a1} to R^{a4} is selected from partially fluorinated C₁ to C₄ alkyl or perfluorinated C₁ to C₄ alkyl, CF₃, halogen, F, CN.

According to an embodiment, wherein R^{a1} to R^{a4} in formula (I), (VII), (II), (IIa), (IIb), (IIc), (IId) if present are independently selected from H, D, CF₃, halogen, F, CN. and wherein at least one of R^{a1} to R^{a4} is selected from CF₃, halogen, F, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₈ alkyl, partially fluorinated C₁ to C₈ alkyl or perfluorinated C₁ to C₈ alkyl, CF₃, substituted or unsubstituted C₆ to C₃₀ aryl, substituted or unsubstituted C₃ to C₃₀ heteroaryl, CN; at least one of R^{a1} to R^{a4} is selected from partially fluorinated C₁ to C₈ alkyl or perfluorinated C₁ to C₈ alkyl, CF₃, CN; and wherein at least one of R^{a1} to R^{a4} is selected from partially fluorinated C₁ to C₈ alkyl or perfluorinated C₁ to C₈ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₂₄ aryl, substituted or unsubstituted C₃ to C₂₄ heteroaryl, CN; and wherein at least one of R^{a1} to R^{a4} is selected from partially fluorinated C₁ to C₄ alkyl or perfluorinated C₁ to C₄ alkyl, CF₃, CN; and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₈ aryl, substituted or unsubstituted C₃ to C₁₈ heteroaryl, CN; and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₂ aryl, substituted or unsubstituted C₃ to C₁₂ heteroaryl, CN; and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ to C₁₀ aryl, substituted or unsubstituted C₃ to C₉ heteroaryl, CN; and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, substituted or unsubstituted C₁ to C₄ alkyl, partially fluorinated C₁ to C₄ alkyl or perfluorinated C₁ to C₄ alkyl, CF₃, substituted or unsubstituted C₆ aryl, substituted or unsubstituted C₃ to C₅ heteroaryl, CN; and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId), (IIe), (IIf), if present, are independently selected from H, D, CF₃, substituted or unsubstituted C₆ aryl, substituted or unsubstituted C₃ to C₅ heteroaryl, CN and wherein at least one of R^{a1} to R^{a4} is selected from partially fluorinated CF₃, CN.

According to an embodiment, wherein R^{a1} to R^{a4} in formula (I), (VII), (II), (IIa), (IIb), (IIc), (IId) if present are independently selected from H, D, partially fluorinated C₁ to C₄ alkyl or perfluorinated C₁ to C₄ alkyl CF₃, CN. and wherein at least one of R^{a1} to R^{a4} is selected from partially fluorinated C₁ to C₄ alkyl or perfluorinated C₁ to C₄ alkyl, CF₃, CN.

According to an embodiment, wherein R^{a1} to R^{a4} in formula (I), (VII), (II), (IIa), (IIb), (IIc), (IId) if present are independently selected from H, D, CF₃, CN. and wherein at least one of R^{a1} to R^{a4} is selected from CF₃, CN.

According to an embodiment, wherein R^{a2} and R^{a3} in formula (I), (VII), (II), (IIa), (IIb), (IIc), (IId), (IIe), (IIf), if present, is not selected CN. Thereby, stability of the compound of formula I or V can be further increased.

### R^{a2} and R^{a3} / R^{a1} and R^{a4} differently defined

According to an embodiment, R^{a2} and R^{a3} in formula (I), (VII), (II), (IIa), (IIb), (IIc), (IId) , (IIe), (IIf), if present, are independently selected from H, D, substituted or unsubstituted C₁ to C₈ alkyl, partially fluorinated C₁ to C₈ alkyl or perfluorinated C₁ to C₈ alkyl, CF₃, halogen, F, CN, and R^{a1} and R^{a4} if present are independently selected from H, D, substituted or unsubstituted C₁ to C₈ alkyl, partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, CN.

### Substituents on R^{a1} to R^{a4}

According to an embodiment, wherein the one or more substituents on R^{a1} to R^{a4} in formula (I), (VII), (II), (IIa), (IIb), (IIc), (IId), (IIe), (IIf), if present, are independently selected from D, partially fluorinated C₁ to C₈ alkyl or perfluorinated C₁ to C₈ alkyl, CF₃, halogen, F, CN.

According to an embodiment, wherein the one or more substituents on R^{a1} to R^{a4} in formula (I), (VII), (II), (IIa), (IIb), (IIc), (IId), (IIe), (IIf), if present, are independently selected from D, partially fluorinated C₁ to C₄ alkyl or perfluorinated C₁ to C₄ alkyl, CF₃, halogen, F, CN.

According to an embodiment, wherein the one or more substituents on R^{a1} to R^{a4} in formula (I), (VII), (II), (IIa), (IIb), (IIc), (IId), (IIe), (IIf), if present, are independently selected from D, CF₃, halogen, F, CN.

According to an embodiment, wherein the one or more substituents on R^{a1} to R^{a4} in formula (I), (VII), (II), (IIa), (IIb), (IIc), (IId), (IIe), (IIf), if present, are independently selected from D, partially fluorinated C₁ to C₈ alkyl or perfluorinated C₁ to C₈ alkyl, CF₃, CN.

According to an embodiment, wherein the one or more substituents on R^{a1} to R^{a4} in formula (I), (VII), (II), (IIa), (IIb), (IIc), (IId), (IIe), (IIf), if present, are independently selected from D, partially fluorinated C₁ to C₄ alkyl or perfluorinated C₁ to C₄ alkyl, CF₃, CN.

According to an embodiment, wherein the one or more substituents on R^{a1} to R^{a4} in formula (I), (VII), (II), (IIa), (IIb), (IIc), (IId), (IIe), (IIf), if present, are independently selected from D, CF₃, CN.

According to an embodiment, at least one of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃, CN.

According to an embodiment, at least one of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃.

According to an embodiment, at least one of R^{a1} to R^{a4} in formula (I), (VII), (II), (IIa), (IIb), (IIc), (IId), (IIe), (IIf), if present, are independently selected from CF₃.

According to an embodiment, at least two of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃,halogen, F, or CN.

According to an embodiment, at least two of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃, or CN.

According to an embodiment, at least two of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃.

According to an embodiment, at least two of R^{a1} to R^{a4} in formula (I), (VII), (II), (IIa), (IIb), (IIc), (IId), (IIe), (IIf), if present, are independently selected from partially fluorinated or perfluorinated C₁ to C₈ alkyl, CF₃.

According to an embodiment, at least two of R^{a1} to R^{a4} in formula (I), (VII), (II), (IIa), (IIb), (IIc), (IId), (IIe), (IIf), if present, are independently selected from partially fluorinated or perfluorinated C₁ to C₄ alkyl, CF₃.

According to an embodiment, at least two of R^{a1} to R^{a4} in formula (I), (VII), (II), (IIa), (IIb), (IIc), (IId, (IIe), (IIf),) if present are independently selected from CF₃.

### R^{b1}to R^{b5}

According to an embodiment, wherein R^{b1} to R^{b5} in formula I, V, VIa, VIb, VIc, VId, VIe, VIf, VIg if present, is not selected halogen or F.

According to an embodiment, wherein R^{b1} to R^{b5} in formula I, V, VIa, VIb, VIc, VId, VIe, VIf, VIg, if present,is not selected halogen or F, and the one or more substituents on R^{b1} to R^{b5} is not selected halogen or F. Thereby, stability of the compound of formula I or V can be further increased.

According to an embodiment, wherein R^{b2} to R^{b3} in formula I, V, VIa, VIb, VIc, VId, VIe, VIf, VIg if present, is not selected CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₃ to C₃₀ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₂₄ aryl, substituted or unsubstituted C₃ to C₂₄ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₈ aryl, substituted or unsubstituted C₃ to C₁₈ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₂ aryl, substituted or unsubstituted C₃ to C₁₂ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₀ aryl, substituted or unsubstituted C₃ to C₉ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl, halogen, F, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₃ to C₃₀ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₂₄ aryl, substituted or unsubstituted C₃ to C₂₄ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₈ aryl, substituted or unsubstituted C₃ to C₁₈ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₂ aryl, substituted or unsubstituted C₃ to C₁₂ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ to C₁₀ aryl, substituted or unsubstituted C₃ to C₉ heteroaryl, CN.

According to an embodiment, wherein R^{b1} to R^{b5} in formula I, V, VIa, VIb, VIc, VId, VIe, VIf, VIg, if present, are independently selected from H, D substituted or unsubstituted C₁ to C₄ alkyl, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl, CN.

According to an embodiment, at least one of R^{b1} to R^{b5} in formula I, V, VIa, VIb, VIc, VId, VIe, VIf, VIg,if present, are independently selected from partially fluorinated or perfluorinated C₁ to C₈ alkyl, CF₃, CN.

According to an embodiment, at least one of R^{b1} to R^{b5} in formula I, V, VIa, VIb, VIc, VId, VIe, VIf, VIg, if present, are independently selected from partially fluorinated or perfluorinated C₁ to C₈ alkyl, CF₃.

According to an embodiment, at least one of R^{b1} to R^{b5} in formula I, V, VIa, VIb, VIc, VId, VIe, VIf, VIg, if present, are independently selected from CF₃

### Substituents on R^{b1} to R^{b5}

According to an embodiment, wherein the at least one or more substituents on R^{b1} to R^{b5} in formula I, V, VIa, VIb, VIc, VId, VIe, VIf, VIg, if present, are independently selected from D, partially fluorinated C₁ to C₈ alkyl, perfluorinated C₁ to C₈ alkyl, CF₃, halogen, F, CN.

According to an embodiment, wherein the at least one or more substituents on R^{b1} to R^{b5} in formula I, V, VIa, VIb, VIc, VId, VIe, VIf, VIg, if present, are independently selected from D, partially fluorinated C₁ to C₈ alkyl, perfluorinated C₁ to C₈ alkyl, CF₃, halogen, F, CN.

According to an embodiment, wherein the at least one or more substituents on R^{b1} to R^{b5} in formula I, V, VIa, VIb, VIc, VId, VIe, VIf, VIg, if present, are independently selected from D, partially fluorinated C₁ to C₄ alkyl, perfluorinated C₁ to C₄ alkyl, CF₃, halogen, F, CN.

According to an embodiment, wherein the at least one or more substituents on R^{b1} to R^{b5} in formula I, V, VIa, VIb, VIc, VId, VIe, VIf, VIg, if present, are independently selected from D, CF₃, halogen, F, CN.

According to an embodiment, wherein the at least one or more substituents on R^{b1} to R^{b5} in formula I, V, VIa, VIb, VIc, VId, VIe, VIf, VIg, if present, are independently selected from D, partially fluorinated C₁ to C₈ alkyl, perfluorinated C₁ to C₈ alkyl, CF₃, CN.

According to an embodiment, wherein the at least one or more substituents on R^{b1} to in formula I, V, VIa, VIb, VIc, VId, VIe, VIf, VIg, if present, are independently selected from D, partially fluorinated C₁ to C₈ alkyl, perfluorinated C₁ to C₈ alkyl, CF₃, CN.

According to an embodiment, wherein the at least one or more substituents on R^{b1} to R^{b5} in formula I, V, VIa, VIb, VIc, VId, VIe, VIf, VIg, if present, are independently selected from D, partially fluorinated C₁ to C₄ alkyl, perfluorinated C₁ to C₄ alkyl, CF₃, CN.

According to an embodiment, wherein the at least one or more substituents on R^{b1} to in formula I, V, VIa, VIb, VIc, VId, VIe, VIf, VIg, if present, are independently selected from D, CF₃, CN.

### R^{a1} to R^{a4} and R^{b1} to R^{b5}

According to one embodiment, wherein R^{a1} to R^{a4} in formula I, R^{b1} to R^{b5} in formula (VIa), R^{b1} to R^{b3} in formula (VIb), R^{b1} to R^{b2} in formula (VIc), R^{b1} in formula (VId), R^{b1} to R^{b4} in formula (Vie), R^{b1}, R^{b2} and R^{b4} in formula (VIf), R^{b1} and R^{b4} in formula (VIg) are not selected halogen or F; and preferably R^{a1} to R^{a4} in formula I, R^{b1} to R^{b5} in formula (VIa), R^{b1} to R^{b3} in formula (VIb), R^{b1} to R^{b2} in formula (VIc), R^{b1} in formula (VId), R^{b1} to R^{b4} in formula (Vie), R^{b1}, R^{b2} and R^{b4} in formula (VIf), R^{b1} and R^{b4} in formula (VIg) are not selected halogen or F, and the one or more substituents on R^{a1} to R^{a4}, R^{b1} to R^{b5} are not selected from halogen or F.

According to one embodiment, wherein R^{a2} to R^{a3} in formula (II), R^{b2} to R^{b3} in formula (VIa), (VIb), (VIe), and R^{b2} in formula (VIc) and (VIf) are not selected CN.

### R^{a1} to R^{a4} / R³, R⁴, R⁵

According to one embodiment, wherein R³, R⁴, R⁵ and R^{a1} to R^{a4} in formula (I), (VII), (II), (IIa), (IIb), (IIc), (IId), (IIe), (IIf), if present, cannot be halogen or F.

According to an embodiment, R³, R⁴, R⁵ and R^{a1} to R^{a4} in formula (I), (VII), (II), (IIa), (IIb), (IIc), (IId), (IIe), (IIf), if present, cannot be halogen or F, and the one or more substituents on R¹, R² , R⁵ and R^{a1} to R^{a4} cannot be halogen or F.

According to an embodiment, R³, R⁴, R⁵ and R^{a1} to R^{a4} in formula (I), (VII), (II), (IIa), (IIb), (IIc), (IId) , (IIe), (IIf), if present, cannot be halogen or F, and R^{b1} to R^{b5} cannot be halogen or F.

. According to an embodiment, R³, R⁴, R⁵ and R^{a1} to R^{a4} in formula (I), (VII), (II), (IIa), (IIb), (IIc), (IId), (IIe), (IIf), if present, cannot be halogen or F, and the one or more substituents on R¹, R² , R⁵ and R^{a1} to R^{a4} cannot be halogen or F, and R^{b1} to R^{b5} cannot be halogen or F, and the one or more substituents on R^{b1} to R^{b5} cannot be halogen or F.

According to an embodiment, the compound comprises 2 to 6 CN groups, preferably 3 to 6 CN groups, and more preferably 4 to CN groups in order to maintain a moderate evaporation temperature, for the manufacturing of the OLED.

### Specific groups of R⁵

According to one embodiment, wherein R⁵ is selected from a group represented by B1 to B160:

| | | | |
|---|---|---|---|
| 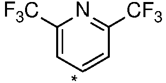 | 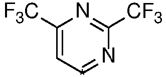 | 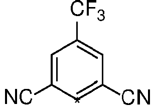 | 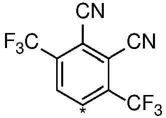 |
| B1 | B2 | B3 | B4 |
| 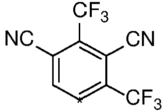 | 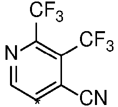 | 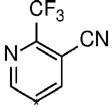 | 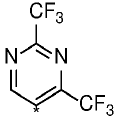 |
| B5 | B6 | B7 | B8 |
| 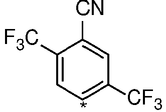 | 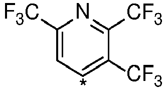 | 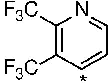 | 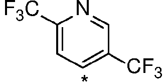 |
| B9 | B10 | B11 | B12 |
| 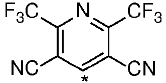 | 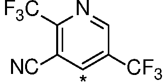 | 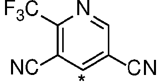 | 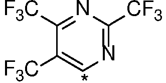 |
| B13 | B14 | B15 | B16 |
| 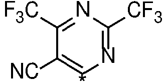 | 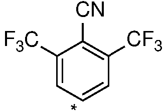 | 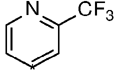 | 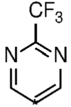 |
| B17 | B18 | B19 | B20 |
| 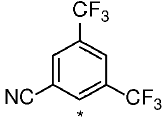 | 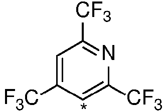 | 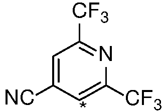 | 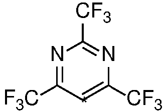 |
| B21 | B22 | B23 | B24 |
| 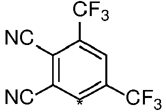 | 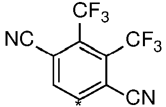 | 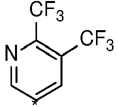 | 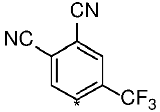 |
| B25 | B26 | B27 | B28 |
| 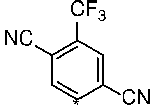 | 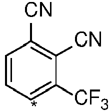 | 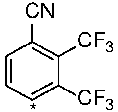 | 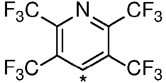 |
| B29 | B30 | B31 | B32 |
| 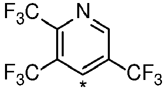 | 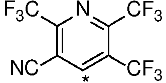 | 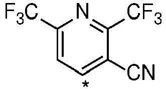 | 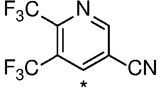 |
| B33 | B34 | B35 | 36B |
| 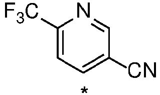 | 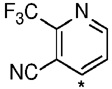 | 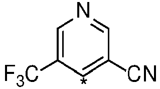 | 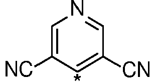 |
| B37 | B38 | B39 | B40 |
| 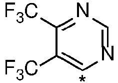 | 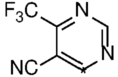 | 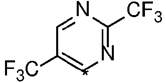 | 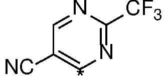 |
| B41 | B42 | B43 | B44 |
| 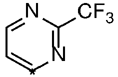 | 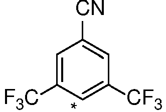 | 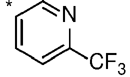 | 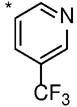 |
| B45 | B46 | B47 | B48 |
| 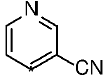 | 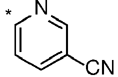 | 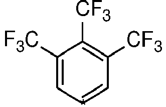 | 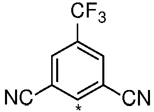 |
| B49 | B50 | B51 | B52 |
| 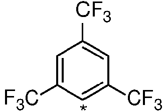 | 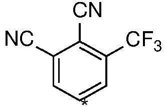 | 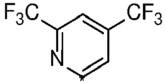 | 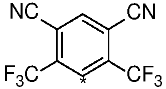 |
| B53 | B54 | B55 | B56 |
| 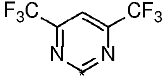 | 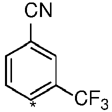 | 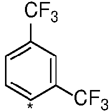 | 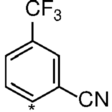 |
| B57 | B58 | B59 | B60 |
| 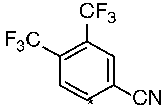 | 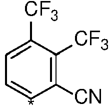 | 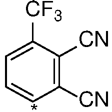 | 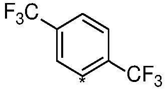 |
| B61 | B62 | B63 | B64 |
| 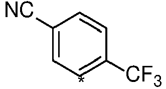 | 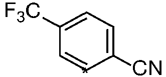 | 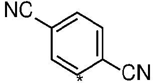 | 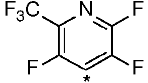 |
| B65 | B66 | B67 | B68 |
| 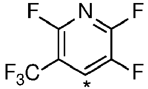 | 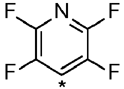 | 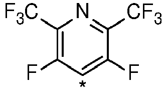 | 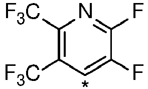 |
| B69 | B70 | B71 | B72 |
| 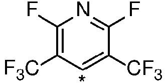 | 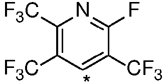 | 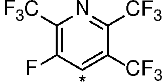 | 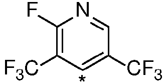 |
| B73 | B74 | B75 | B76 |
| 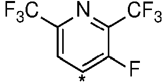 | 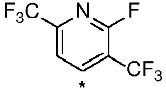 | 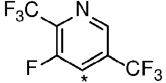 | 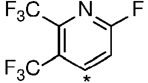 |
| B77 | B78 | B79 | B80 |
| 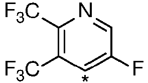 | 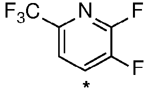 | 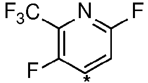 | 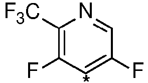 |
| B81 | B82 | B83 | B84 |
| 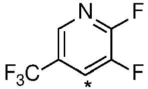 | 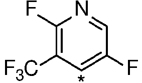 | 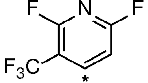 | 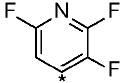 |
| B85 | B86 | B87 | B88 |
| 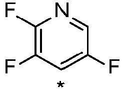 | 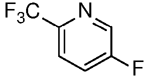 | 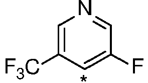 | 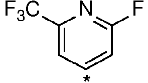 |
| B89 | B90 | B91 | B92 |
| 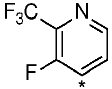 | 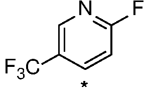 | 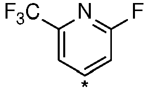 | 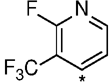 |
| B93 | B94 | B95 | B96 |
| 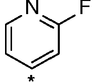 | 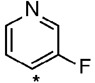 | 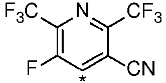 | 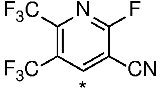 |
| B97 | B98 | B99 | B100 |
| 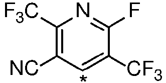 | 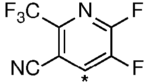 | 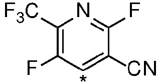 | 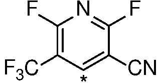 |
| B101 | B102 | B103 | B104 |
| 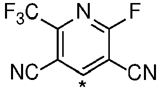 | 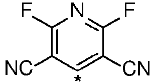 | 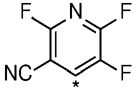 | 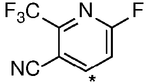 |
| B105 | B106 | B107 | B108 |
| 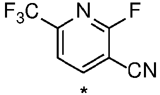 | 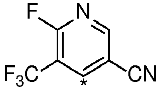 | 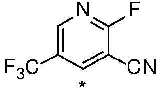 | 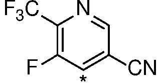 |
| B109 | B110 | B111 | B112 |
| 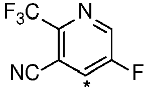 | 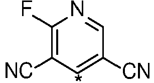 | 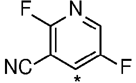 | 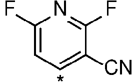 |
| B113 | B114 | B115 | B116 |
| 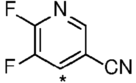 | 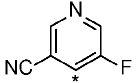 | 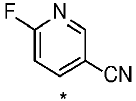 | 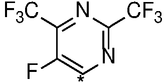 |
| B117 | B118 | B119 | B120 |
| 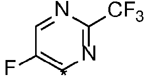 | 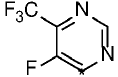 | 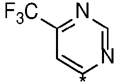 | 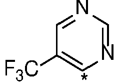 |
| B121 | B122 | B123 | B124 |
| 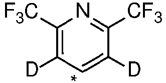 | 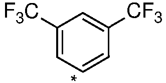 | 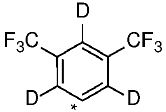 | 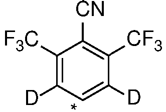 |
| B125 | B126 | B127 | B128 |
| 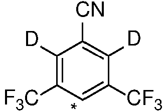 | 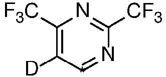 | 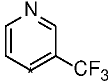 | 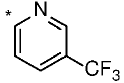 |
| B129 | B130 | B131 | B132 |
| 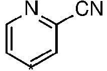 | 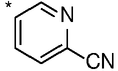 | 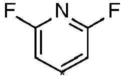 | 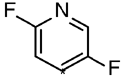 |
| B133 | B134 | B135 | B136 |
| 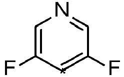 | 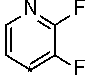 | 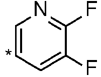 | 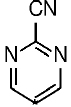 |
| B137 | B138 | B139 | B140 |
| 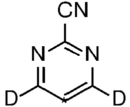 | 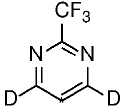 | 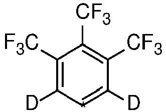 | 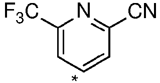 |
| B141 | B142 | B143 | B144 |
| 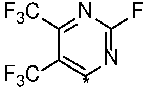 | 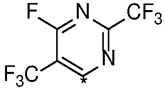 | 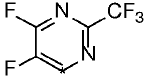 | 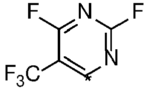 |
| B145 | B146 | B147 | B148 |
| 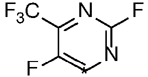 | 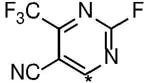 | 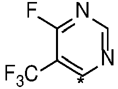 | 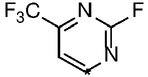 |
| B149 | B150 | B151 | B152 |
| 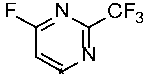 | 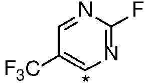 | 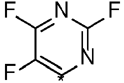 | 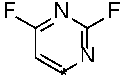 |
| B153 | B154 | B155 | B156 |
| 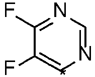 | 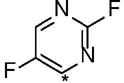 | 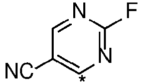 | 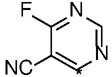 |
| B157 | B158 | B159 | B160 |

, wherein the "*" denotes the binding position.

According to a preferred embodiment, wherein R⁵ is selected from **B1 to B124.** According to a more preferred embodiment, wherein R⁵ is selected from **B1 to B45.** According to a most preferred embodiment, wherein R⁵ is selected from **B1 to B17.**

### A¹ and A²

According to one embodiment of the compound of formula (I), wherein A¹ and A² may be selected from formula (V): 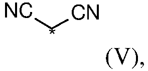 wherein the asterisk "*" denotes the binding position.

### Formula (II) of HetAr

According to an embodiment, formula II is selected from formula IIa

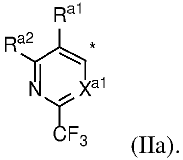

According to a preferred embodiment, formula II is selected from formula IIb

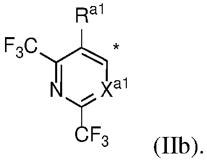

According to a more preferred embodiment, formula II is selected from formula IIc

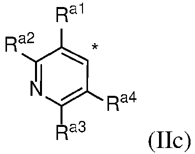

According to an even more preferred embodiment, formula II is selected from formula IId

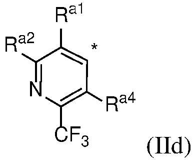

According to an even more preferred embodiment, formula II is selected from formula IIe

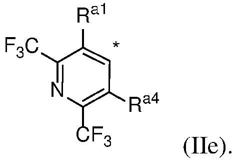

According to a most preferred embodiment, formula II is selected from formula IIf 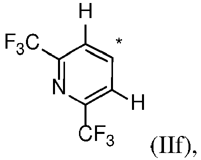 wherein formula IIf can also be partially deuterated or fully deuterated.

According to one embodiment of the compound of formula (I), wherein formula (II) is selected from formulas (IIa), (IIb), (IIc), (IId), (IIe), and (IIf): 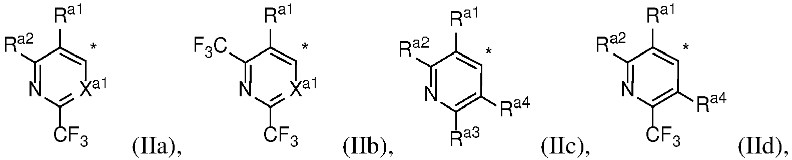 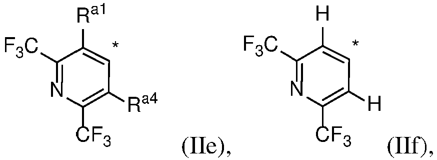 wherein the "*" denotes the binding position, and preferably wherein formulas (IIa), (IIb), (IIc), (IId), (IIe), and (IIf) are partially deuterated or fully deuterated, and further preferred formula (IIf) is partially deuterated or fully deuterated.

### HetAr

According to one embodiment of the compound of formula (I), wherein HetAr is selected from a group represented by C1 to C109:

| | | | |
|---|---|---|---|
| 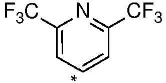 | 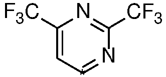 | 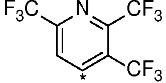 | 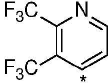 |
| C1 | C2 | C3 | C4 |
| 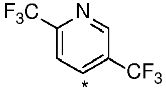 | 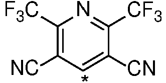 | 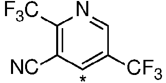 | 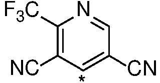 |
| C5 | C6 | C7 | C8 |
| 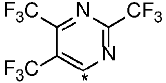 | 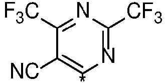 | 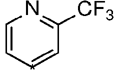 | 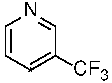 |
| C9 | C10 | C11 | C12 |
| 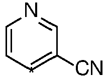 | 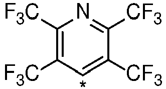 | 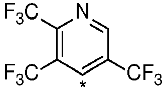 | 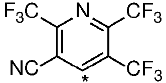 |
| C13 | C14 | C15 | C16 |
| 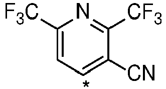 | 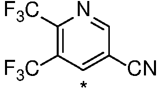 | 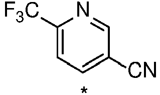 | 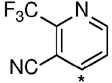 |
| C17 | C18 | C19 | C20 |
| 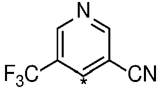 | 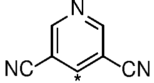 | 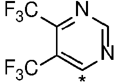 | 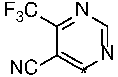 |
| C21 | C22 | C23 | C24 |
| 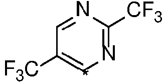 | 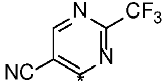 | 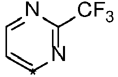 | 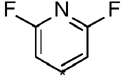 |
| C25 | C26 | C27 | C28 |
| 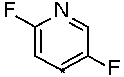 | 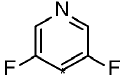 | 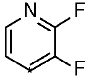 | 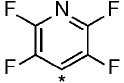 |
| C29 | C30 | C31 | C32 |
| 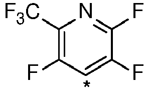 | 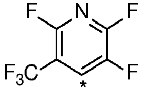 | 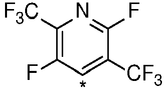 | 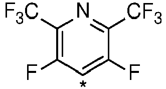 |
| C33 | C34 | C35 | C36 |
| 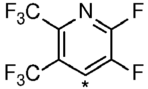 | 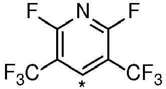 | 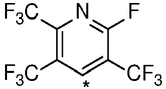 | 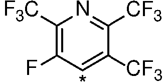 |
| C37 | C38 | C39 | C40 |
| 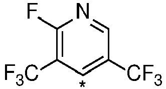 | 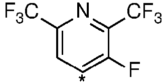 | 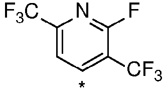 | 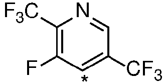 |
| C41 | C42 | C43 | C44 |
| 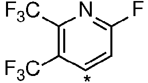 | 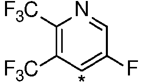 | 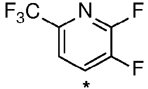 | 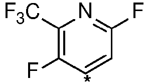 |
| C45 | C46 | C47 | C48 |
| 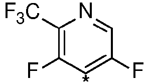 | 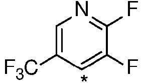 | 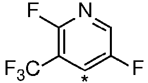 | 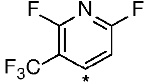 |
| C49 | C50 | C51 | C52 |
| 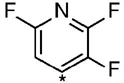 | 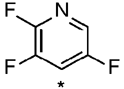 | 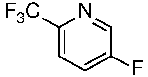 | 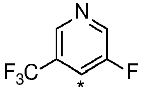 |
| C53 | C54 | C55 | C56 |
| 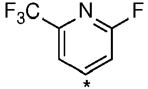 | 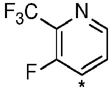 | 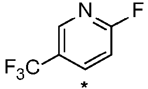 | 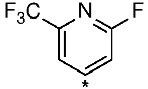 |
| C57 | C58 | C59 | C60 |
| 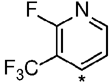 | 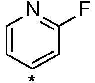 | 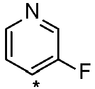 | 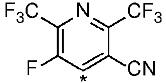 |
| C61 | C62 | C63 | C64 |
| 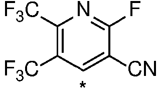 | 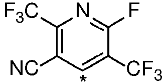 | 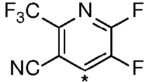 | 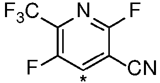 |
| C65 | C66 | C67 | C68 |
| 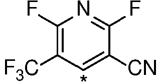 | 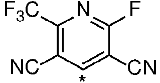 | 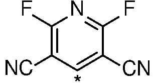 | 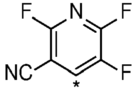 |
| C69 | C70 | C71 | C72 |
| 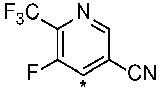 | 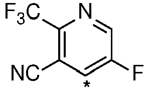 | 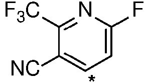 | 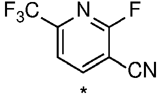 |
| C73 | C74 | C75 | C76 |
| 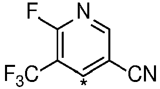 | 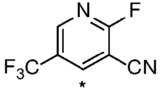 | 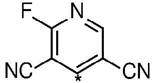 | 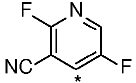 |
| *C77* | C78 | C79 | C80 |
| 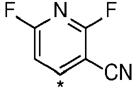 | 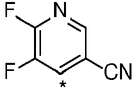 | 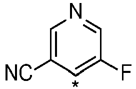 | 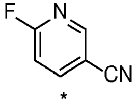 |
| C81 | C82 | C83 | C84 |
| 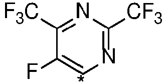 | 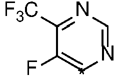 | 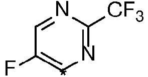 | 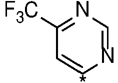 |
| C85 | C86 | C87 | C88 |
| 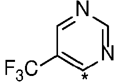 | 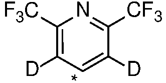 | 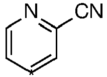 | 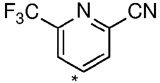 |
| C89 | C90 | C91 | C92 |
| 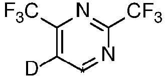 | 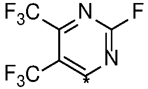 | 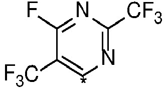 | 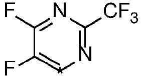 |
| C93 | C94 | C95 | C96 |
| 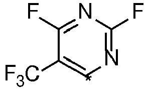 | 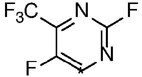 | 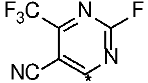 | 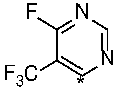 |
| C97 | C98 | C99 | C100 |
| 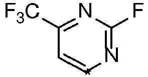 | 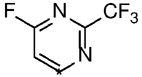 | 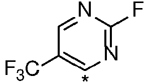 | 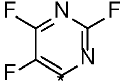 |
| C101 | C102 | C103 | C104 |
| 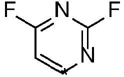 | 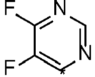 | 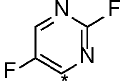 | 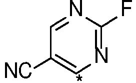 |
| C105 | C106 | C107 | C108 |
| 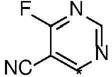 | | | |
| C109 | | | |

, wherein the "*" denotes the binding position.

According to one embodiment of the compound of formula (I), wherein for HetAr the group represented by C90 to C109 are suitable for use.

According to one embodiment of the compound of formula (I), wherein for HetAr the group represented by C28 to C89 are preferred.

According to one embodiment of the compound of formula (I), wherein for HetAr the group represented by C11 to C27 are more preferred.

According to one embodiment of the compound of formula (I), wherein for HetAr the group represented by C1 to C10 are most preferred.

According to an embodiment, HetAr is selected from C1 to C89. According to a further embodiment, HetAr is selected from C1 to C27.

### HetAr / R⁵

According to one embodiment of the compound of formula (I), wherein HetAr and R⁵ is selected the same.

### HetAr / R⁵ / A¹ and A²

According to an embodiment, HetAr is selected from formula II 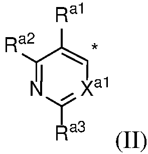 and R⁵ is selected from formula VIa 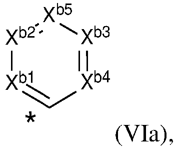 wherein preferably R³ and R⁴ are independently selected from H, D, and A¹ and A² are selected from 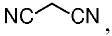 wherein the asterisk "*" denotes the binding position.

According to an embodiment, HetAr is selected from formula II 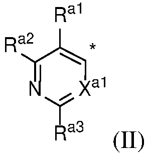 and formula VIa is selected from formula VIb 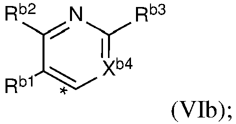 wherein preferably R³ and R⁴ are independently selected from H, D, and A¹ and A² are selected from 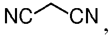 wherein the asterisk "*" denotes the binding position.

According to an embodiment, HetAr is selected from formula II 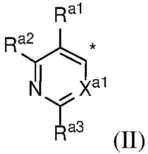 and formula VIa is selected from formula VIc 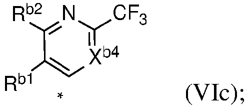 wherein preferably R³ and R⁴ are independently selected from H, D, and A¹ and A² are selected from 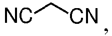 wherein the asterisk "*" denotes the binding position.

According to an embodiment, formula II is selected from formula IIa 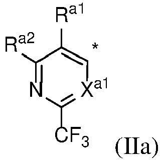 and R⁵ is selected from formula VIa 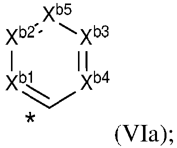 wherein preferably R³ and R⁴ are independently selected from H, D, and A¹ and A² are selected from 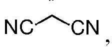 wherein the asterisk "*" denotes the binding position.

According to an embodiment, formula II is selected from formula IIa 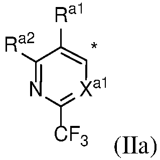 and formula VIa is selected from formula VIb 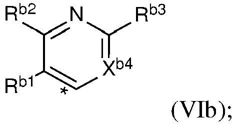 wherein preferably R³ and R⁴ are independently selected from H, D, and A¹ and A² are selected from 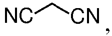 wherein the asterisk "*" denotes the binding position.

According to an embodiment, formula II is selected from formula IIa 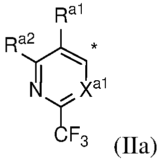 and R⁵ is selected from formula VIc 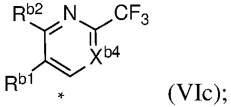 wherein preferably R³ and R⁴ are independently selected from H, D, and A¹ and A² are selected from 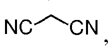 wherein the asterisk "*" denotes the binding position.

According to an embodiment, formula II is selected from formula IIa 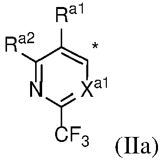 and R⁵ is selected from formula VId 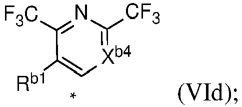 wherein preferably R³ and R⁴ are independently selected from H, D, and A¹ and A² are selected from 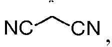 wherein the asterisk "*" denotes the binding position.

According to an embodiment, formula II is selected from formula IIb 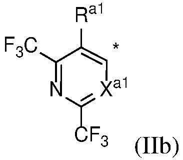 and R⁵ is selected from formula VIa 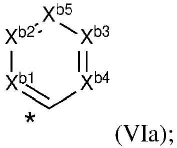 wherein preferably R³ and R⁴ are independently selected from H, D, and A¹ and A² are selected from 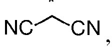 wherein the asterisk "*" denotes the binding position.

According to an embodiment, formula II is selected from formula IIb 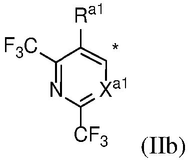 and formula VIa is selected from formula VIb 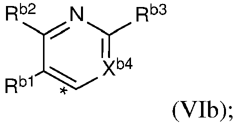 wherein preferably R³ and R⁴ are independently selected from H, D, and A¹ and A² are selected from 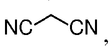 wherein the asterisk "*" denotes the binding position.

According to an embodiment, formula II is selected from formula IIb 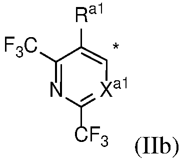 and R⁵ is selected from formula VIc 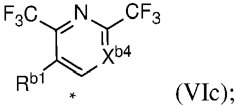 wherein preferably R³ and R⁴ are independently selected from H, D, and A¹ and A² are selected from 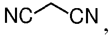 wherein the asterisk "*" denotes the binding position.

According to an embodiment, formula II is selected from formula IIb 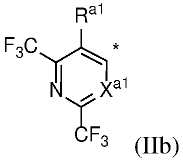 and R⁵ is selected from formula VId 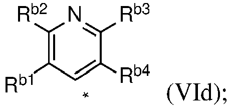 wherein preferably R³ and R⁴ are independently selected from H, D, and A¹ and A² are selected from 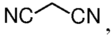 wherein the asterisk "*" denotes the binding position.

According to an embodiment, formula II is selected from formula IIb 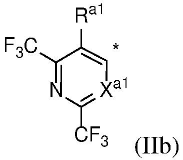 and formula VIa is selected from formula VIe is 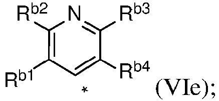 wherein preferably R³ and R⁴ are independently selected from H, D, and A¹ and A² are selected from 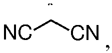 wherein the asterisk "*" denotes the binding position.

According to an embodiment, formula II is selected from formula IIc 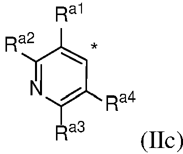 and R⁵ is selected from formula VIa 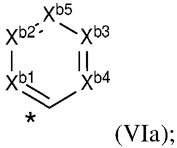 wherein preferably R³ and R⁴ are independently selected from H, D, and A¹ and A² are selected from 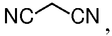 wherein the asterisk "*" denotes the binding position.

According to an embodiment, formula II is selected from formula IIc 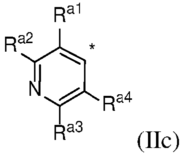 and formula VIa is selected from formula VIb 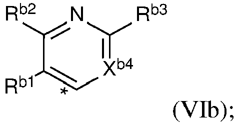 wherein preferably R³ and R⁴ are independently selected from H, D, and A¹ and A² are selected from 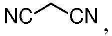 wherein the asterisk "*" denotes the binding position.

According to an embodiment, formula II is selected from formula IIc 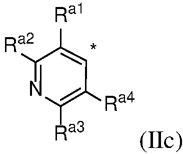 and R⁵ is selected from formula VIc 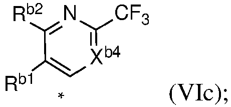 wherein preferably R³ and R⁴ are independently selected from H, D, and A¹ and A² are selected from 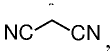 wherein the asterisk "*" denotes the binding position.

According to an embodiment, formula II is selected from formula IIc 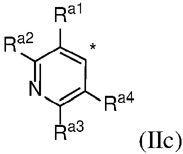 and R⁵ is selected from formula VId 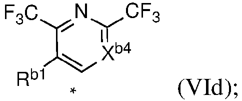 wherein preferably R³ and R⁴ are independently selected from H, D, and A¹ and A² are selected from 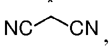 wherein the asterisk "*" denotes the binding position.

According to an embodiment, formula II is selected from formula IIc 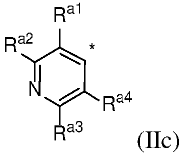 and formula VIa is selected from VIe is 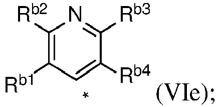 wherein preferably R³ and R⁴ are independently selected from H, D, and A¹ and A² are selected from 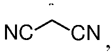 wherein the asterisk "*" denotes the binding position.

According to an embodiment, formula II is selected from formula IId 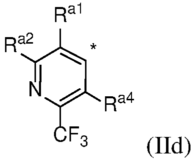 and R⁵ is selected from formula VIa 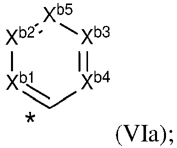 wherein preferably R³ and R⁴ are independently selected from H, D, and A¹ and A² are selected from 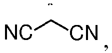 wherein the asterisk "*" denotes the binding position.

According to an embodiment, formula II is selected from formula IId 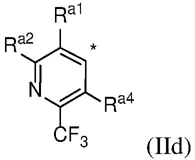 and formula VIa is selected from formula VIb 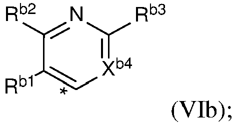 wherein preferably R³ and R⁴ are independently selected from H, D, and A¹ and A² are selected from 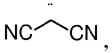 wherein the asterisk "*" denotes the binding position.

According to an embodiment, formula II is selected from formula IId 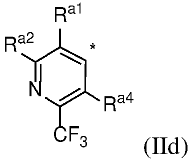 and R⁵ is selected from formula VIc 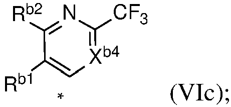 wherein preferably R³ and R⁴ are independently selected from H, D, and A¹ and A² are selected from 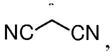 wherein the asterisk "*" denotes the binding position.

According to an embodiment, formula II is selected from formula IId 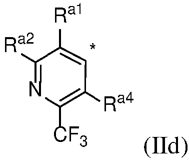 and R⁵ is selected from formula VId 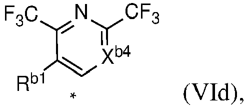 wherein preferably R³ and R⁴ are independently selected from H, D, and A¹ and A² are selected from 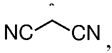 wherein the asterisk "*" denotes the binding position.

According to an embodiment, formula II is selected from formula IId 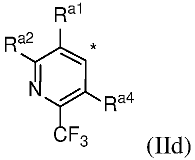 and formula VIa is selected from VIe is 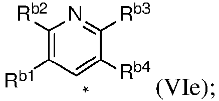 wherein preferably R³ and R⁴ are independently selected from H, D, and A¹ and A² are selected from 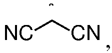 wherein the asterisk "*" denotes the binding position.

According to an embodiment, formula II is selected from formula IId 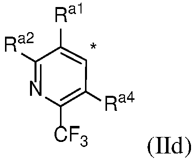 and formula VIa is selected from VIf 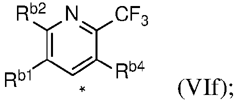 wherein preferably R³ and R⁴ are independently selected from H, D, and A¹ and A² are selected from 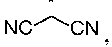 wherein the asterisk "*" denotes the binding position.

According to an embodiment, formula II is selected from formula IIe 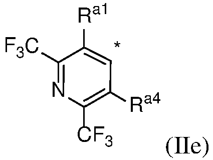 and R⁵ is selected from formula VIa 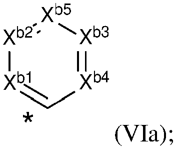 wherein preferably R³ and R⁴ are independently selected from H, D, and A¹ and A² are selected from 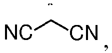 wherein the asterisk "*" denotes the binding position.

According to an embodiment, formula II is selected from formula IIe 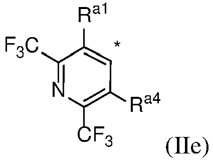 and formula VIa is selected from VIb is 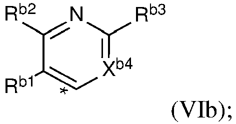 wherein preferably R³ and R⁴ are independently selected from H, D, and A¹ and A² are selected from 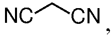 wherein the asterisk "*" denotes the binding position.

According to an embodiment, formula II is selected from formula IIe 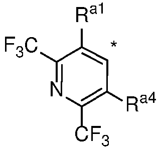 (IIe) and R⁵ is selected from formula VIc 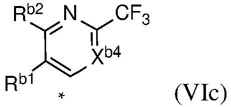 wherein preferably R³ and R⁴ are independently selected from H, D, and A¹ and A² are selected from 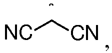 wherein the asterisk "*" denotes the binding position.

According to an embodiment, formula II is selected from formula IIe 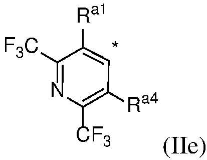 and R⁵
is selected from formula VId 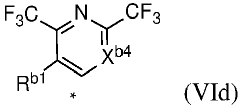 ; wherein preferably R³ and R⁴ are independently selected from H, D, and A¹ and A² are selected from 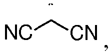 wherein the asterisk "*" denotes the binding position.

According to an embodiment, formula II is selected from formula IIe 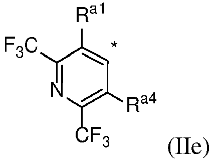 and formula VIa is selected from VIe is 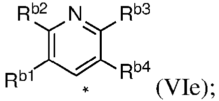 wherein preferably R³ and R⁴ are independently selected from H, D, and A¹ and A² are selected from 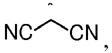 wherein the asterisk "*" denotes the binding position.

According to an embodiment, formula II is selected from formula IIe 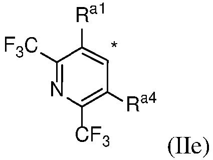 and formula VIa is selected from VIf is 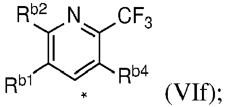 wherein preferably R³ and R⁴ are independently selected from H, D, and A¹ and A² are selected from 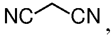 wherein the asterisk "*" denotes the binding position.

According to an embodiment, formula II is selected from formula IIe 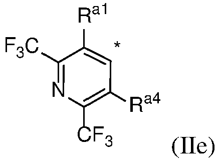 and formula VIa is selected from VIg is 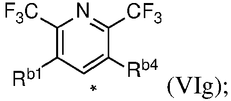 wherein preferably R³ and R⁴ are independently selected from H, D, and A¹ and A² are selected from 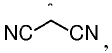 wherein the asterisk "*" denotes the binding position.

According to an embodiment, formula II is selected from formula IIf 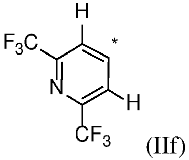 and R⁵ is selected from formula VIa 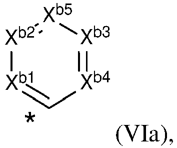 wherein HetAr can be partially deuterated or fully deuterated; wherein preferably R³ and R⁴ are independently selected from H, D, and A¹ and A² are selected from 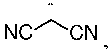 wherein the asterisk "*" denotes the binding position.

According to an embodiment, formula II is selected from formula IIf 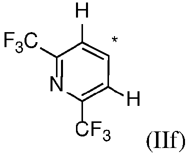 and formula VIa is selected from VIb is 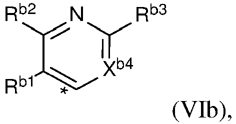 wherein HetAr can be partially deuterated or fully deuterated; wherein preferably R³ and R⁴ are independently selected from H, D, and A¹ and A ² are selected from 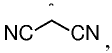 wherein the asterisk "*" denotes the binding position.

According to an embodiment, formula II is selected from formula IIf 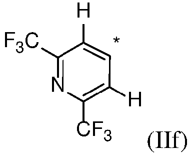 and R⁵ is selected from formula VIc 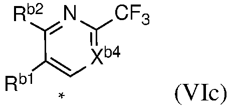 wherein HetAr can be partially deuterated or fully deuterated; wherein preferably R³ and R⁴ are independently selected from H, D, and A¹ and A² are selected from 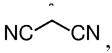 wherein the asterisk "*" denotes the binding position.

According to an embodiment, formula II is selected from formula IIf 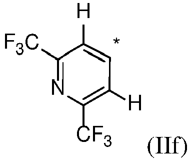 and R⁵ is selected from formula VIc 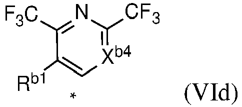 wherein HetAr can be partially deuterated or fully deuterated; wherein preferably R³ and R⁴ are independently selected from H, D, and A¹ and A² are selected from 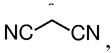 wherein the asterisk "*" denotes the binding position.

According to an embodiment, formula II is selected from formula IIf 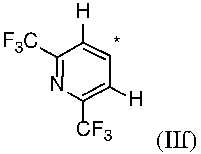 and formula VIa is selected from VIe is 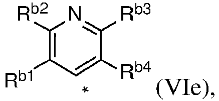 wherein HetAr can be partially deuterated or fully deuterated; wherein preferably R³ and R⁴ are independently selected from H, D, and A¹ and A ² are selected from 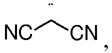 wherein the asterisk "*" denotes the binding position.

According to an embodiment, formula II is selected from formula IIf 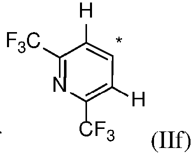 and formula VIa is selected from VIf is 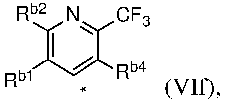 wherein HetAr can be partially deuterated or fully deuterated; wherein preferably R³ and R⁴ are independently selected from H, D, and A¹ and A ² are selected from 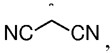 wherein the asterisk "*" denotes the binding position.

According to an embodiment, formula II is selected from formula IIf 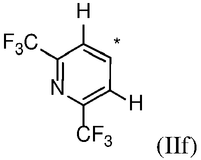 and formula VIa is selected from VIg is 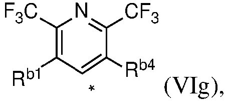 wherein HetAr can be partially deuterated or fully deuterated; wherein preferably R³ and R⁴ are independently selected from H, D, and A¹ and A ² are selected from 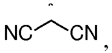 wherein the asterisk "*" denotes the binding position.

According to an embodiment, wherein R⁵ and HetAr is the same.

### Compound of formula (VII)

According to an embodiment, the compound of formula (I) is selected from a compound of formula (VII): 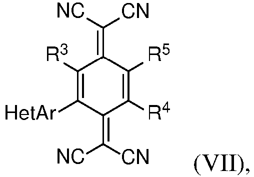 wherein
HetAr is selected from formulas (II), (IIa), (IIb), (IIc), (IId), (IIe), and (IIf): 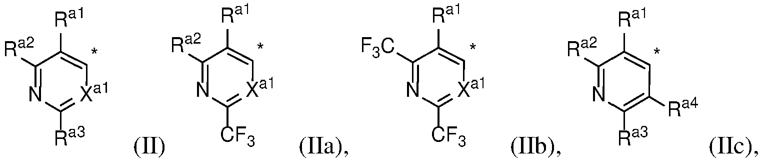 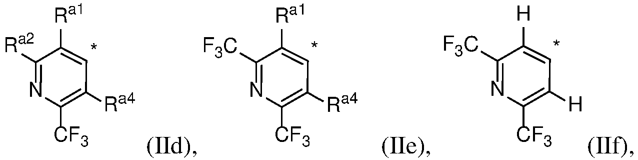 wherein the asterisk "*" denotes the binding position, and wherein
R³ and R⁴ are selected from H, D;
X^{a1} is selected from N or CR^{a4};
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, or CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, or CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, or CN.

According to an embodiment, the compound of formula (I) is selected from a compound of formula (VII) 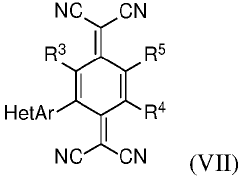 wherein
HetAr is selected from formula II 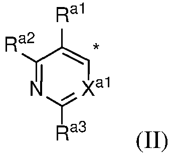
R³ and R⁴ are selected from H, D;
X^{a1} is selected from N or CR^{a4};
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position.

According to an embodiment, the compound of formula (I) is selected from a compound of formula (VII) 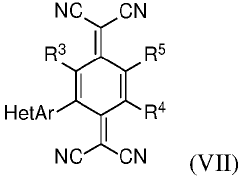 wherein
HetAr is selected from formula IIa 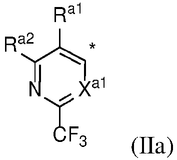
R³ and R⁴ are selected from H, D;
X^{a1} is selected from N or CR^{a4};
R^{a1}, R^{a2}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃,CN;
and wherein the asterisk "*" denotes the binding position.

According to an embodiment, the compound of formula (I) is selected from a compound of formula (VII) 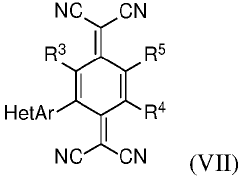 wherein
HetAr is selected from formula IIb 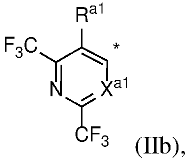
R³ and R⁴ are selected from H, D;
X^{a1} is selected from N or CR^{a4},
R^{a1}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN.
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position.

According to an embodiment, the compound of formula (I) is selected from a compound of formula (VII) 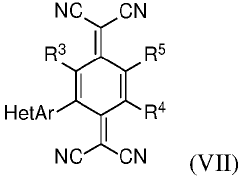 wherein
HetAr is selected from formula IIc 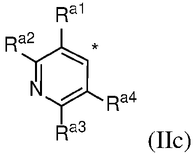
R³ and R⁴ are selected from H, D;
X^{a1} is selected from N or CR^{a4},
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position,

According to an embodiment, the compound of formula (I) is selected from a compound of formula (VII) 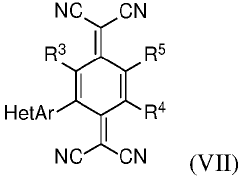 wherein
HetAr is selected from 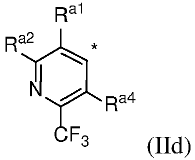
R³ and R⁴ are selected from H, D;
X^{a1} is selected from N or CR^{a4};
R^{a1}, R^{a2} and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, CN,
   wherein the one or more substituents on R^{a1} to R^{a4} are independently selected from D, partially fluorinated C₁ to C₈ alkyl or perfluorinated C₁ to C₈ alkyl, CF₃, , CN;
at least one of R^{a1} to R^{a4} is selected from partially fluorinated C₁ to C₈ alkyl or perfluorinated C₁ to C₈ alkyl, CF₃, CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position.

According to an embodiment, the compound of formula (I) is selected from a compound of formula (VII) 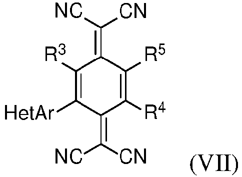 wherein
HetAr is selected from formula IIe 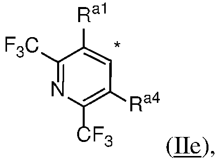
R³ and R⁴ are selected from H, D;
R^{a1}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position.

According to an embodiment, the compound of formula (I) is selected from a compound of formula (VII): 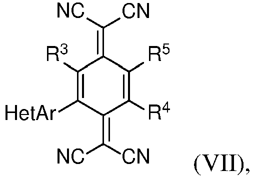 wherein
HetAr is selected from formulas (II), (IIa), (IIb), (IIc), (IId), (IIe), and (IIf): 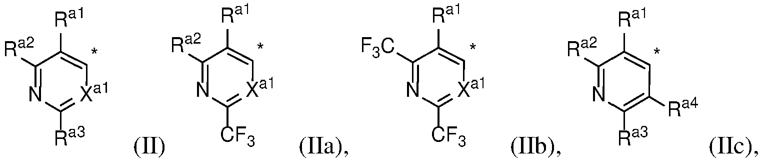 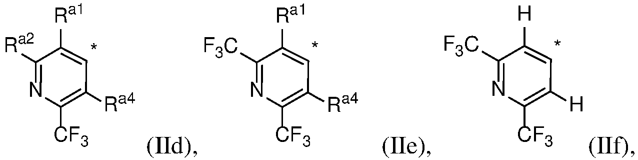 wherein the asterisk "*" denotes the binding position, and wherein
R³ and R⁴ are selected from H, D;
X^{a1} is selected from N or CR^{a4};
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, or CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, or CN;
R⁵ is selected from formula (VIa) 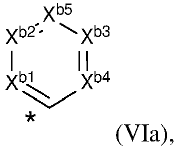 wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, or CN,
   wherein the one or more substituents on R^{b1} to R^{b5} are independently selected from D, partially fluorinated C₁ to C₈ alkyl, perfluorinated C₁ to C₈ alkyl, CF₃, or CN,
and wherein the asterisk "*" denotes the binding position.

According to an embodiment, the compound of formula (I) is selected from a compound of formula (VII) 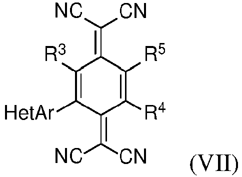 wherein
HetAr is selected from formula IIf 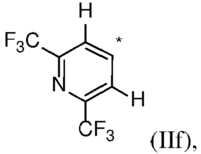
wherein formula IIf can be partially or fully deuterated;
R³ and R⁴ are selected from H, D,
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
   wherein the one or more substituents on R⁵ are independently selected from D, partially fluorinated C₁ to C₈ alkyl or perfluorinated C₁ to C₈ alkyl, CF₃, CN;
and wherein the asterisk "*" denotes the binding position.

According to an embodiment, the compound of formula (I) is selected from a compound of formula (VII) 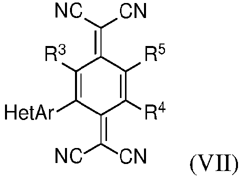 wherein
HetAr is selected from formula II 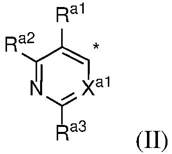
R³ and R⁴ are selected from H, D,
X^{a1} is selected from N or CR^{a4},
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 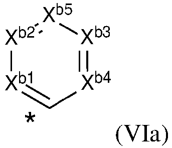
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 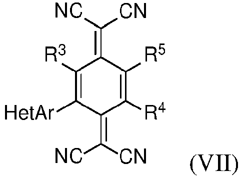 wherein
HetAr is selected from formula IIa 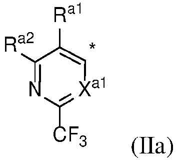
R³ and R⁴ are selected from H, D,
X^{a1} is selected from N or CR^{a4},
R^{a1} R^{a2}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 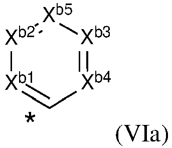
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 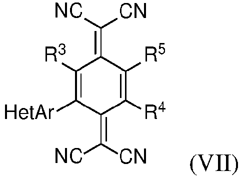 wherein
HetAr is selected from formula IIb 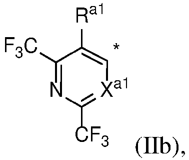
R³ and R⁴ are selected from H, D,
X^{a1} is selected from N or CR^{a4},
R^{a1}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 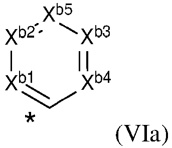
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 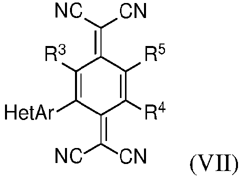 wherein
HetAr is selected from formula IIc 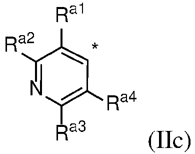
R³ and R⁴ are selected from H, D,
X^{a1} is selected from N or CR^{a4},
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 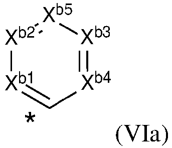
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 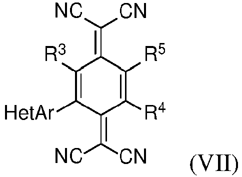 wherein
HetAr is selected from formula IId 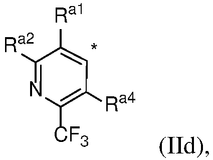
R³ and R⁴ are selected from H, D,
X^{a1} is selected from N or CR^{a4},
R^{a1}, R^{a2}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 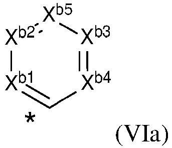
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 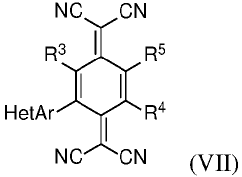 wherein
HetAr is selected from formula IIe 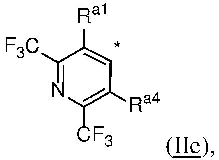
R³ and R⁴ are selected from H, D,
X^{a1} is selected from N or CR^{a4},
R^{a1}, and R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, CN;
R⁵ is selected from formula VIa 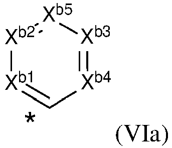
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

According to an embodiment, the compound of formula (I) is selected from a compound of formula (VII) 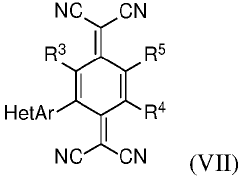 wherein
HetAr is selected from formula IIf 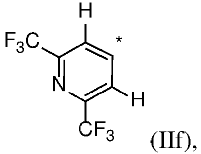
wherein formula IIf can be partially or fully deuterated;
R³ and R⁴ are selected from H, D,
R⁵ is selected from formula VIa 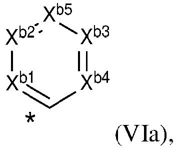
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, CN,
   wherein the one or more substituents on R^{b1} to R^{b5} are independently selected from D, partially fluorinated C₁ to C₈ alkyl, perfluorinated C₁ to C₈ alkyl, CF₃, CN,
and wherein the asterisk "*" denotes the binding position.

### Compound of formula (I)

According to one embodiment of the present invention, the compound of formula (I) is selected from a compound represented by I-1 to I-40:

| | | |
|---|---|---|
| 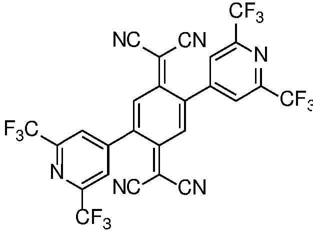 | 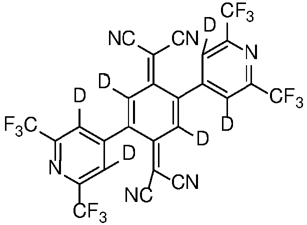 | 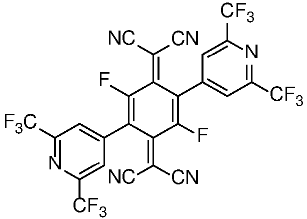 |
| I-1 | I-2 | I-3 |
| 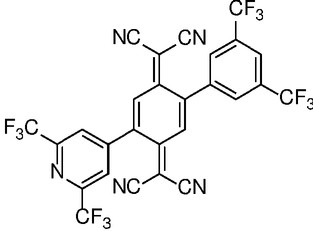 | 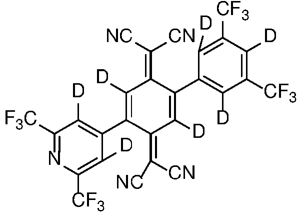 | 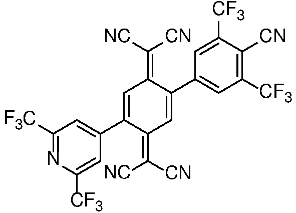 |
| I-4 | I-5 | I-6 |
| 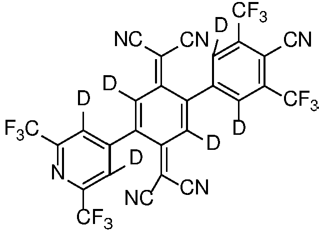 | 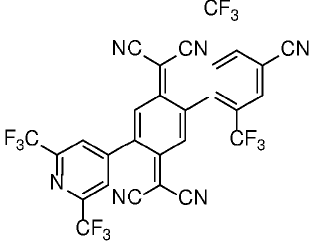 | 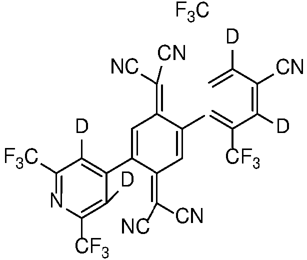 |
| I-7 | I-8 | I-9 |
| 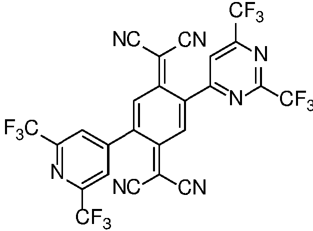 | 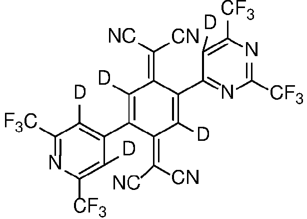 | 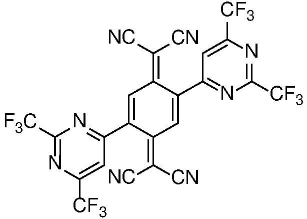 |
| I-10 | I-11 | I-12 |
| 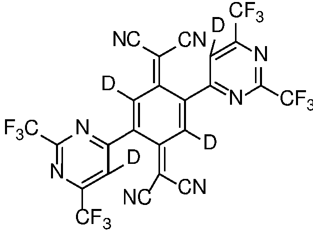 | 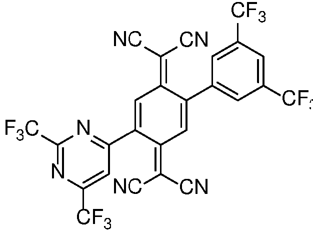 | 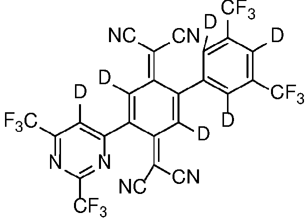 |
| I-13 | I-14 | I-15 |
| 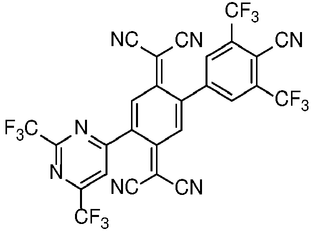 | 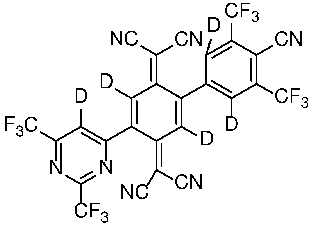 | 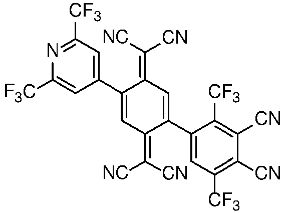 |
| I-16 | I-17 | I-18 |
| 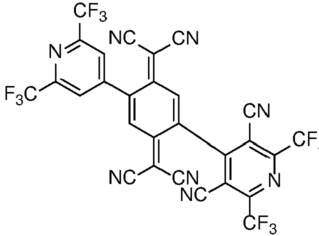 | 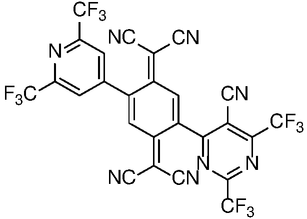 | 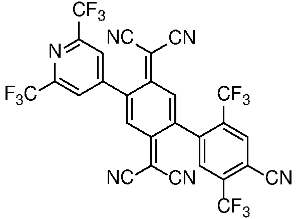 |
| I-19 | I-20 | I-21 |
| 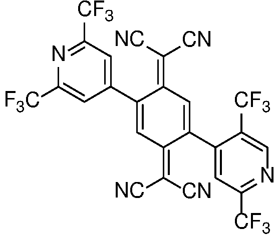 | 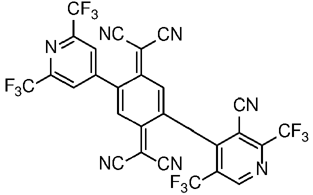 | 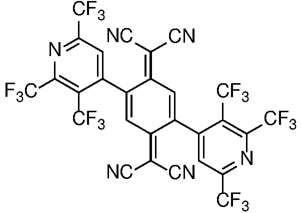 |
| I-22 | I-23 | I-24 |
| 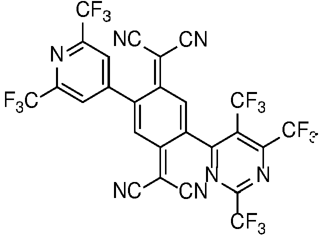 | 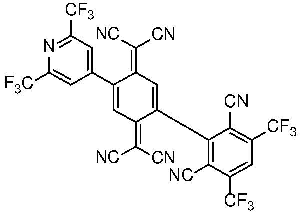 | 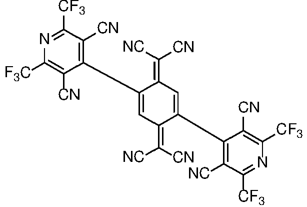 |
| I-25 | I-26 | I-27 |
| 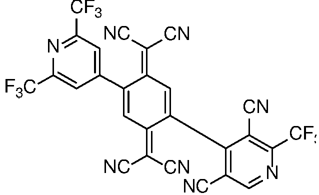 | 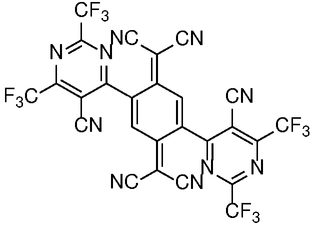 | 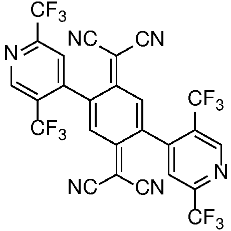 |
| I-28 | I-29 | I-30 |
| 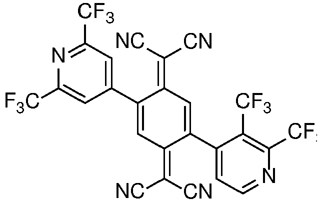 | 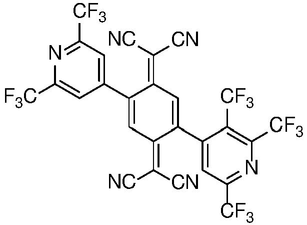 | 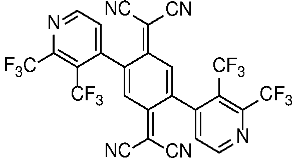 |
| I-32 | I-33 | I-34 |
| 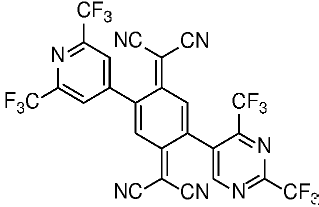 | 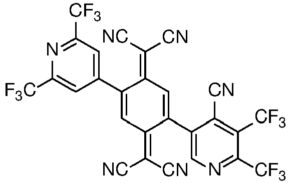 | 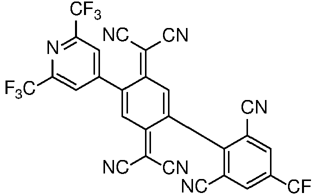 |
| I-35 | I-36 | I-37 |
| 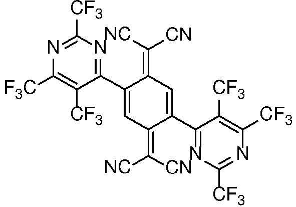 | 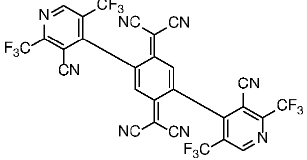 | 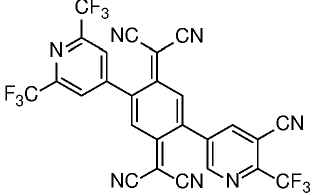 |
| I-38 | I-39 | I-40. |

According to one embodiment, the compounds I-4, I-8 and I-14 are less preferred. According to one embodiment, the compounds I-4, I-8 and I-14 may be excluded.

According to a preferred embodiment, wherein the compound of formula (I) is selected from I-1, I-6, 1-10, 1-12, I-16, I-18 to I-30, and I-32 to I-40:

| | | |
|---|---|---|
| 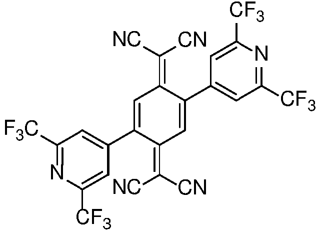 | 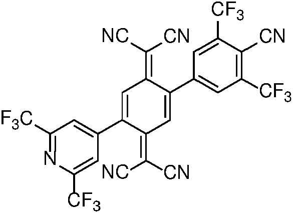 | 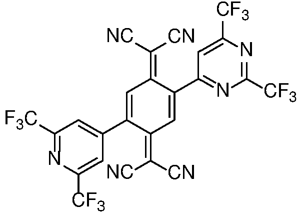 |
| I-1 | I-6 | I-10 |
| 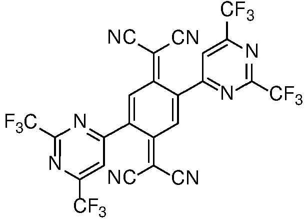 | 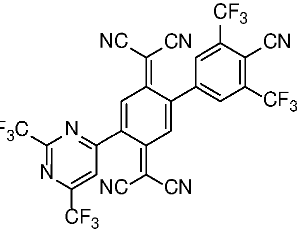 | 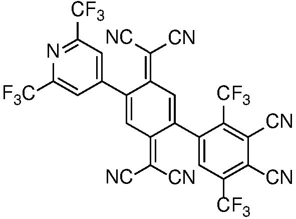 |
| I-12 | I-16 | I-18 |
| 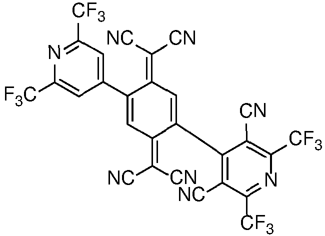 | 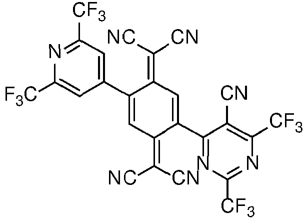 | 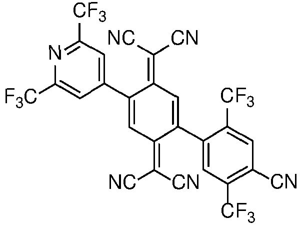 |
| I-19 | I-20 | I-21 |
| 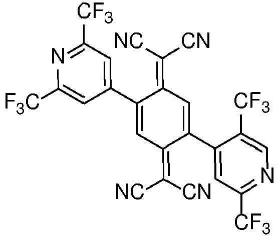 | 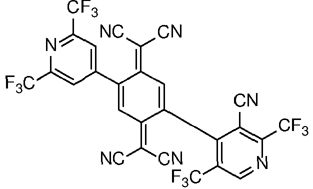 | 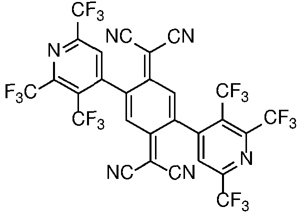 |
| I-22 | I-23 | I-24 |
| 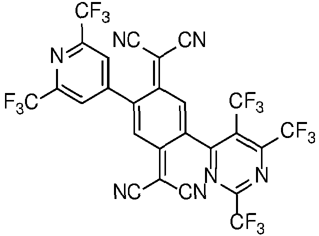 | 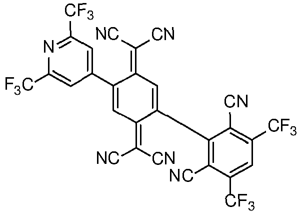 | 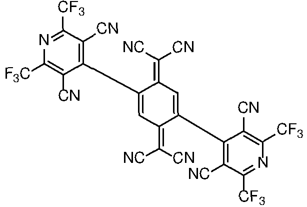 |
| I-25 | I-26 | I-27 |
| 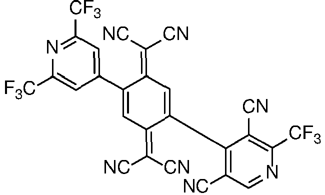 | 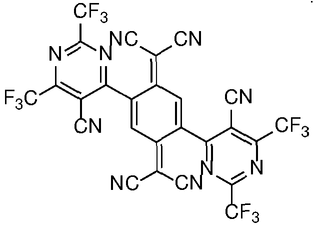 | 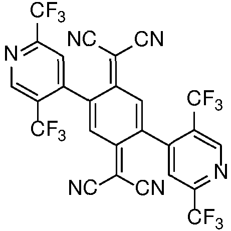 |
| I-28 | I-29 | I-30 |
| 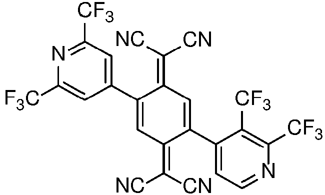 | 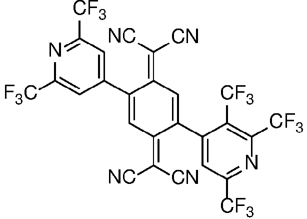 | 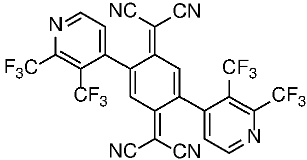 |
| I-32 | I-33 | I-34 |
| 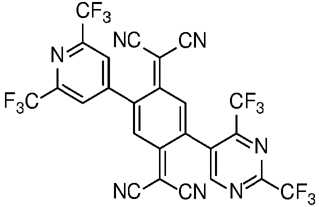 | 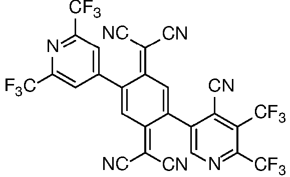 | 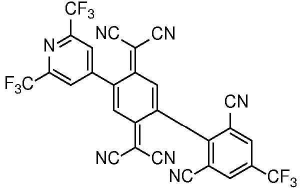 |
| I-35 | I-36 | I-37 |
| 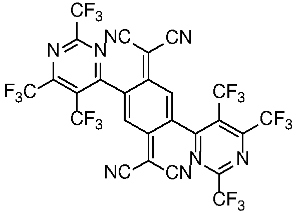 | 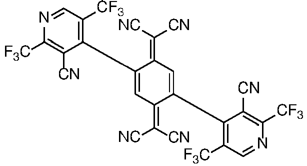 | 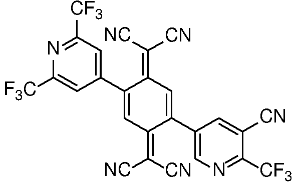 |
| I-38 | I-39 | I-40 |

According to a more preferred embodiment, wherein the compound of formula (I) is selected from I-1, 1-12, I-24, I-27, I-30, I-34, I-38 and I-39:

| | | |
|---|---|---|
| 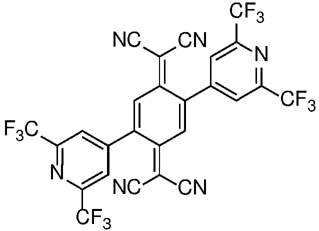 | 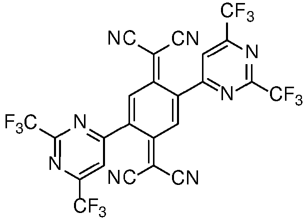 | 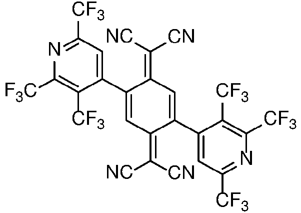 |
| I-1 | I-12 | I-24 |
| 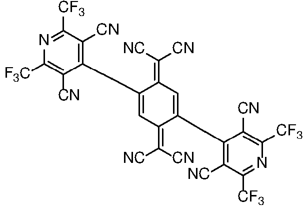 | 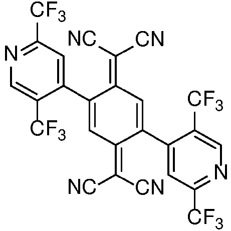 | 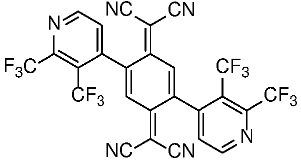 |
| I-27 | I-30 | I-34 |
| 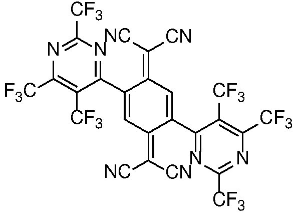 | 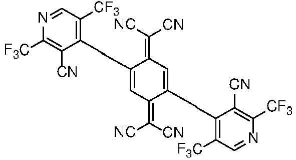 | |
| I-38 | I-39 | |

According to a most preferred embodiment, wherein the compound of formula (I) is selected from I-1, I-24, I-27, I-30, I-34 and I-39:

| | | |
|---|---|---|
| 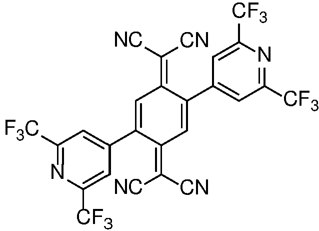 | 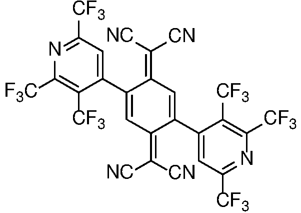 | 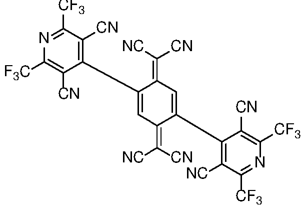 |
| I-1 | I-24 | I-27 |
| 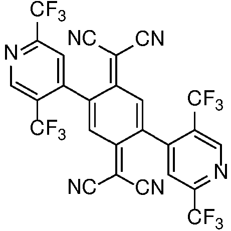 | 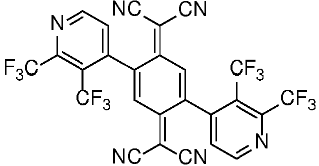 | 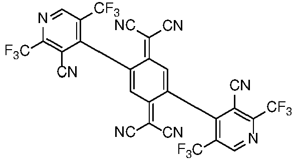 |
| I-30 | I-34 | I-39 |

### Organic semiconductor layer

Another aspect of the present invention is directed to an organic semiconductor layer, comprising the compound of formula (I) and wherein the compound of formula (I) is preferably the compound of formula (VII).

According to an embodiment, the organic semiconductor layer comprises the compound of formula (I), and wherein the compound of formula (I) is preferably the compound of formula (VII), and a hole transport matrix compound.

According to an embodiment, the organic semiconductor layer is a hole injection layer or a p-type charge generation layer.

The present invention furthermore relates to an organic semiconductor layer, whereby the organic semiconductor layer comprises a compound according to formula (VII), preferably selected from a compound I-1 to 1-40.

In case the organic semiconductor layer comprises a compound according to the invention, throughout this application text the term "compound of formula (I)" shall also intend to include a composition, wherein the composition comprises at least one compound according to the invention as described above.

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer may further comprise at least one matrix compound, also named covalent matrix compound, or substantially covalent matrix compound.

### Organic electronic device

Another aspect of the present invention is directed to an organic electronic device, wherein the organic electronic device comprises the organic semiconductor layer comprising the compound of formula (I) and wherein the compound of formula (I) is preferably the compound of formula (VII).

According to an embodiment, wherein the organic electronic device comprises an anode layer, a cathode layer, and at least one organic semiconductor layer, wherein the at least one organic semiconductor layer is arranged between the anode layer and cathode layer, and wherein the at least one organic semiconductor layer is the organic semiconductor layer according to the invention.

According to an embodiment, wherein the at least one organic semiconductor layer is a hole injection layer and/or a p-type charge generation layer.

According to an embodiment, wherein the hole injection layer is in direct contact to the anode layer.

According to an embodiment, wherein the hole injection layer is in direct contact to the anode layer, and the anode layer is in direct contact with a substrate, wherein the substrate is selected from a glass substrate, a plastic substrate, a metal substrate or a backplane.

According to an embodiment, the organic electronic device comprises an anode layer, a cathode layer, and at least one organic semiconductor layer, and at least one photoactive layer; wherein the at least one organic semiconductor layer is arranged between the anode layer and cathode layer, and wherein the at least one organic semiconductor layer is the organic semiconductor layer according to the invention; wherein the at least one organic semiconductor layer is arranged between the anode layer and the least one photoactive layer.

According to an embodiment, the organic electronic device comprises an anode layer, a cathode layer, a first photoactive layer, a second photoactive layer, at least one organic semiconductor layer, wherein the at least one organic semiconductor layer is an organic semiconductor layer according to the invention, wherein the at least one organic semiconductor layer is arranged between the anode layer and cathode layer, wherein the least one organic semiconductor layer is a hole injection layer and/or p-type charge generation layer, wherein the hole injection layer is arranged in direct contact to the anode layer, and wherein the p-type charge generation layer is arranged between the first photoactive layer and the second photoactive layer, wherein the hole injection layer is closer to the anode layer than the p-type charge generation layer.

According to an embodiment, the organic electronic device comprises an anode layer, a cathode layer, a first photoactive layer, a second photoactive layer, a hole injection layer, a p-type charge generation layer, wherein the hole injection layer is arranged in direct contact to the anode layer, and wherein the p-type charge generation layer is arranged between the first photoactive layer and the second photoactive layer, wherein the hole injection layer is closer to the anode layer than the p-type charge generation layer, wherein the hole injection layer or the p-type charge generation layer comprises a compound of formula (I) and wherein the compound of formula (I) is preferably the compound of formula (VII), and wherein the first photoactive layer, the second photoactive layer, the hole injection layer, the p-type charge generation layer are arranged between the anode layer and cathode layer.

According to an embodiment, the organic electronic device comprises an anode layer, a cathode layer, a first photoactive layer, a second photoactive layer, a hole injection layer, a p-type charge generation layer, wherein the hole injection layer is arranged in direct contact to the anode layer, and wherein the p-type charge generation layer is arranged between the first photoactive layer and the second photoactive layer, wherein the hole injection layer is closer to the anode layer than the p-type charge generation layer, wherein the hole injection layer or the p-type charge generation layer is an organic semiconductor layer according to the invention, wherein the first photoactive layer, the second photoactive layer, the hole injection layer, the p-type charge generation layer are arranged between the anode layer and cathode layer.

According to an embodiment, the organic electronic device comprises an anode layer, a cathode layer, at least two photoactive layer, at least one organic semiconductor layer, wherein the at least one organic semiconductor layer is an organic semiconductor layer according to the invention, wherein the at least one organic semiconductor layer is arranged between the anode layer and cathode layer, wherein the least one organic semiconductor layer is a hole injection layer and/or p-type charge generation layer, wherein the hole injection layer is arranged in direct contact to the anode layer, and wherein the p-type charge generation layer is arranged between the at least two photoactive layer wherein the hole injection layer is closer to the anode layer than the p-type charge generation layer.

According to an embodiment, the organic electronic device comprises an anode layer, a cathode layer, at least two photoactive layer, a hole injection layer, a p-type charge generation layer, wherein the hole injection layer is arranged in direct contact to the anode layer, and wherein the p-type charge generation layer is arranged between the at least two photoactive layer wherein the hole injection layer is closer to the anode layer than the p-type charge generation layer, wherein the hole injection layer or the p-type charge generation layer comprises a compound of formula (I), wherein the at least two photoactive layer the hole injection layer, the p-type charge generation layer are arranged between the anode layer and cathode layer.

According to an embodiment, the organic electronic device comprises an anode layer, a cathode layer, at least two photoactive layer, a hole injection layer, a p-type charge generation layer, wherein the hole injection layer is arranged in direct contact to the anode layer, and wherein the p-type charge generation layer is arranged between the at least two photoactive layer wherein the hole injection layer is closer to the anode layer than the p-type charge generation layer, wherein the hole injection layer or the p-type charge generation layer comprises a compound of formula (I) and wherein the compound of formula (I) is preferably the compound of formula (VII), wherein the at least two photoactive layer the hole injection layer, the p-type charge generation layer are arranged between the anode layer and cathode layer.

According to an embodiment, the organic electronic device comprises an anode layer, a cathode layer, at least two photoactive layer, a hole injection layer, a p-type charge generation layer, wherein the hole injection layer is arranged in direct contact to the anode layer, and wherein the p-type charge generation layer is arranged between the at least two photoactive layer, wherein the hole injection layer is closer to the anode layer than the p-type charge generation layer, wherein the hole injection layer or the p-type charge generation layer is an organic semiconductor layer according to the invention, wherein the at least two photoactive layer, the hole injection layer, the p-type charge generation layer are arranged between the anode layer and cathode layer.

According to one embodiment of the present invention, the hole injection layer is in direct contact with the anode layer and the anode layer is in direct contact with the substrate, wherein the substrate is selected from a glass substrate, a plastic substrate, a metal substrate, or a backplane.

According to one embodiment of the organic electronic device according to the invention whereby the anode layer comprises at least a first anode sub-layer and a second anode sub-layer.

According to a preferred embodiment, the photoactive layer is an emission layer preferably a light emitting layer.

According to an embodiment, wherein the organic electronic device is an electroluminescent device, organic electroluminescent device, an organic light emitting diode (OLED), a light-emitting device, thin film transistor, a battery, a display device, or an organic photovoltaic cell (OPV).

According to a preferred embodiment, the organic electronic device is an organic electroluminescent device.

### Display device

Another aspect of the present invention is directed to a display device comprising an organic electronic device, wherein the organic electronic device comprises an organic semiconductor layer, wherein the organic semiconductor layer comprises a compound of formula (I), and wherein the compound of formula (I) is preferably the compound of formula (VII).

According to an embodiment, wherein the organic electronic device comprises an organic semiconductor layer according to the invention.

### Substantially covalent matrix compound

The organic semiconductor layer may further comprises a covalent matrix compound also named substantially covalent matrix compound. According to one embodiment the substantially covalent matrix compound may be selected from at least one organic compound. The substantially covalent matrix may consists substantially of covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially of covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (VIII) or a compound of formula (IX)

According to another aspect of the present invention, the at least one matrix compound, also referred to as "substantially covalent matrix compound", may comprises at least one arylamine compound, diarylamine compound, triarylamine compound, a compound of formula (VIII) or a compound of formula (IX) 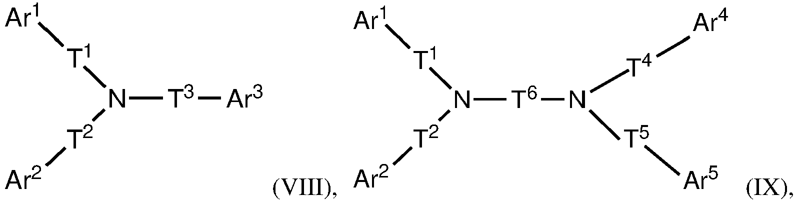 wherein:
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings may be selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
   wherein the substituents of Ar¹, Ar², Ar³, Ar⁴ and Ar⁵ may be selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings may be selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
   wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from K1 to K16: 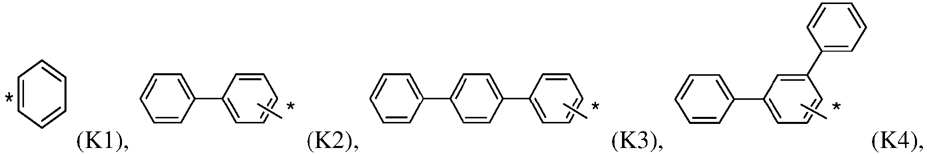 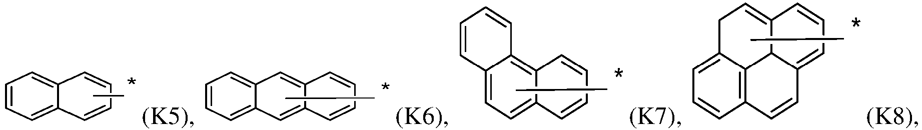 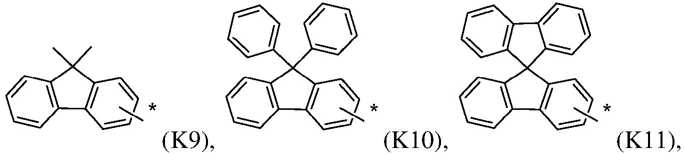 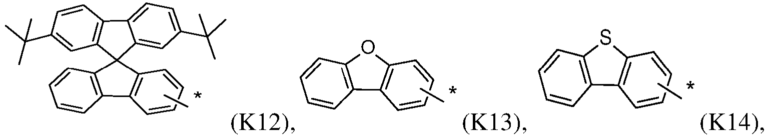 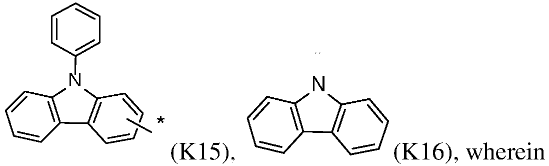 the asterix "*" denotes the binding position.

According to an embodiment, wherein Ar¹, Ar², Ar³, Ar⁴ and Ar⁵ may be independently selected from K1 to K15; alternatively selected from K1 to K10 and K13 to K15.

According to an embodiment, wherein Ar¹, Ar², Ar³, Ar⁴ and Ar⁵ may be independently selected from the group consisting of K1, K2, K5, K7, K9, K10, K13 to K16.

The rate onset temperature may be in a range particularly suited to mass production, when Ar¹, Ar², Ar³, Ar⁴ and Ar⁵ may be selected in this range.

The "matrix compound of formula (VIII) or formula (IX)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents may be independently selected from methyl, phenyl or fluorenyl.

According to an embodiment of the electronic device, wherein the matrix compound of formula (VIII) or formula (IX) may be selected from L1 to L21:

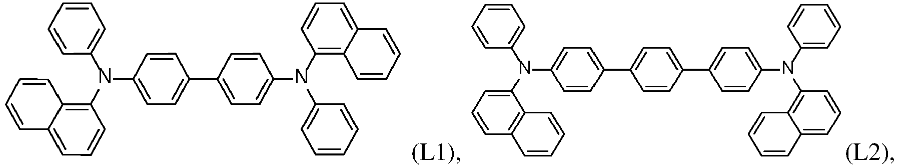

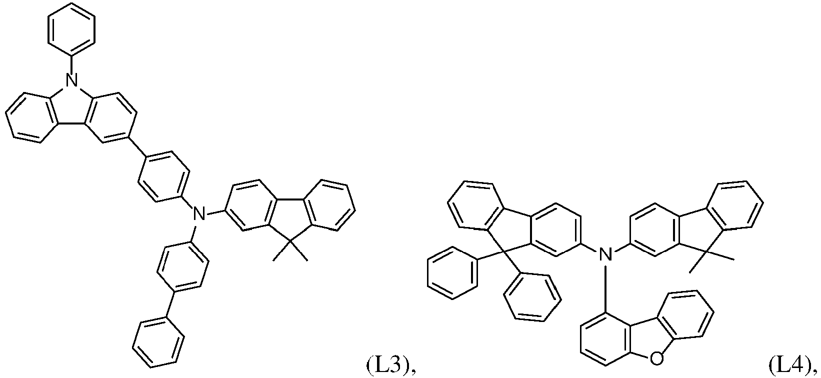

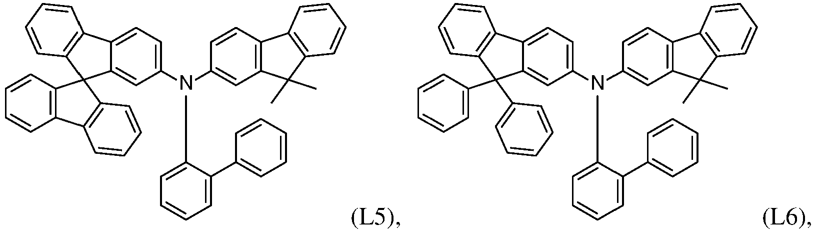

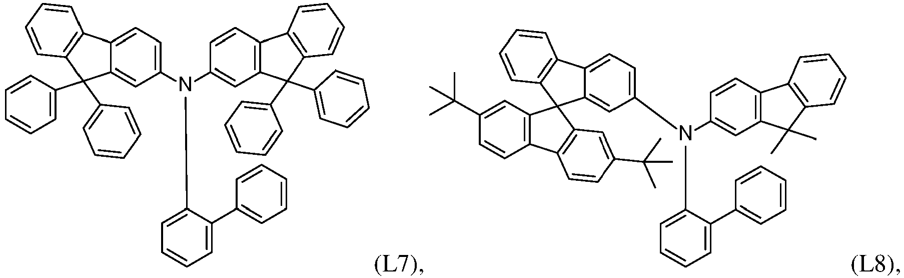

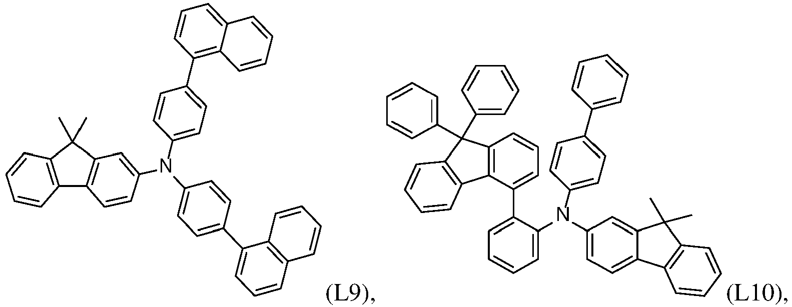

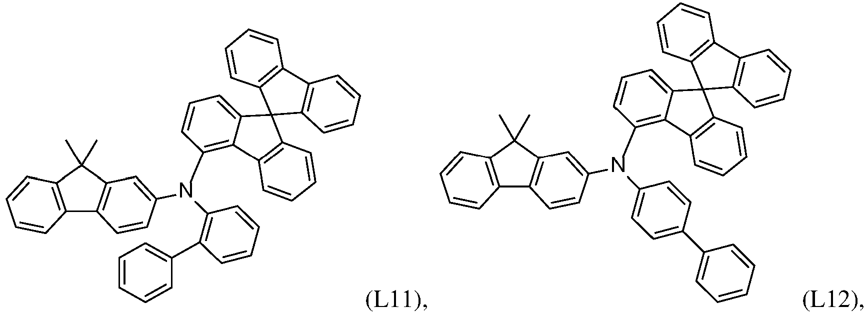

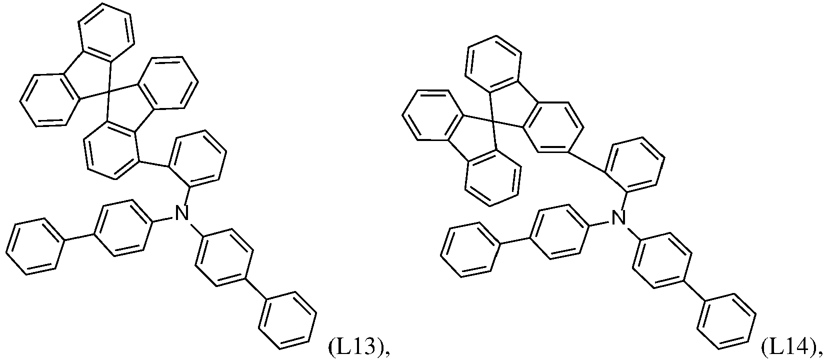

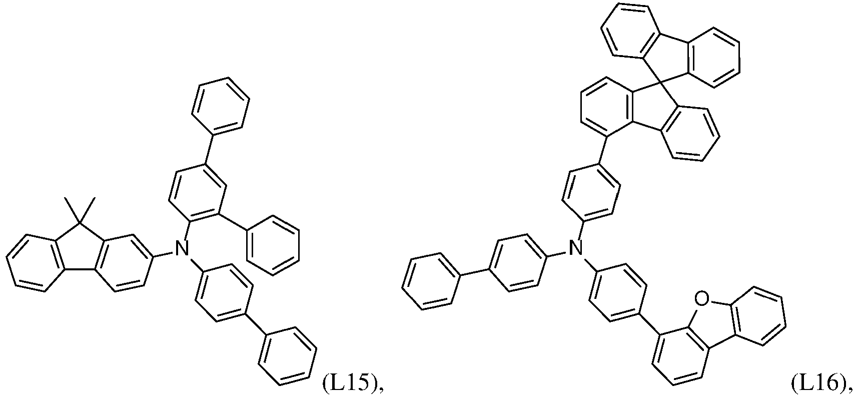

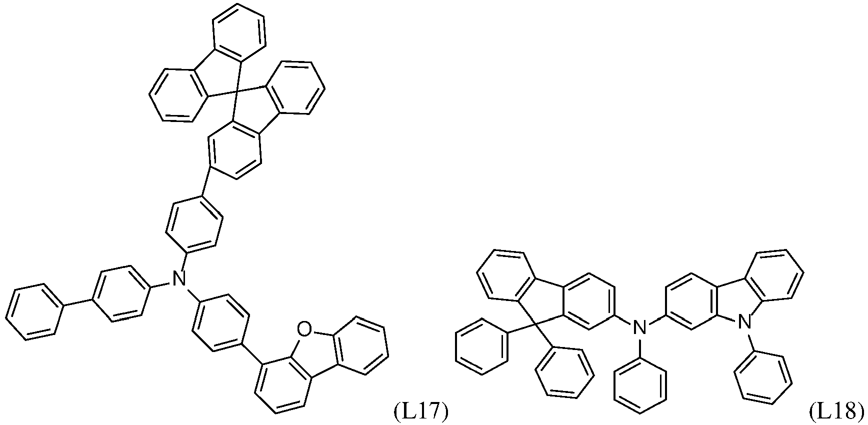

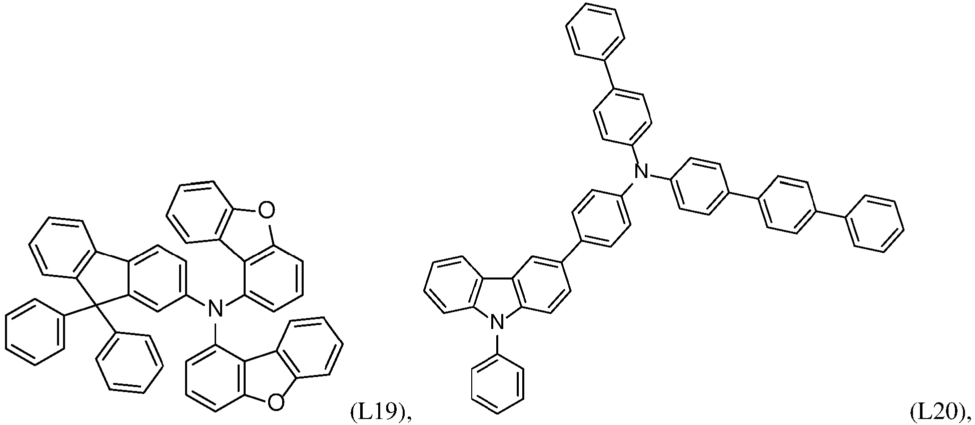

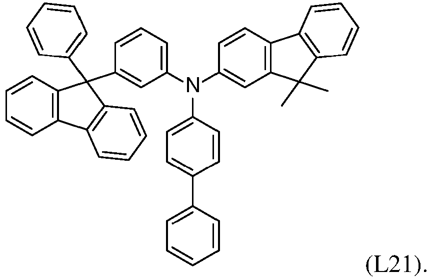

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to one embodiment of the present invention, the anode layer comprises a first anode sub-layer and a second anode sub-layer, wherein
- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the anode layer comprises a first anode sub-layer comprising of Ag, a second anode sub-layer comprising of transparent conductive oxide, preferably ITO, and a third anode sub-layer comprising of transparent conductive oxide, preferably ITO; wherein the first anode sub-layer is arranged between the second and the third anode sub-layer.

### Hole transport layer

According to one embodiment of the present invention, the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to one embodiment of the present invention, the hole transport layer may comprise a compound of formula (VIII) or (IX) as described above.

According to one embodiment of the present invention, the hole injection layer and the hole transport layer comprises the same substantially covalent matrix compound as described above.

According to one embodiment of the present invention, the hole injection layer and the hole transport layer comprises the same compound of formula (VIII) or (IX) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Charge generation layer

The organic electronic device according to the present invention may further comprise a charge generation layer, wherein the charge generation layer comprises a p-type charge generation layer, wherein the p-type charge generation layer is an organic semiconductor layer according to the invention, wherein the charge generation layer is arranged between two photoactive layer.

The organic electronic device according to the present invention may further comprise a charge generation layer, wherein the charge generation layer comprises a p-type charge generation layer, wherein the p-type charge generation layer is an organic semiconductor layer according to the invention, wherein the p-type charge generation layer is arranged closer to the cathode layer.

The organic electronic device according to the present invention may further comprise a charge generation layer, wherein the charge generation layer comprises a p-type charge generation layer, wherein the p-type charge generation layer is an organic semiconductor layer according to the invention, wherein the charge generation layer is arranged between two photoactive layer.

The charge generation layer may further comprise an n-type charge generation layer, wherein the n-type charge generation layer is arranged between the p-type charge generation layer and the anode layer, and wherein the p-type charge generation layer is arranged closer to the cathode layer than the n-type charge generation layer.

Preferably, the n-type charge generation layer and the p-type charge generation layer are arranged in direct contact.

The thickness of the n-type charge generation layer may be in the range from about 0.5 nm to about 15 nm, for example, in the range from about 1 nm to about 10 nm. When the thickness of the n-type charge generation layer is within this range, the EIL may have satisfactory electron-injecting properties, without a substantial penalty in driving voltage.

The n-type charge generation layer may comprise a metal dopant, wherein the metal dopant is selected from an alkali metal, alkaline earth metal or rare earth metal.

The n-type charge generation layer may comprise an azine compound. According to a preferred embodiment, the nCGL may comprise an azine compound and a metal dopant, wherein the metal dopant is selected from an alkali metal, alkaline earth metal or rare earth metal.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Organic light-emitting diode (OLED)

The organic electronic device according to the invention may be an organic light-emitting device.

According to an embodiment, wherein the organic light-emitting device comprises a substrate, an anode layer, formed on the substrate, a hole injection layer comprising a compound of formula (I), a hole transport layer, an emission layer, an electron transport layer, and a cathode layer.

According to an embodiment, wherein the organic light-emitting device comprises a substrate, an anode layer, formed on the substrate, a hole injection layer comprising a compound of formula (I), a hole transport layer, an electron blocking layer, an emission layer, a hole blocking layer, an electron transport layer, and a cathode layer.

According to an embodiment, wherein the organic light-emitting device comprises a substrate, an anode layer, formed on the substrate, a hole injection layer comprising a compound of formula (I), a hole transport layer, an electron blocking layer, an emission layer, a hole blocking layer, an electron transport layer, an electron injection layer and a cathode layer.

According to an embodiment, the organic light-emitting device may comprise a substrate an anode layer, a hole injection layer, a first hole transport layer, a first electron blocking layer, the first electron blocking layer, a first emission layer, a first electron transport layer, a n-type charge generation layer, a p-type charge generation layer, a second hole transport layer, the a second electron blocking layer, second emission layer, a cathode layer, wherein between the second emission layer and the cathode layer an optional hole blocking layer, optional electron transport layer and/or an optional electron injection layer are arranged.

The organic semiconductor layer according to the invention may be the first hole injection layer and/or the p-type charge generation layer.

According to an embodiment, the organic light-emitting device may comprise a layer structure of a substrate that is adjacent arranged to an anode layer, the anode layer is adjacent arranged to a hole injection layer, the hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generation layer, the p-type charge generation layer is adjacent arranged to a second hole transport layer, the second hole transport layer is adjacent arranged to a second electron blocking layer, the second electron blocking layer is adjacent arranged to a second emission layer, a cathode layer, wherein between the second emission layer and the cathode layer an optional hole blocking layer, optional electron transport layer and/or an optional electron injection layer are arranged.

The organic semiconductor layer according to the invention may be the first hole injection layer and/or the p-type charge generation layer.

### Method of manufacturing an organic light-emitting device

According to one aspect of the present invention, there is provided an organic light-emitting diode (OLED) comprising: a substrate; an anode layer formed on the substrate; a hole injection layer comprising a compound of formula (I), a hole transport layer, an emission layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer, an electron injection layer, and a cathode layer.

According to various embodiments of the present invention, there may be provided OLEDs layers arranged between the above-mentioned layers, on the substrate or on the top layer.

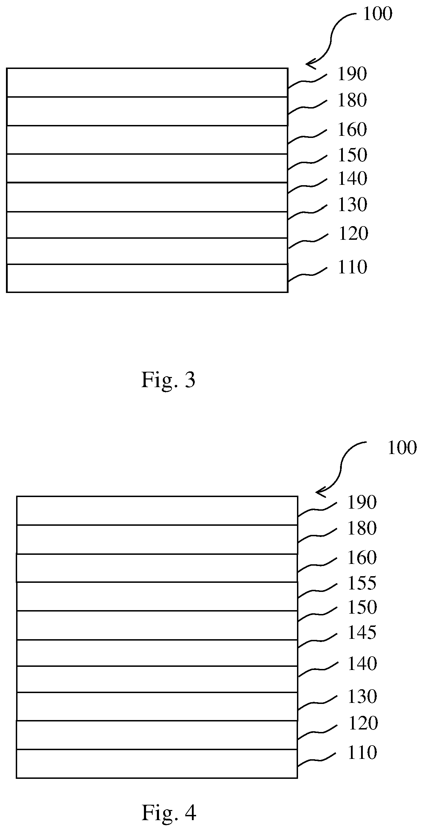

For example, the OLED according to Fig. 3 may be formed by a process, wherein on a substrate (110), an anode (120), a hole injection layer (130) which may comprise compound of formula (I), a hole transport layer (140), an electron blocking layer (145), an emission layer (150), a hole blocking layer (155), an electron transport layer (160), an electron injection layer (180) and the cathode layer (190) are subsequently formed in that order.

According to one aspect, the OLED may comprise a layer structure of a substrate that is adjacent arranged to an anode layer, the anode layer is adjacent arranged to a hole injection layer, the hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generation layer, the p-type charge generation layer is adjacent arranged to a second hole transport layer, the second hole transport layer is adjacent arranged to a second electron blocking layer, the second electron blocking layer is adjacent arranged to a second emission layer, between the second emission layer and the cathode layer an optional electron transport layer and/or an optional injection layer are arranged.

The organic semiconductor layer according to the invention may be the first hole injection layer and/or the p-type charge generation layer.

### Method of manufacturing an organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound;
   the method comprising the steps of forming the hole injection layer; whereby for an organic light-emitting diode (OLED):
- the hole injection layer is formed by releasing the compound of formula (I) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole blocking layer, and an emission layer between the anode layer and the first electron transport layer.

According to various embodiments of the present invention, the method may further include the steps for forming an organic light-emitting diode (OLED), wherein
- on a substrate an anode layer is formed,
- on the anode layer a hole injection layer comprising a compound of formula (I) is formed,
- on the hole injection layer comprising a compound of formula (I) a hole transport layer is formed,
- on the hole transport layer an emission layer is formed,
- on the emission layer an electron transport layer is formed, optionally a hole blocking layer is formed on the emission layer,
- and finally, a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, hole injection layer comprising a compound of formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
- FIG. 1: is a schematic sectional view of an organic electronic device, according to an exemplary embodiment of the present invention;
- FIG. 2: is a schematic sectional view of an organic electronic device, according to an exemplary embodiment of the present invention;
- FIG. 3: is a schematic sectional view of an organic electronic device , according to an exemplary embodiment of the present invention;
- FIG. 4: is a schematic sectional view of an OLED, according to an exemplary embodiment of the present invention.
- FIG. 5: is a schematic sectional view of an OLED, according to an exemplary embodiment of the present invention.
- FIG. 6: is a schematic sectional view of an OLED, according to an exemplary embodiment of the present invention.
- FIG. 7: is a schematic sectional view of an OLED, according to an exemplary embodiment of the present invention.

Hereinafter, the figures are illustrated in more detail with reference to examples.

However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

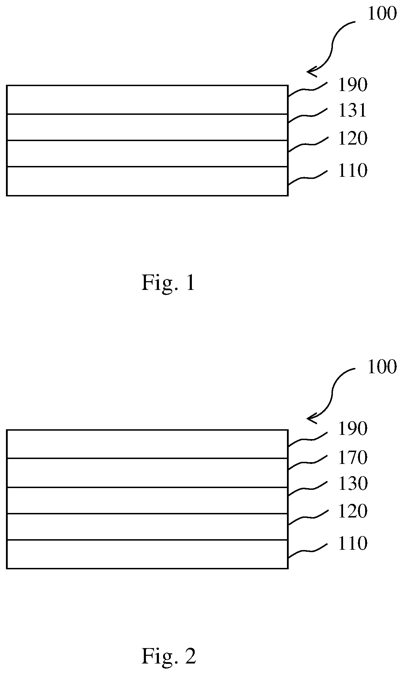

FIG. 1 is a schematic sectional view of an organic electronic device 100, according to an exemplary embodiment of the present invention. The organic electronic device 100 includes an anode layer 120 and an organic semiconductor layer 131 which may comprise a compound of formula (I). The organic semiconductor layer 131 is disposed on the anode layer 120. Onto the organic semiconductor layer 131 a cathode layer 190 are disposed.

FIG. 2 is a schematic sectional view of an organic electronic device 100, according to an exemplary embodiment of the present invention. The organic electronic device 100 includes a substrate 110, an anode layer 120 and a hole injection layer (HIL) 130 which may comprise a compound of formula (I). The HIL 130 is disposed on the anode layer 120. Onto the HIL 130, a photoactive layer (PAL) 170 and a cathode layer 190 are disposed.

FIG. 3 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

FIG. 4 is a schematic sectional view of an OLED 100, according to another exemplary embodiment of the present invention. Fig. 4 differs from Fig. 3 in that the OLED 100 of Fig. 4 comprises an electron blocking layer (EBL) 145 and a hole blocking layer (HBL) 155.

Referring to Fig. 4, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

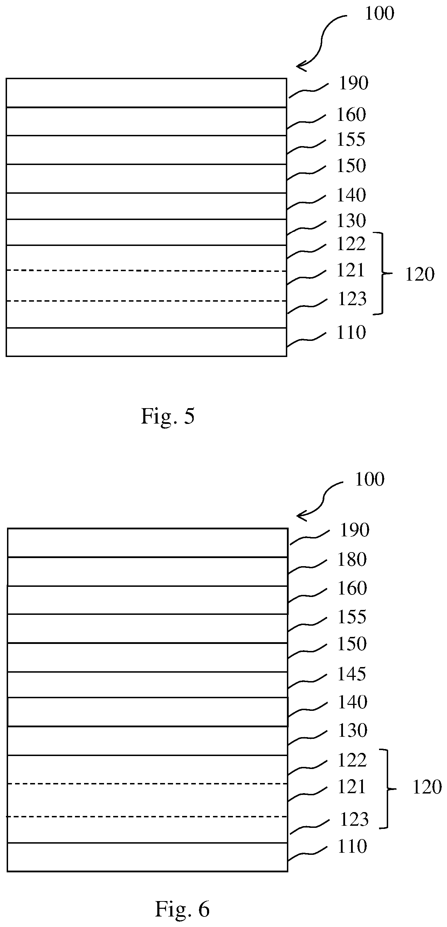

FIG. 5 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a compound of formula (I).

FIG. 6 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a compound of formula (I).

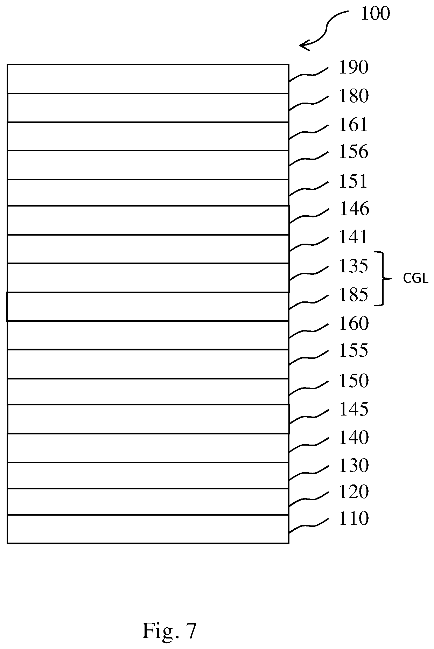

FIG. 7 is a schematic sectional view of an organic electronic device 100, according to an exemplary embodiment of the present invention. The organic electronic device 100 includes a substrate 110, an anode layer 120, a hole injection layer (HIL) 130, a first hole transport layer (HTL) 140, a first electron blocking layer (EBL1) 145, a first emission layer (EML1) 150, an optional first hole blocking layer (HBL) 155, a first electron transport layer (ETL1) 160, an n-type charge generation layer (n-CGL) 185, a p-type charge generation layer (p-GCL) 135 which may comprise compound of formula (I), a second hole transport layer (HTL2) 141, a second electron blocking layer (EBL2) 146, a second emission layer (EML2) 151, an optional second hole blocking layer (HBL2) 156, a second electron transport layer (ETL2) 161, an electron injection layer (EIL) 180 and a cathode layer 190. The HIL may also comprise a compound of formula (I). The hole injection layer may comprise compound of formula (I). The anode layer may comprise a first anode sub-layer, a second anode sub-layer, and an optional third anode sub-layer.

While not shown in Fig. 1 to Fig. 7, a capping and/or sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Synthesis

The invention is furthermore illustrated by the following examples which are illustrative only and non-binding.

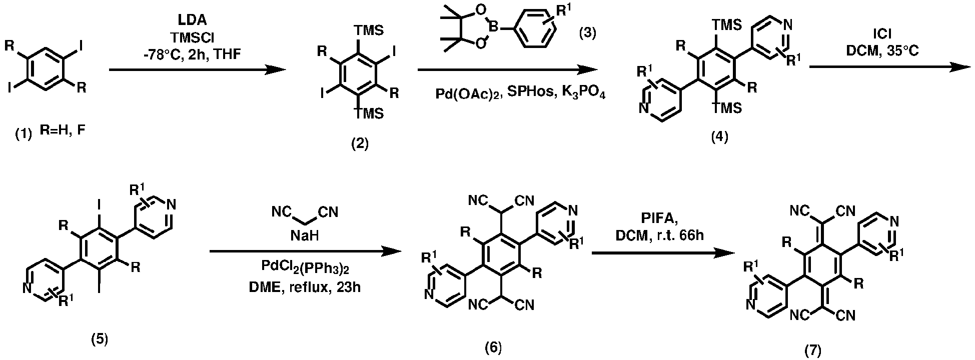

### Intermediate (2)

A schlenk-flask charged with a magnetic stirrer and diiodobenzene (1) was evacuated and filled with inert gas. Tetrahydrofurane and 2.2 equivalents Trimethylsilylchloride were added, and the reaction mixture was cooled to -78 °C. 2.2 equivalents Lithium diisopropylamine were added dropwise over 15 minutes and the solution was stirred for 1 h at -78 °C and then gradually warmed to room temperature. The reaction was quenched with diluted sulfuric acid. Organic and aqueous layers were separated, and the aqueous phase was extracted with diethyl ether. The combined organic layers were dried with sodium sulfate and the solvents were evaporated. The crude orange solid was crystallized from Dichloromethane/Methanol 1:1 to give the pure product.

### Intermediate (4)

An apparatus charged with a magnetic stirrer, intermediate (2), 3 equivalents pinacolester, 10 mol% SPhos and 4 equivalents potassium phosphate were suspended in a 10:1 mixture of Dioxane/water. The mixture was degassed using sonification for 15 minutes, before 5 mol% palladium (II) acetate were added. After stirring for 45 hours at reflux under inert gas atmosphere, the mixture was cooled down to room temperature, diluted with ethyl acetate and water. After separating layers, the aqueous phase was extracted with ethyl acetate and the combined organic layers were dried over sodium sulfate, filtrated over a pad of celite and the solvents were evaporated. The crude product was purified by trituration in hexane overnight, filtration and washing with cooled hexane.

### Intermediate (5)

Intermediate (4) was dissolved in dichloromethane in a ratio of 6 ml/g, 4 equivalents of iodinechloride were added and the solution was stirred at 35°C for 23 hours. After cooling down the reaction to room temperature and subsequently in the freezer, the precipitate was filtered off and washed with cold dichloromethane. The product was used without further purification.

### Intermediate (6)

A flask charged with a magnetic stirrer and 4 equivalents malonitrile was evacuated and filled with inert gas. After addition of DME, the solution was cooled down to 0°C and 6 equivalents sodium hydride were added over the course of 15 Minutes. The mixture was stirred at 0°C and room temperature for 15 minutes each and intermediate (5) and Pd(PPh₃)₂Cl₂ were added. After stirring at reflux overnight, the mixture was acidified with 1M hydrochloric acid to precipitate the product. The crude product was filtrated, washed with water, dried to constant weight, dissolved in acetone, and filtrated over a pad of Celite and silica gel on top. After trituration in dichloromethane, the product was cooled in the freezer before it was filtered of the mother liquor. The solid was washed with cold dichloromethane until the filtrate was clear to give the pure product.

### Quinone (7)

A flask with a magnetic stirrer was charged with intermediate (6), evacuated and flushed with argon. After addition of dichloromethane and 1.15 equivalents PIFA, flask was covered with aluminium foil and stirred at rt for three days. The solution was concentrated, and acetic acid was added in a ratio of DCM/HOAc 4:1. After stirring for one hour at room temperature and 30 minutes at 10°C, the product was filtered of and washed with cold dichloromethane to give the final product in high purity.

### Calculated HOMO and LUMO

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a 6-31G* basis set from the optimized geometries obtained by applying the functional BP86 with a Def2-SVP basis set in the gas phase. All the calculations were performed in the gas phase. If more than one conformation is viable, the conformation with the lowest total energy is selected. For molecules containing a deuterium atom (D), a mass of 2.00141 amu (atomic mass unit) has been assigned to the specific atom.

### Melting point

The melting point (Tm) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Thermogravimetric analysis

The term "TGA5%" denotes the temperature at which 5 % weight loss occurs during thermo gravimetric analysis and is measured in °C.

The TGA5% value may be determined by heating a 9-11 mg sample in a thermogravimetric analyzer at a heating rate of 10 K/min in an open 100 µL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

According to one embodiment, the TGA5% value of compound of formula (I) is selected in the range of ≥ 280 °C and ≤ 420 °C; preferably of ≥ 290 °C and ≤ 410 °C, also preferred of ≥ 295 °C and ≤ 410 °C.

### Rate onset temperature

The rate onset temperature (T_{RO}) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 185 to 280 °C. If the rate onset temperature is below 185 °C the evaporation may be too rapid and therefore difficult to control. If the rate onset temperature is above 280 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### Dipole moment

The dipole moment |*̅µ̅*̅| of a molecule containing N atoms is given by: $\vec{μ} = {\sum }_{i}^{N} {q}_{i} \vec{{r}_{ι}}$ $\left|\vec{μ}\right| = \sqrt{{μ}_{x}^{2} + {μ}_{y}^{2} + {μ}_{z}^{2}}$ where *qᵢ* and *̅r̅ᵢ̅*̅ are the partial charge and position of atom *i* in the molecule. The dipole moment was obtained from the optimized geometries at the same level of theory. The geometries of the molecular structures are optimized using the hybrid functional B3LYP with the 6-31G* basis set in the gas phase (program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany).). If more than one conformation is viable, the conformation with the lowest total energy is selected to determine the structural and electronic parameters of the molecules.

### Experimental data

### HV-TGA 5% mass loss temperature

10 mg compound are loaded into 2 ccm Ah03 crucible, which is mounted in high vacuum-thermo gravimetric analysis (HV-TGA) setup. The HV-TGA setup consists of an evaporation source (Creaphys DE-2-CF40), a thermocouple (Thermo Sensor GmbH NiCr-Ni, Typ K) placed inside the crucible and a quartz crystal microbalance (QCM, Inficon 750-1000-G10, 6 MHz). The HV-TGA setup is part of vacuum chamber system equipped with a scroll pump, a turbomolecular pump, a nitrogen inlet with mass flow controller and a progressive valve between scroll and turbomolecular pump. The combination of nitrogen inlet and pump valve allows pressures of 1 e 2 mbar to 1 e-6 mbar, whereas standard operational pressure is 1 e-4 mbar with a stability of +I- 10%. Subsequent to reaching desired pressure the evaporation source temperature is ramped from room temperature to 600 °C at a rate of 10 °C/min. The compound is fully evaporated and detected by the QCM. The frequency shift of the QCM during the whole temperature ramp corresponds to a mass loss of 100%.

The reference temperature for dopant materials is taken at a mass loss of 5% as the obtained values have closest match to processing temperature in linear evaporation sources at mass production.

**Table 1**

| Reference temperature for dopant materials according to formula I | | |
|---|---|---|
| Compound of formula I | | HV-TGA5% [°C] |
| 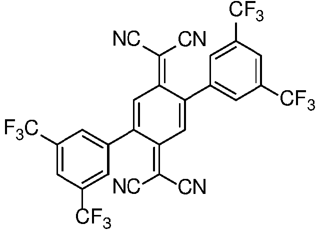 | Comparative compound C1 | 209 |
| 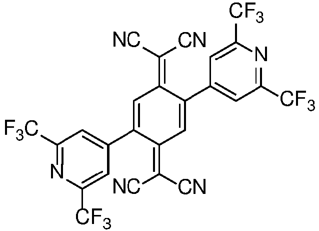 | Compound I-1 | 221 |

It is apparent from table 1 that the compound of the invention exhibits a higher HV-TGA5% value, and thus evaporate at a higher temperature. Thus, the high HV-TGA5% of the compounds of the invention may be beneficial in minimizing or avoiding tool contamination during the manufacturing of e.g., an organic electroluminescent device.

**Table 2**

| | | LUMO [eV] *¹ | HOMO [eV] *¹ | Egap [eV] *¹ | Dipole Moment [Debye] *¹ |
|---|---|---|---|---|---|
| Comparative compound C1 | 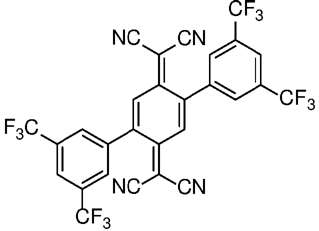 | -4.95 | -7.40 | 2.45 | 1.58 |
| I-1 | 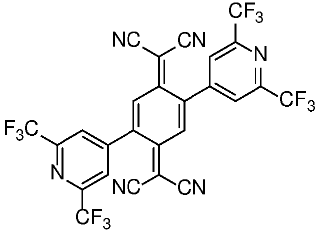 | -5.28 | -7.75 | 2.47 | 0.63 |
| I-3 | 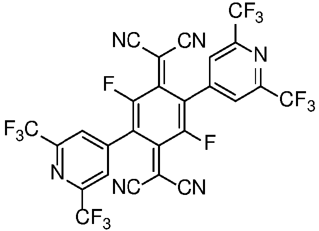 | -5.52 | -7.78 | 2.26 | 0.12 |
| I-4 | 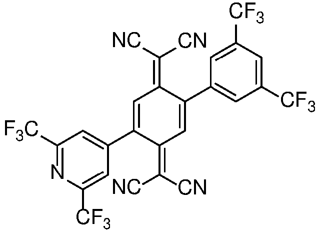 | -5.23 | -7.51 | 2.28 | 2.93 |
| I-6 | 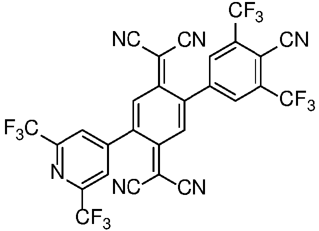 | -5.42 | -7.70 | 2.38 | 1.36 |
| I-8 | 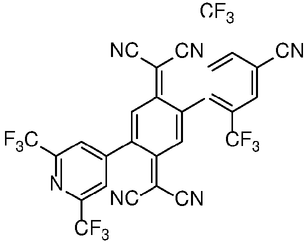 | -5.26 | -7.57 | 2.31 | 2.50 |
| I-10 | 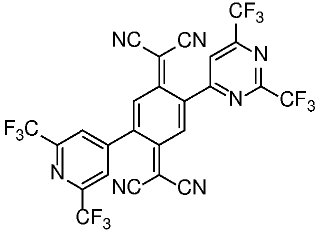 | -5.41 | -7.7 | 2.29 | 2.28 |
| I-12 | 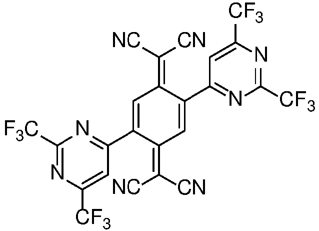 | -5.38 | -7.67 | 2.29 | 2.45 |
| I-14 | 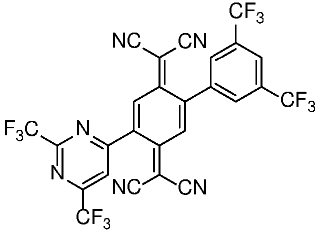 | -5.21 | -7.49 | 2.28 | 1.80 |
| I-16 | 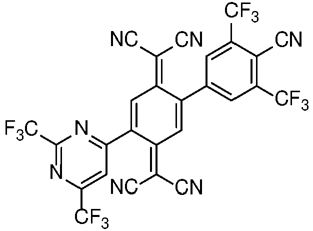 | -5.44 | -7.71 | 2.27 | 2.81 |
| I-18 | 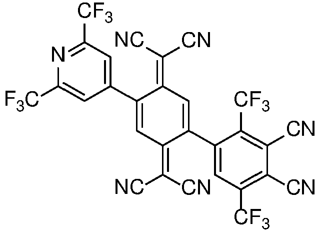 | -5.49 | -7.8 | 2.31 | 2.72 |
| I-19 | 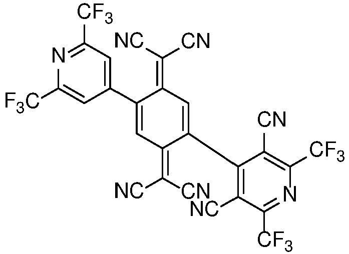 | -5.53 | -7.83 | 2.30 | 2.77 |
| I-20 | 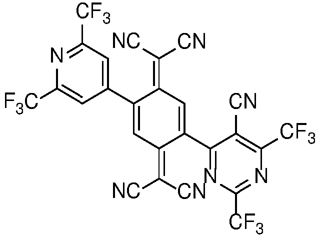 | -5.47 | -7.77 | 2.30 | 2.18 |
| I-21 | 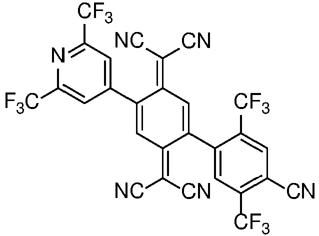 | -5.35 | -7.65 | 2.30 | 0.65 |
| I-22 | 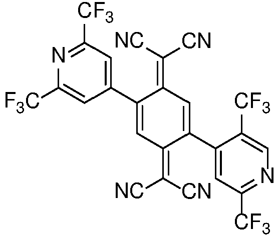 | -5.30 | -7.60 | 2.30 | 2.18 |
| I-23 | 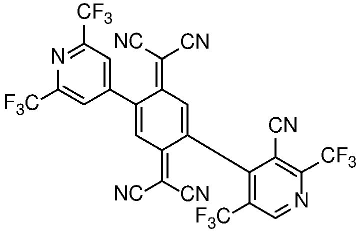 | -5.37 | -7.38 | 2.31 | 4.64 |
| I-24 | 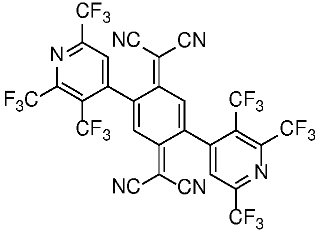 | -5.36 | -7.68 | 2.32 | 0.76 |
| I-25 | 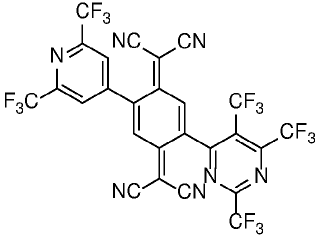 | -5.39 | -7.69 | 2.31 | 1.35 |
| I-26 | 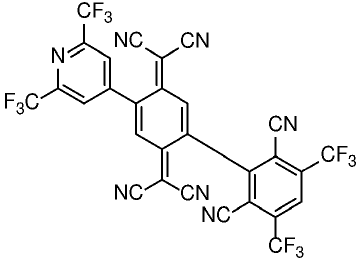 | -5.39 | -7.69 | 2.30 | 5.43 |
| I-27 | 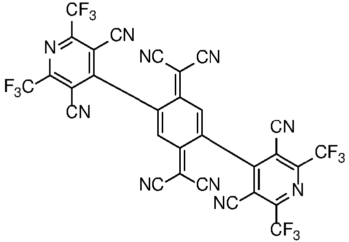 | -5.66 | -7.69 | 2.30 | 7.90 |
| I-28 | 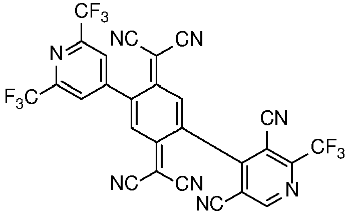 | -5.46 | -7.75 | 2.29 | 4.03 |
| I-29 | 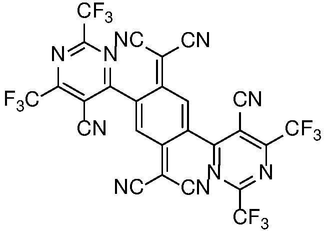 | -5.53 | -7.83 | 2.30 | 1.42 |
| I-30 | 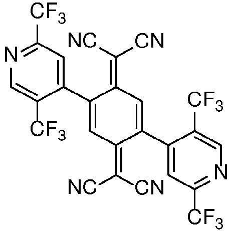 | -5.21 | -7.53 | 2.32 | 0.14 |
| I-31 | 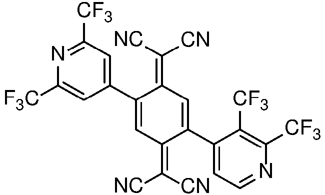 | -5.26 | -7.58 | 2.32 | 3.42 |
| I-32 | 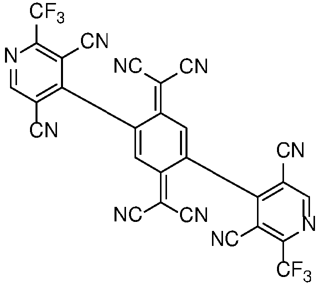 | -5.51 | -7.81 | 2.30 | 1.31 |
| I-33 | 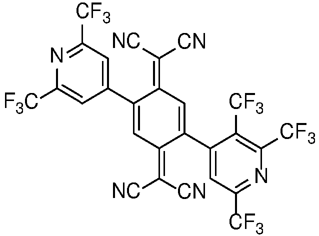 | -5.38 | -7.68 | 2.30 | 2.13 |
| I-34 | 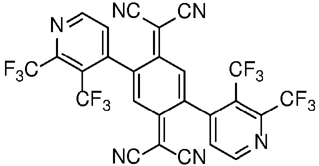 | -5.16 | -7.48 | 2.32 | 6.36 |
| I-35 | 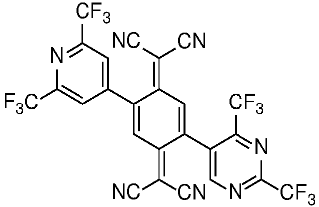 | -5.38 | -7.68 | 2.30 | 1.44 |
| I-36 | 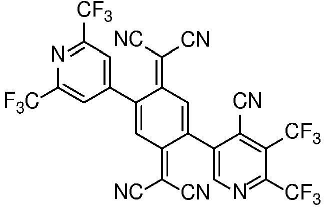 | -5.41 | -7.72 | 2.31 | 2.85 |
| I-37 | 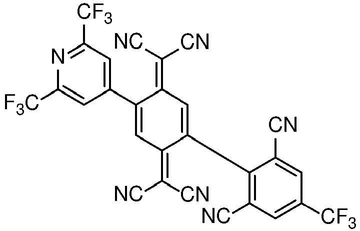 | -5.33 | -7.63 | 3.00 | 5.52 |
| I-38 | 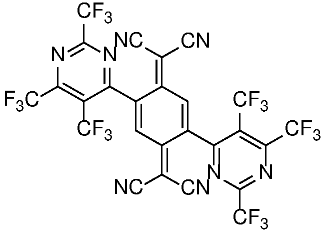 | -5.36 | -7.68 | 2.32 | 0.89 |
| I-39 | 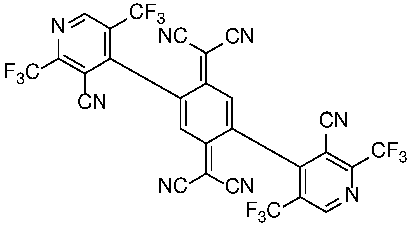 | -5.36 | -7.67 | 2.31 | 0.02 |
| I-40 | 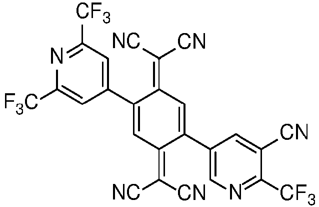 | -5.43 | -7.72 | 2.29 | 2.00 |

| | | | | | |
|---|---|---|---|---|---|
| *¹ = Calculated with Orca | | | | | |

### General procedure for fabrication of OLEDs

### Preparation of an OLED device containing a hole injection layer comprising a compound of the invention

For the examples according to the invention and comparative examples in Table IX, a glass substrate with an anode layer comprising a first anode sub-layer of 10 nm ITO, a second anode sub-layer of 120 nm Ag and a third anode sub-layer of 8 nm ITO was cut to a size of 100 mm x 100 mm x 0.7 mm, ultrasonically washed with water for 60 minutes and then with isopropanol for 20 minutes. The liquid film was removed in a nitrogen stream, followed by plasma treatment, see Table 2, to prepare the anode layer. The plasma treatment was performed in an atmosphere comprising 97.6 vol.-% nitrogen and 2.4 vol.-% oxygen.

Then N-(9,9-diphenyl-9H-fluoren-2-yl)-N,9-diphenyl-9H-carbazol-2-amine (N-5) as a hole transport matrix compound was vacuum deposited with 2 wt.% of an inventive compound of table 2 or a comparative compound as a dopant to form a hole injection layer (HIL) having a thickness 10 nm according to Table 4.

Then N-(9,9-diphenyl-9H-fluoren-2-yl)-N,9-diphenyl-9H-carbazol-2-amine (N-5) was vacuum deposited, to form a hole transport layer (HTL) having a thickness of 128 nm

Then N,N-di([1,1'-biphenyl]-4-yl)-3'-(9H-carbazol-9-yl)-[1,1'-biphenyl]-4-amine (N-3) was vacuum deposited on the HTL, to form an electron blocking layer (EBL) having a thickness of 5 nm.

Then, an emission layer (EML) having a thickness of 19 nm is formed on the EBL by co-depositing 99 vol.-% Dibenzofuran, 7-(phenyl-2 ,3 ,4 ,5 ,6 -d )-1-[10-(phenyl-2 ,3 ,4 ,5 ,6 -d )-9-anthracenyl] [2457172-82-4] as EML host and 1 vol.-% 5H ,9H -[1]Benzothieno-[2',3':5,6][1,4]azaborino[2,3,4-kl ]phenazaborine, 2,7,11-tris(1,1-dimethylethyl)-5,9-bis[4-(1,1-dimethylethyl)phenyl] [2482607-57-6] as blue dopant.

Then, a hole blocking layer having a thickness of 5 nm is formed on the EML by depositing compound 4-([1,1'-biphenyl]-4-yl)-6-(3'-(9,9-dimethyl-9H-fluoren-4-yl)-[1,1'-biphenyl]-4-yl)-2-phenylpyrimidine (N-14).

Then, an electron transport layer having a thickness of 31 nm is formed on the HBL by co-depositing compound 6,6'-(naphthalene-1,2-diylbis(4,1-phenylene))bis(2,4-diphenyl-1,3,5-triazine) (N-15) and LiQ in a ratio of 50:50 wt.-%.

Then Yb was evaporated at a rate of 0.01 to 1 Å/s at 10⁻⁷ mbar to form an electron injection layer (EIL) with a thickness of 1.3 nm on the electron transporting layer.

Ag/Mg (1.8 wt.%) is evaporated at a rate of 0.01 to 1 Å/s at 10⁻⁷ mbar to form a cathode with a thickness of 13 nm.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

**Table 3**

| Compounds used | | |
|---|---|---|
| N-1 | N-([1,1'-biphenyl]-4-yl)-9,9-dimethyl-N-(4-(9-phenyl-9H-carbazol-3-yl)phenyl)-9H-fluoren-2-amine | 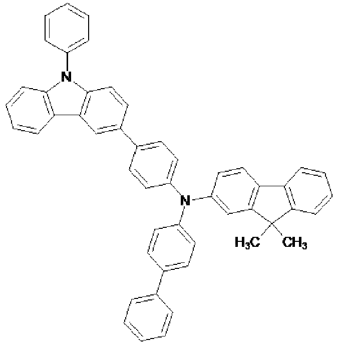 |
| N-2 | 4,4',4"-((1E,1'E,1"E)-cyclopropane-1,2,3-triylidenetris(cyanomethanylylidene))tris(2 ,3,5,6-tetrafluorobenzonitrile) | 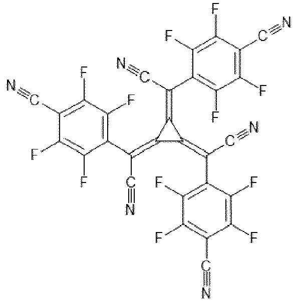 |
| N-3 | N,N-di([1,1'-biphenyl]-4-yl)-3'-(9H-carbazol-9-yl)-[1, 1'-biphenyl]-4-amine | 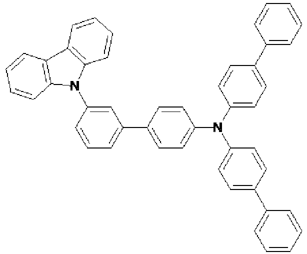 |
| N-4 | 2,2'-(1,3-Phenylene)bis[9-phenyl-1,10-phenanthroline | 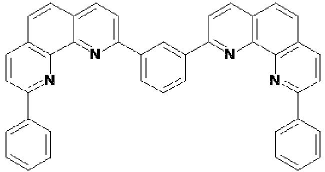 |
| N-5 | N-(9,9-diphenyl-9H-fluoren-2-yl)-N,9-diphenyl-9H-carbazol-2-amine | 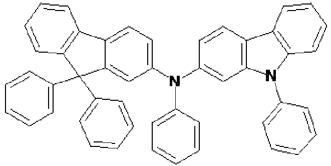 |
| N-14 | 4-([1,1'-biphenyl]-4-yl)-6-(3'-(9,9-dimethyl-9H-fluoren-4-yl)-[1,1'-biphenyl]-4-yl)-2-phenylpyrimidine | 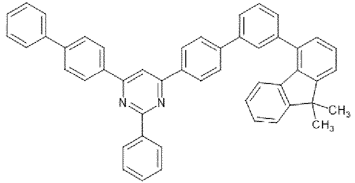 |
| N-15 | 6,6'-(naphthalene-1,2-diylbis(4,1-phenylene))bis(2,4-diphenyl-1,3,5-triazine) | 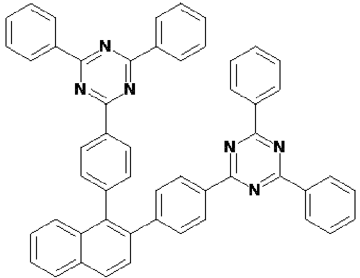 |

### Preparation of an OLED device containing p-type charge generation comprising a compound of the invention

For the examples according to the invention and comparative examples in Table IX, a glass substrate with an anode layer comprising a first anode sub-layer of 10 nm ITO, a second anode sub-layer of 120 nm Ag and a third anode sub-layer of 8 nm ITO was cut to a size of 100 mm x 100 mm x 0.7 mm, ultrasonically washed with water for 60 minutes and then with isopropanol for 20 minutes. The liquid film was removed in a nitrogen stream, followed by plasma treatment, see Table 4, to prepare the anode layer. The plasma treatment was performed in an atmosphere comprising 97.6 vol.-% nitrogen and 2.4 vol.-% oxygen.

Then N-([1,1'-biphenyl]-4-yl)-9,9-dimethyl-N-(4-(9-phenyl-9H-carbazol-3-yl)phenyl)-9H-fluoren-2-amine (N-1) as first hole transport matrix compound was vacuum deposited with 2 wt.% of 4,4',4"-((1E,1'E,1"E)-cyclopropane-1,2,3-triylidenetris(cyanomethanylylidene))tris-(2,3,5,6-tetrafluorobenzonitrile) (N-2) to form a hole injection layer (pHIL) having a thickness 10 nm.

Then N-([1,1'-biphenyl]-4-yl)-9,9-dimethyl-N-(4-(9-phenyl-9H-carbazol-3-yl)phenyl)-9H-fluoren-2-amine (N-1) was vacuum deposited, to form a first hole transport layer having a thickness of 29 nm

Then N,N-di([1,1'-biphenyl]-4-yl)-3'-(9H-carbazol-9-yl)-[1,1'-biphenyl]-4-amine (N-3) was vacuum deposited on the HTL, to form an electron blocking layer (EBL) having a thickness of 5 nm.

Then, a first emission layer (EML1) having a thickness of 19 nm is formed on the EBL1 by co-depositing 99 vol.-% Dibenzofuran, 7-(phenyl-2 ,3 ,4 ,5 ,6 -d )-1-[10-(phenyl-2 ,3 ,4 ,5 ,6 -d )-9-anthracenyl] [2457172-82-4]as EML host and 1 vol.-% 5H ,9H - [1]Benzothieno[2',3':5,6][1,4]azaborino[2,3,4-kl ]phenazaborine, 2,7,11-tris(1,1-dimethylethyl)-5,9-bis[4-(1,1-dimethylethyl)phenyl] [2482607-57-6] as blue dopant.

Then, the first electron transporting layer (ETL1) having a thickness of 7.5 nm is formed on first emission layer by depositing 2,2'-(1,3-Phenylene)bis[9-phenyl-1,10-phenanthroline] (N-4) according to Table 4.

Then a first n-type charge generation layer (n-CGL1) having a thickness of 10 nm is formed on the first electron transport layer (ETL1) by co-depositing 98 vol% of 2,2'-(1,3-Phenylene)bis[9-phenyl-1,10-phenanthroline (N-4) and 2 vol% Yb.

Then a first p-type charge generation layer (p-CGL) having a thickness of 10 nm is formed on the first n-type CGL by co-depositing 90 vol.-% of N-(9,9-diphenyl-9H-fluoren-2-yl)-N,9-diphenyl-9H-carbazol-2-amine (N-5) with 10 vol.-% of an inventive compound or comparative according to Table 2 as a dopant.

Then a second hole transport layer having a thickness of 43 nm is formed on the first p-type CGL by depositing N-(9,9-diphenyl-9H-fluoren-2-yl)-N,9-diphenyl-9H-carbazol-2-amine (N-5).

Then a second electron blocking layer having a thickness of 5 nm is formed on the second hole transport layer by depositing N,N-di([1,1'-biphenyl]-4-yl)-3'-(9H-carbazol-9-yl)-[1,1'-biphenyl]-4-amine (N-3).

Then, a second emission layer (EML2) having a thickness of 19 nm is formed on the EBL1 by co-depositing 99 vol.-% Dibenzofuran, 7-(phenyl-2 ,3 ,4 ,5 ,6 -d )-1-[10-(phenyl-2 ,3 ,4 ,5 ,6 -d )-9-anthracenyl] [2457172-82-4]as EML host and 1 vol.-% 5H ,9H - [1]Benzothieno[2',3':5,6][1,4]azaborino[2,3,4-kl ]phenazaborine, 2,7,11-tris(1,1-dimethylethyl)-5,9-bis[4-(1,1-dimethylethyl)phenyl] [2482607-57-6] as blue dopant.

Then, a first hole blocking layer (HBL1) having a thickness of 5 nm is formed on the EML2 by depositing compound 4-([1,1'-biphenyl]-4-yl)-6-(3'-(9,9-dimethyl-9H-fluoren-4-yl)-[1,1'-biphenyl]-4-yl)-2-phenylpyrimidine (N-14).

Then, a second electron transport layer (ETL2) having a thickness of 31 nm is formed on the HBL by co-depositing compound 6,6'-(naphthalene-1,2-diylbis(4,1-phenylene))bis(2,4-diphenyl-1,3,5-triazine) (N-15) and LiQ in a ratio of 50:50 wt.-%.

Then Yb was evaporated at a rate of 0.01 to 1 Å/s at 10⁻⁷ mbar to form an electron injection layer (EIL) with a thickness of 2 nm on the electron transporting layer.

Ag/Mg (1.8 wt.%) is evaporated at a rate of 0.01 to 1 Å/s at 10⁻⁷ mbar to form a cathode with a thickness of 13 nm.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 15 mA/cm² is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 15 mA/cm².

In top emission devices, the emission is forward directed, non-Lambertian and also highly dependent on the micro-cavity. Therefore, the efficiency EQE will be higher compared to bottom emission devices. To determine the efficiency EQE in % the light output of the device is measured using a calibrated photodiode at 15 mA/cm².

Lifetime LT of the device is measured at ambient conditions (20°C) and 10 mA/cm² or 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

The increase in operating voltage ΔU is used as a measure of the operational voltage stability of the device. This increase is determined during the LT measurement and by subtracting the operating voltage after 1 hour after the start of operation of the device from the operating voltage after 100 hours. $ΔU = \left[U\left(100 h\right)-U\left(1h\right)\right] .$

The smaller the value of ΔU the better is the operating voltage stability.

### Technical Effect of the invention

**Table 4**

| Example | Dopant in HIL | LUMO B3LYP/6-31G* | CIEY | Voltage at 10mA/cm² [V] | Ceff at 10mA/cm² [cd/A] | Ceff/CIEy at 10mA/cm² [cd/A] | EQE at 10mA/cm² [%] | LT97 at 20mA/cm² [h] | ΔU at 20mA/cm² (1-100h) [V] |
|---|---|---|---|---|---|---|---|---|---|
| Comparative Example Compound C1 | 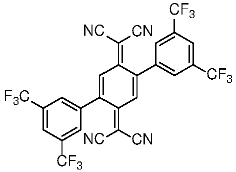 | -4.95 eV | 0.044 | 3.59 | 10.0 | 227 | 21.4 | 48 | 0.335 |
| Inv. Example I-1 | 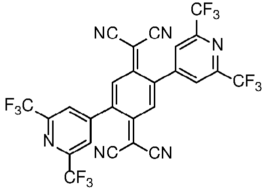 | -5.28 eV | 0.043 | 3.32 | 10.3 | 239 | 22.4 | 125 | 0.064 |

The OLED according to the inventive example (inv.Ex.1) comprising a compound of the invention exhibits a lower operational voltage than the comparative OLED (comp. ex. 1).

A lower operating voltage may be important for the battery life of organic electronic devices, in particular mobile devices.

The OLED according to the inventive example (inv.Ex.1) comprising a compound of the invention exhibits a higher current efficiency as well as external quantum efficiency than the comparative OLED (comp. ex. 1).

A high efficiency such as external quantum efficiency as well as current efficiency may be beneficial for reduced power consumption and improved battery life, in particular in mobile devices.

The OLED according to the inventive example (inv.Ex.1) comprising a compound of the invention exhibits a lower rise of the operational voltage over time than the comparative OLED (comp. ex. 1).

A low voltage rise over time may result in improved long-term stability of electronic devices.

The OLED according to the inventive example (inv.Ex.1) comprising a compound of the invention exhibits a higher lifetime than the comparative OLED (comp. ex. 1).

A long lifetime may result in improved long-term stability of electronic devices.

**Table 5**

| Exempels | Dopant in p-CGL | LUMO B3LYP/6-31G* | conc [vol%] | CIEY | Voltage at 10mA/cm² [V] | Ceff at 10mA/cm² [cd/A] | Ceff/CIEy at 10mA/cm² [cd/A] | EQE at 10mA/cm² [%] | LT97 at 30mA/cm² [h] | ΔU at 30mA/cm² (1-100h) [V] |
|---|---|---|---|---|---|---|---|---|---|---|
| Comp. Ex. 2 | compound C1 | -4.95 eV | 7 | 0.049 | 8.56 | 12.2 | 249 | 24.1 | 89 | 0.342 |
| Inv. ex | I-1 | -5.28 eV | 8 | 0.049 | 6.70 | 16.3 | 334 | 32.2 | 98 | 0.277 |

The OLED according to the inventive example (inv.Ex.2) comprising a compound of the invention exhibits a lower operational voltage than the comparative OLED (comp. ex. 2).

A lower operating voltage may be important for the battery life of organic electronic devices, in particular mobile devices.

The OLED according to the inventive example (inv.Ex.2) comprising a compound of the invention exhibits a higher current efficiency as well as external quantum efficiency than the comparative OLED (comp. ex. 2).

A high efficiency such as external quantum efficiency as well as current efficiency may be beneficial for reduced power consumption and improved battery life, in particular in mobile devices.

The OLED according to the inventive example (inv.Ex.2) comprising a compound of the invention exhibits a lower rise of the operational voltage over time than the comparative OLED (comp. ex. 2).

A low voltage rise over time may result in improved long-term stability of electronic devices.

The OLED according to the inventive example (inv.Ex.2) comprising a compound of the invention exhibits a higher lifetime than the comparative OLED (comp. ex. 2).

A long lifetime may result in improved long-term stability of electronic devices.

The invention's scope is defined in the following claims.

Furthermore, reference signs used in the description and claims do not limit the scope of the invention as claimed.

## Claims

1. A compound of formula (I): 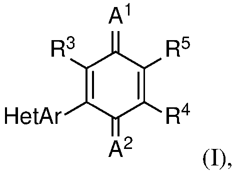 wherein
HetAr is selected from formula (II): 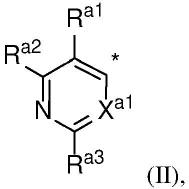
A¹ is selected from formula (III): 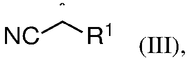
A² is selected from formula (IV) 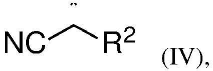
wherein the asterisk "*" denotes the binding position,
R¹ and R² are independently selected from CN, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, SF₅,
wherein the one or more substituents on R¹ and R² are independently selected from D, partially fluorinated C₁ to C₈ alkyl, perfluorinated C₁ to C₈ alkyl, CF₃, halogen, F, CN or SF₅;
R³ and R⁴ are independently selected from H, D, halogen, F, CN, partially fluorinated C₁ to C₄ alkyl, perfluorinated C₁ to C₄ alkyl, SF₅ or CF₃;
R⁵ is independently selected from H, D, halogen, F, CN, partially fluorinated C₁ to C₈ alkyl, perfluorinated C₁ to C₈ alkyl, CF₃, substituted or unsubstituted C₆ to C₃₀ aryl or substituted or unsubstituted C₂ to C₃₀ heteroaryl, SF₅,
wherein the one or more substituents on R⁵ are independently selected from D, partially fluorinated C₁ to C₈ alkyl or perfluorinated C₁ to C₈ alkyl, CF₃, halogen, F, CN, SF₅;
X^{a1} is selected from N or CR^{a4};
R^{a1}, R^{a2}, R^{a3} and R^{a4} are independently selected from H, D, substituted or unsubstituted C₁ to C₈ alkyl; partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN, SF₅,
wherein the one or more substituents on R^{a1}, R^{a2}, R^{a3} and R^{a4} are independently selected from D, partially fluorinated C₁ to C₈ alkyl or perfluorinated C₁ to C₈ alkyl, CF₃, halogen, F, CN, SF₅;
wherein at least one of R^{a1}, R^{a2}, R^{a3} and R^{a4} is selected from partially fluorinated C₁ to C₈ alkyl, perfluorinated C₁ to C₈ alkyl, CF₃, halogen, F, CN, or SF₅.

2. The compound of formula (I) according of claim 1, wherein R¹ and R² are independently selected from CN, partially fluorinated C₁ to C₄ alkyl, perfluorinated C₁ to C₄ alkyl, CF₃, substituted or unsubstituted C₆ to C₁₀ aryl, or substituted or unsubstituted C₃ to C₉ heteroaryl; preferably R¹ and R² are independently selected from CN, partially fluorinated C₁ to C₄ alkyl, perfluorinated C₁ to C₄ alkyl, CF₃, substituted or unsubstituted C₆ aryl, substituted or unsubstituted C₃ to C₅ heteroaryl; and further preferred R¹ and R² are independently selected from CN, partially fluorinated C₁ to C₄ alkyl, perfluorinated C₁ to C₄ alkyl, CF₃, substituted or unsubstituted C₆ aryl, substituted or unsubstituted C₄ to C₅ heteroaryl.

3. The compound of formula (I) according of claim 1 or 2, wherein the one or more substituents on R¹ and R² are independently selected from D, partially fluorinated C₁ to C₈ alkyl, perfluorinated C₁ to C₈ alkyl, CF₃, or CN; preferably wherein the one or more substituents on R¹ and R² are independently selected from D, partially fluorinated C₁ to C₄ alkyl, perfluorinated C₁ to C₄ alkyl, CF₃, or CN; further preferred the one or more substituents on R¹ and R² are independently selected from D, CF₃, CN.

4. The compound of formula (I) according to any of the preceding claims 1 to 3, wherein A¹ and A² are selected from formula (V): 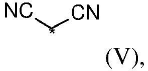 wherein the asterisk "*" denotes the binding position.

5. The compound of formula (I) according to any of the preceding claims 1 to 4, wherein R³ and R⁴ are independently selected from H, D, CN, partially fluorinated C₁ to C₄ alkyl, perfluorinated C₁ to C₄ alkyl, or CF₃, preferably R³ and R⁴ are independently selected from H, D, CN, CF₃, and further preferred R³ and R⁴ are independently selected from H, D.

6. The compound of formula (I) according to any of the preceding claims 1 to 5, wherein R⁵ is selected from H, D, CN, CF₃, substituted or unsubstituted C₆ aryl, substituted or unsubstituted C₄ to C₅ heteroaryl; preferably R⁵ is selected from H, D, CN, substituted or unsubstituted C₆ aryl or substituted or unsubstituted C₄ to C₅ heteroaryl; or further preferred R⁵ is selected from substituted or unsubstituted C₆ aryl or substituted or unsubstituted C₄ to C₅ heteroaryl.

7. The compound of formula (I) according to any of the preceding claims 1 to 6, wherein the one or more substituents on R⁵ are independently selected from D, partially fluorinated C₁ to C₄ alkyl, perfluorinated C₁ to C₄ alkyl, CF₃, halogen, F, or CN; preferably the one or more substituents on R⁵ are independently selected from D, partially fluorinated C₁ to C₄ alkyl, perfluorinated C₁ to C₄ alkyl, CF₃, or CN; and further preferred the one or more substituents on R⁵ are independently selected from D, CF₃, or CN.

8. The compound of formula (I) according to any of the preceding claims 1 to 7, wherein a CN group is not bound to an aromatic carbon atom when said aromatic carbon is directly bound to a sp2-hybridized ring nitrogen atom.

9. The compound of formula (I) according to any of the preceding claims 1 to 8, wherein R⁵ is selected from formula (VIa): 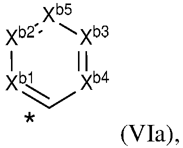 wherein the "*" denotes the binding position, and
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, or CN,
wherein the one or more substituents on R^{b1} to R^{b5} are independently selected from D, partially fluorinated C₁ to C₈ alkyl, perfluorinated C₁ to C₈ alkyl, CF₃, halogen, F, or CN;
preferably at least one of R^{b1} to R^{b5} is selected from partially fluorinated or perfluorinated C₁ to C₈ alkyl, CF₃, halogen, F, or CN; and more preferred at least one of R¹¹ to R^{b5} is selected from partially fluorinated or perfluorinated C₁ to C₈ alkyl, CF₃, or CN.

10. The compound of formula (I) according to any of the preceding claims 1 to 9, wherein when R^{bn} is N with n=1 to 5 then the neighbored R^{bm} with m=1 to 5 is not a CN group.

11. The compound of formula (I) according to any of the preceding claims 1 to 10, wherein R⁵ is selected from formula (VIb): 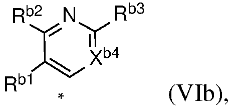 wherein the "*" denotes the binding position, and
wherein
X^{b4} is selected from N or CR^{b4},
R^{b1} to R^{b3} are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} to R^{b3} are independently selected from D, partially fluorinated C₁ to C₈ alkyl, perfluorinated C₁ to C₈ alkyl, CF₃, halogen, F, CN,
preferably at least one of R^{b1} to R^{b3} is selected from partially fluorinated or perfluorinated C₁ to C₈ alkyl, CF₃, halogen, F, or CN; and more preferably at least one of R^{b1} to R^{b3} is selected from partially fluorinated or perfluorinated C₁ to C₈ alkyl, CF₃, or CN.

12. The compound of formula (I) according to any of the preceding claims 1 to 11, wherein R⁵ is selected from formula (VIc): 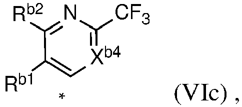 wherein the "*" denotes the binding position, and
wherein
X^{b4} is selected from N or CR^{b4},
R^{b1} and R^{b2} are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} and R^{b2} are independently selected from D, partially fluorinated C₁ to C₈ alkyl, perfluorinated C₁ to C₈ alkyl, CF₃, halogen, F, CN,
preferably at least one of R^{b1} and R^{b2} is selected from partially fluorinated or perfluorinated C₁ to C₈ alkyl, CF₃, halogen, F, or CN; and more preferably at least one of R^{b1} and R^{b2} is selected from partially fluorinated or perfluorinated C₁ to C₈ alkyl, CF₃, or CN.

13. The compound of formula (I) according to any of the preceding claims 1 to 12, wherein R⁵ is selected from formula (VId): 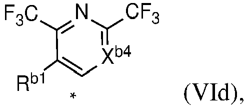 wherein the "*" denotes the binding position, and
wherein
X^{b4} is selected from N or CR^{b4},
R^{b1} is independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} is independently selected from D, partially fluorinated C₁ to C₈ alkyl, perfluorinated C₁ to C₈ alkyl, CF₃, halogen, F, CN,
preferably R^{b1} is selected from partially fluorinated or perfluorinated C₁ to C₈ alkyl, CF₃, halogen, F, or CN; and more preferably R^{b1} is selected from partially fluorinated or perfluorinated C₁ to C₈ alkyl, CF₃, or CN.

14. The compound of formula (I) according to any of the preceding claims 1 to 13, wherein R⁵ is selected from formula (VIe): 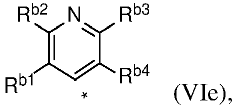 wherein the "*" denotes the binding position, and
wherein
R^{b1} to R^{b4} are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} to R^{b4} are independently selected from D, partially fluorinated C₁ to C₈ alkyl, perfluorinated C₁ to C₈ alkyl, CF₃, halogen, F, CN,
preferably at least one of R^{b1} to R^{b4} are selected from partially fluorinated or perfluorinated C₁ to C₈ alkyl, CF₃, halogen, F, or CN; and more preferably at least one of R^{b1} to R^{b4} are selected from partially fluorinated or perfluorinated C₁ to C₈ alkyl, CF₃, or CN.

15. The compound of formula (I) according to any of the preceding claims 1 to 14, wherein R⁵ is selected from formula (VIf): 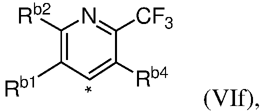 wherein the "*" denotes the binding position, and
wherein
R^{b1}, R^{b2} and R^{b4} are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1}, R^{b2} and R^{b4} are independently selected from D, partially fluorinated C₁ to C₈ alkyl, perfluorinated C₁ to C₈ alkyl, CF₃, halogen, F, CN,
preferably at least one of R^{b1}, R^{b2} and R^{b4} are selected from partially fluorinated or perfluorinated C₁ to C₈ alkyl, CF₃, halogen, F, or CN; and more preferably at least one of R^{b1} to R^{b4} are selected from partially fluorinated or perfluorinated C₁ to C₈ alkyl, CF₃, or CN.

16. The compound of formula (I) according to any of the preceding claims 1 to 15, wherein R⁵ is selected from formula (VIg): 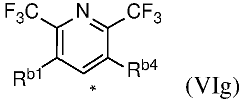 wherein the "*" denotes the binding position, and
wherein
R^{b1} and R^{b4} are independently selected from H, D, substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, halogen, F, CN,
wherein the one or more substituents on R^{b1} and R^{b4} are independently selected from D, partially fluorinated C₁ to C₈ alkyl, perfluorinated C₁ to C₈ alkyl, CF₃, halogen, F, CN,
preferably at least one of R^{b1} and R^{b4} are selected from partially fluorinated or perfluorinated C₁ to C₈ alkyl, CF₃, halogen, F, or CN; and more preferably at least one of R^{b1} and R^{b4} are selected from partially fluorinated or perfluorinated C₁ to C₈ alkyl, CF₃, or CN.

17. The compound of formula (I) according to any of the preceding claims 1 to 16, wherein R^{a1} to R^{a4} in formula I, R^{b1} to R^{b5} in formula (VIa), R^{b1} to R^{b3} in formula (VIb), R^{b1} to R^{b2} in formula (VIc), R^{b1} in formula (VId), R^{b1} to R^{b4} in formula (Vie), R^{b1}, R^{b2} and R^{b4} in formula (VIf), R^{b1} and R^{b4} in formula (VIg) are not selected halogen or F; and preferably R^{a1} to R^{a4} in formula I, R^{b1} to R^{b5} in formula (VIa), R^{b1} to R^{b3} in formula (VIb), R^{b1} to R^{b2} in formula (VIc), R^{b1} in formula (VId), R^{b1} to R^{b4} in formula (Vie). R^{b1}, R^{b2} and R^{b4} in formula (VIf), R^{b1} and R^{b4} in formula (VIg) are not selected halogen or F, and the one or more substituents on R^{a1} to R^{a4}, R^{b1} to R^{b5} are not selected from halogen or F.

18. The compound of formula (I) according to any of the preceding claims 1 to 17, wherein:
R^{a2} to R^{a3} in formula (II), R^{b2} to R^{b3} in formula (VIa), (VIb), (VIe), and R^{b2} in formula (VIc) and (VIf) are not selected CN.

19. The compound of formula (I) according to any of the preceding claims 1 to 18, wherein R⁵ is selected from a group represented by B1 to B160:
| | | | |
|---|---|---|---|
| 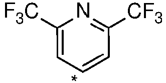 | 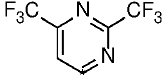 | 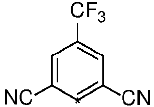 | 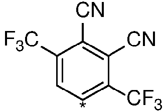 |
| B1 | B2 | B3 | B4 |
| 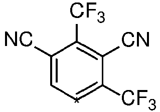 | 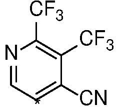 | 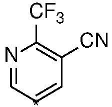 | 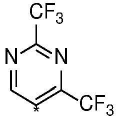 |
| B5 | B6 | B7 | B8 |
| 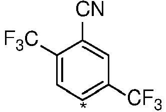 | 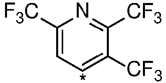 | 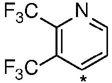 | 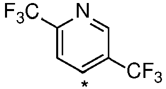 |
| B9 | B10 | B11 | B12 |
| 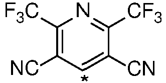 | 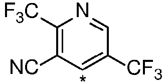 | 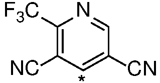 | 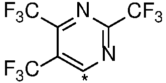 |
| B13 | B14 | B15 | B16 |
| 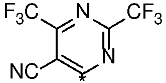 | 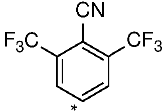 | 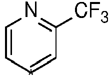 | 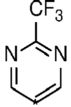 |
| B17 | B18 | B19 | B20 |
| 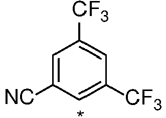 | 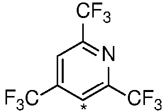 | 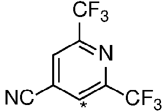 | 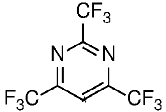 |
| B21 | B22 | B23 | B24 |
| 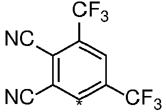 | 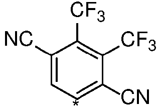 | 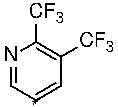 | 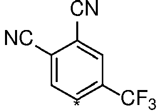 |
| B25 | B26 | B27 | B28 |
| 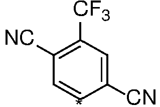 | 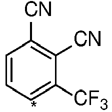 | 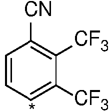 | 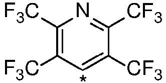 |
| B29 | B30 | B31 | B32 |
| 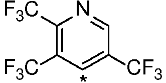 | 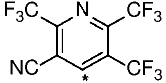 | 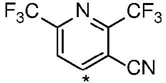 | 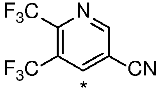 |
| B33 | B34 | B35 | 36B |
| 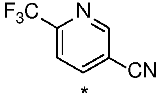 | 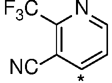 | 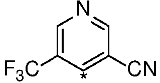 | 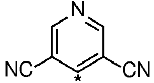 |
| B37 | B38 | B39 | B40 |
| 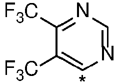 | 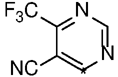 | 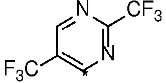 | 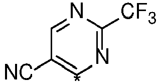 |
| B41 | B42 | B43 | B44 |
| 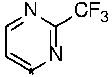 | 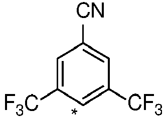 | 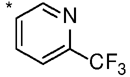 | 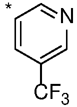 |
| B45 | B46 | B47 | B48 |
| 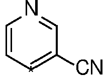 | 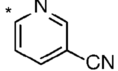 | 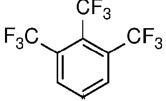 | 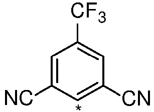 |
| B49 | B50 | B51 | B52 |
| 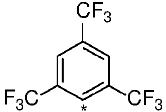 | 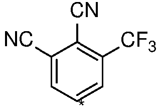 | 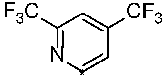 | 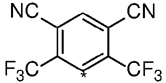 |
| B53 | B54 | B55 | B56 |
| 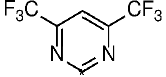 | 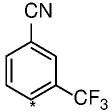 | 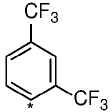 | 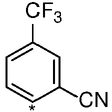 |
| B57 | B58 | B59 | B60 |
| 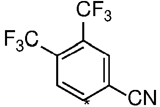 | 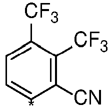 | 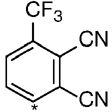 | 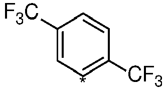 |
| B61 | B62 | B63 | B64 |
| 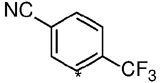 | 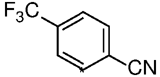 | 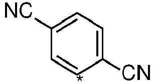 | 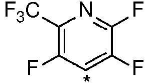 |
| B65 | B66 | B67 | B68 |
| 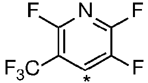 | 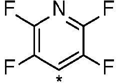 | 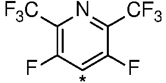 | 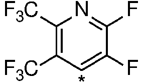 |
| B69 | B70 | B71 | B72 |
| 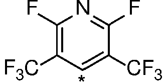 | 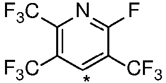 | 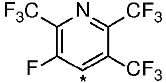 | 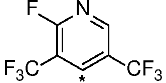 |
| B73 | B74 | B75 | B76 |
| 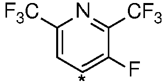 | 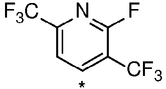 | 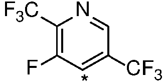 | 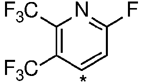 |
| B77 | B78 | B79 | B80 |
| 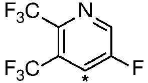 | 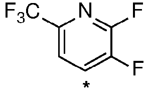 | 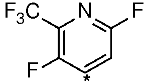 | 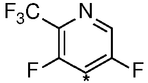 |
| B81 | B82 | B83 | B84 |
| 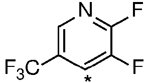 | 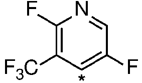 | 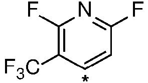 | 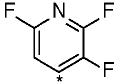 |
| B85 | B86 | B87 | B88 |
| 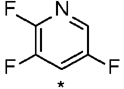 | 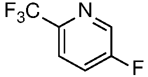 | 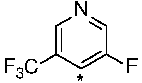 | 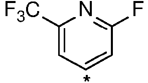 |
| B89 | B90 | B91 | B92 |
| 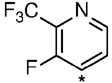 | 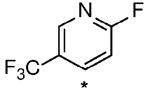 | 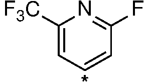 | 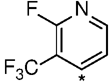 |
| B93 | B94 | B95 | B96 |
| 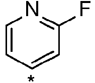 | 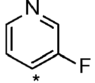 | 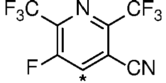 | 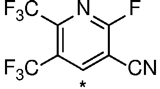 |
| B97 | B98 | B99 | B100 |
| 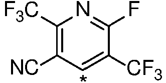 | 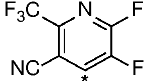 | 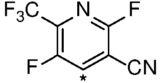 | 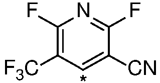 |
| B101 | B102 | B103 | B104 |
| 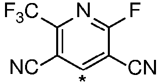 | 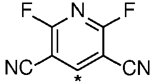 | 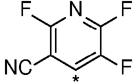 | 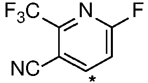 |
| B105 | B106 | B107 | B108 |
| 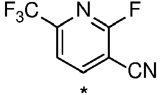 | 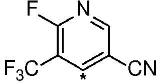 | 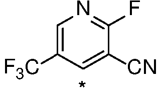 | 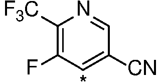 |
| B109 | B110 | B111 | B112 |
| 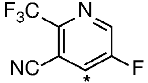 | 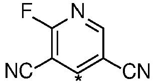 | 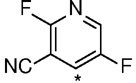 | 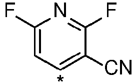 |
| B113 | B114 | B115 | B116 |
| 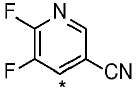 | 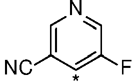 | 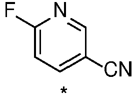 | 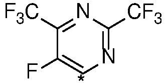 |
| B117 | B118 | B119 | B120 |
| 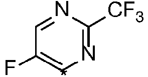 | 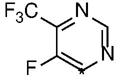 | 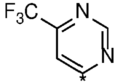 | 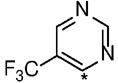 |
| B121 | B122 | B123 | B124 |
| 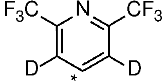 | 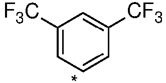 | 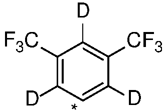 | 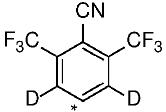 |
| B125 | B126 | B127 | B128 |
| 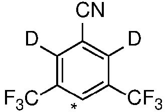 | 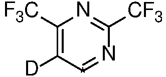 | 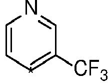 | 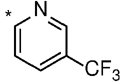 |
| B129 | B130 | B131 | B132 |
| 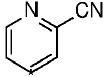 | 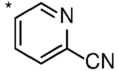 | 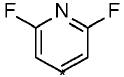 | 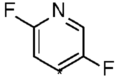 |
| B133 | B134 | B135 | B136 |
| 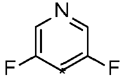 | 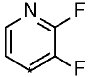 | 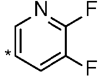 | 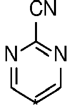 |
| B137 | B138 | B139 | B140 |
| 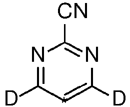 | 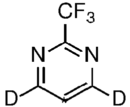 | 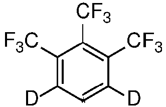 | 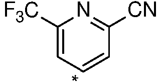 |
| B141 | B142 | B143 | B144 |
| 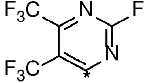 | 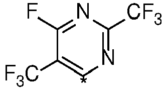 | 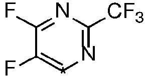 | 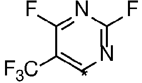 |
| B145 | B146 | B147 | B148 |
| 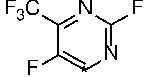 | 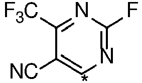 | 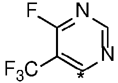 | 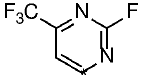 |
| B149 | B150 | B151 | B152 |
| 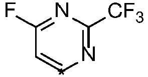 | 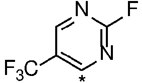 | 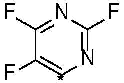 | 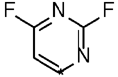 |
| B153 | B154 | B155 | B156 |
| 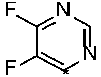 | 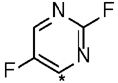 | 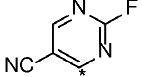 | 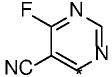 |
| B157 | B158 | B159 | B160 |
, wherein the "*" denotes the binding position.

20. The compound of formula (I) according to any of the preceding claims 1 to 19, wherein formula (II) is selected from formulas (IIa), (IIb), (IIc), (IId), (IIe), and (IIf): 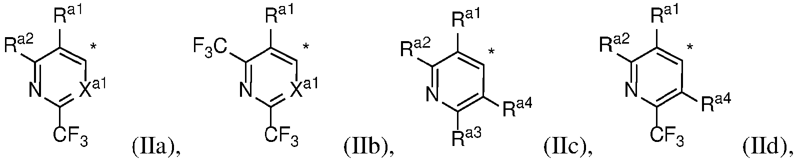 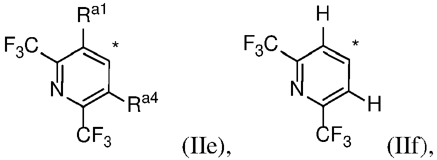 wherein the "*" denotes the binding position, and preferably wherein formulas (IIa), (IIb), (IIc), (IId), (IIe), and (IIf) are partially deuterated or fully deuterated, and further preferred formula (IIf) is partially deuterated or fully deuterated.

21. The compound of formula (I) according to any of the preceding claims 1 to 20, wherein HetAr is selected from a group represented by C1 to C109:
| | | | |
|---|---|---|---|
| 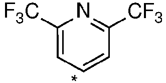 | 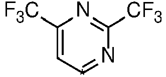 | 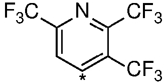 | 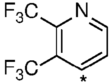 |
| C1 | C2 | C3 | C4 |
| 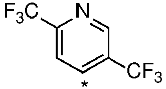 | 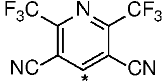 | 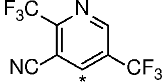 | 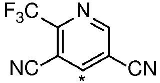 |
| C5 | C6 | C7 | C8 |
| 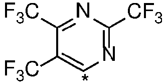 | 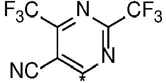 | 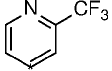 | 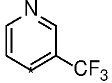 |
| C9 | C10 | C11 | C12 |
| 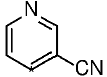 | 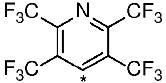 | 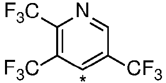 | 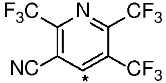 |
| C13 | C14 | C15 | C16 |
| 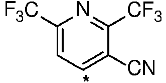 | 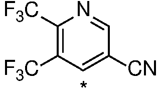 | 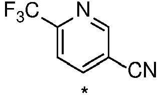 | 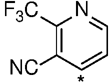 |
| C17 | C18 | C19 | C20 |
| 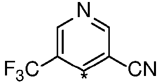 | 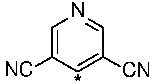 | 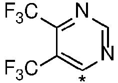 | 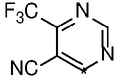 |
| C21 | C22 | C23 | C24 |
| 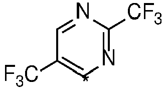 | 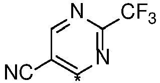 | 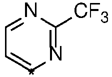 | 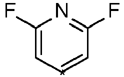 |
| C25 | C26 | C27 | C28 |
| 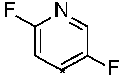 | 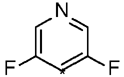 | 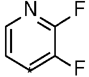 | 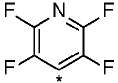 |
| C29 | C30 | C31 | C32 |
| 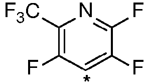 | 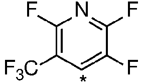 | 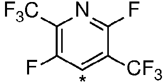 | 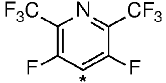 |
| C33 | C34 | C35 | C36 |
| 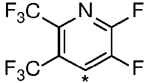 | 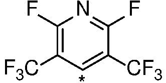 | 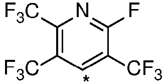 | 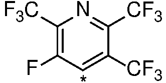 |
| C37 | C38 | C39 | C40 |
| 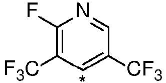 | 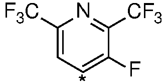 | 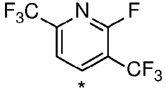 | 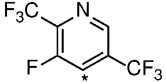 |
| C41 | C42 | C43 | C44 |
| 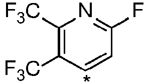 | 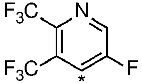 | 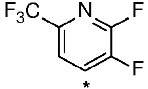 | 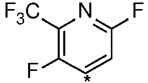 |
| C45 | C46 | C47 | C48 |
| 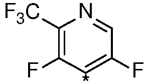 | 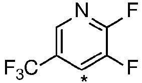 | 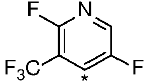 | 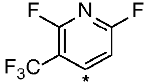 |
| C49 | C50 | C51 | C52 |
| 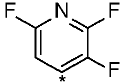 | 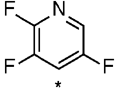 | 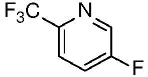 | 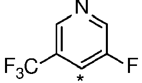 |
| C53 | C54 | C55 | C56 |
| 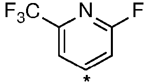 | 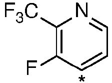 | 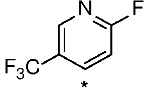 | 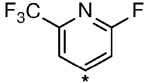 |
| C57 | C58 | C59 | C60 |
| 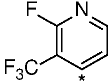 | 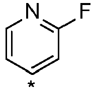 | 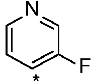 | 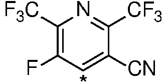 |
| C61 | C62 | C63 | C64 |
| 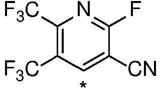 | 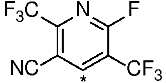 | 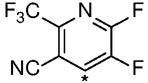 | 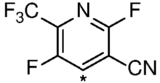 |
| C65 | C66 | C67 | C68 |
| 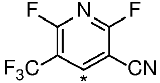 | 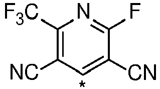 | 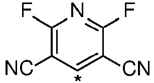 | 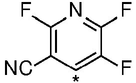 |
| C69 | C70 | C71 | C72 |
| 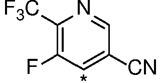 | 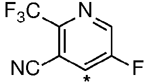 | 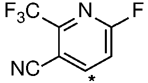 | 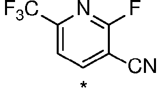 |
| C73 | C74 | C75 | C76 |
| 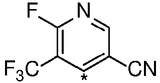 | 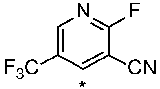 | 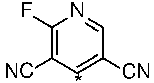 | 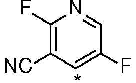 |
| *C77* | C78 | C79 | C80 |
| 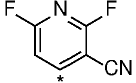 | 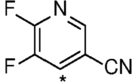 | 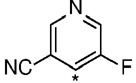 | 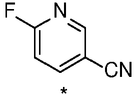 |
| C81 | C82 | C83 | C84 |
| 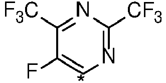 | 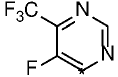 | 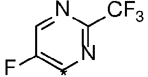 | 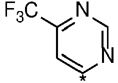 |
| C85 | C86 | C87 | C88 |
| 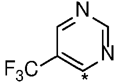 | 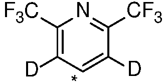 | 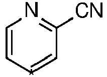 | 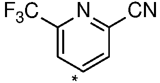 |
| C89 | C90 | C91 | C92 |
| 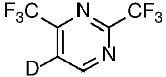 | 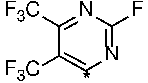 | 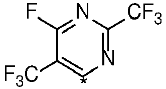 | 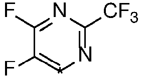 |
| C93 | C94 | C95 | C96 |
| 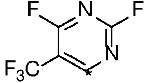 | 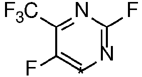 | 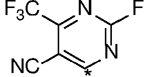 | 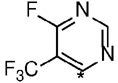 |
| C97 | C98 | C99 | C100 |
| 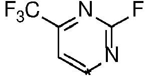 | 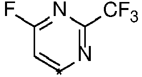 | 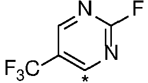 | 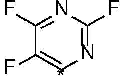 |
| C101 | C102 | C103 | C104 |
| 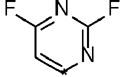 | 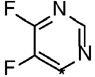 | 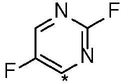 | 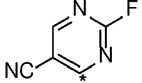 |
| C105 | C106 | C107 | C108 |
| 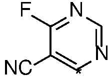 | | | |
| C109 | | | |
, wherein the "*" denotes the binding position.

22. The compound of formula (I) according to any of the preceding claims 1 to 21, wherein the compound of formula (I) is selected from a compound of formula (VII): 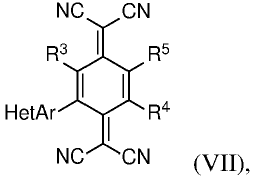 wherein
HetAr is selected from formulas (II), (IIa), (IIb), (IIc), (IId), (IIe), and (IIf): 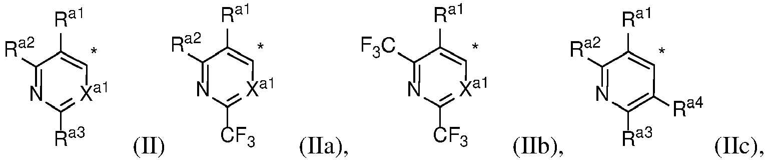 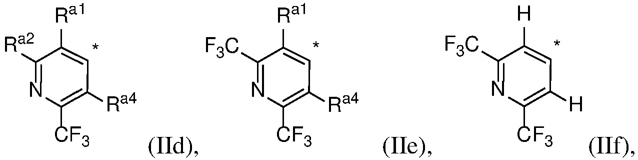 wherein the asterisk "*" denotes the binding position, and
wherein
R³ and R⁴ are selected from H, D;
X^{a1} is selected from N or CR^{a4};
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, or CN,
wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, or CN;
R⁵ is selected from H, D, CN, partially fluorinated C₁ to C₈ alkyl or perfluorinated C₁ to C₈ alkyl, CF₃, substituted or unsubstituted C₆ to C₃₀ aryl or substituted or unsubstituted C₂ to C₃₀ heteroaryl,
wherein the one or more substituents on R⁵ are independently selected from D, partially fluorinated C₁ to C₈ alkyl or perfluorinated C₁ to C₈ alkyl, CF₃, or CN.

23. The compound of formula (I) according to any of the preceding claims 1 to 22, wherein the compound of formula (I) is selected from a compound of formula (VII): 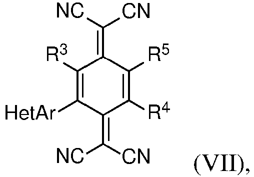 wherein
HetAr is selected from formulas (II), (IIa), (IIb), (IIc), (IId), (IIe), and (IIf): 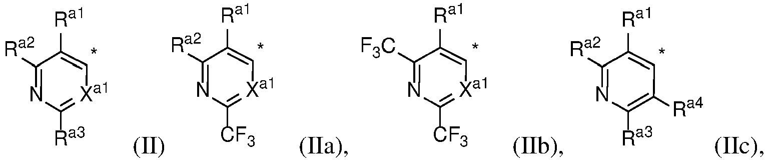 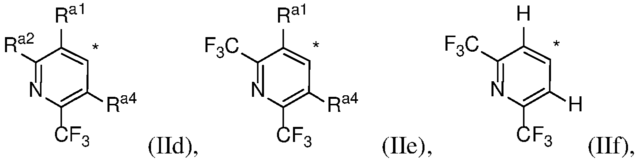
wherein the asterisk "*" denotes the binding position, and
wherein
R³ and R⁴ are selected from H, D;
X^{a1} is selected from N or CR^{a4};
R^{a1} to R^{a4} are independently selected from H, D, substituted or unsubstituted C₁ to C₈ alkyl; partially fluorinated C₁ to C₈ alkyl or perfluorinated C₁ to C₈ alkyl, CF₃, substituted or unsubstituted C₆ to C₃₀ aryl, substituted or unsubstituted C₂ to C₃₀ heteroaryl, halogen, F, or CN,
wherein the one or more substituents on R^{a1} to R^{a4} are independently selected from D, partially fluorinated C₁ to C₈ alkyl or perfluorinated C₁ to C₈ alkyl, CF₃, CN;
at least one of R^{a1} to R^{a4} is selected from partially fluorinated C₁ to C₈ alkyl or perfluorinated C₁ to C₈ alkyl, CF₃, or CN;
R⁵ is selected from formula (VIa) 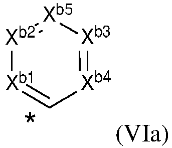
wherein
X^{b1} is selected from N or CR^{b1},
X^{b2} is selected from N or CR^{b2},
X^{b3} is selected from N or CR^{b3},
X^{b4} is selected from N or CR^{b4},
X^{b5} is selected from N or CR^{b5},
R^{b1} to R^{b5} are independently selected from H, D substituted or unsubstituted C₁ to C₈ alkyl, partially fluorinated C₁ to C₈ alkyl, perfluorinated C₁ to C₈ alkyl, CF₃, substituted or unsubstituted C₆ to C₃₀ aryl, substituted or unsubstituted C₂ to C₃₀ heteroaryl, or CN,
wherein the one or more substituents on R^{b1} to R^{b5} are independently selected from D, partially fluorinated C₁ to C₈ alkyl, perfluorinated C₁ to C₈ alkyl, CF₃, or CN,
and wherein the asterisk "*" denotes the binding position.

24. The compound of formula (I) according to any of the preceding claims 1 to 23, wherein the compound of formula (I) is selected from a compound represented by I-1 to I-40:
| | | |
|---|---|---|
| 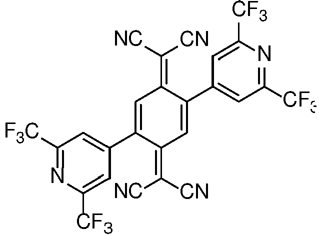 | 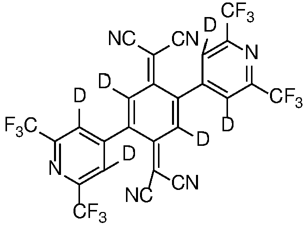 | 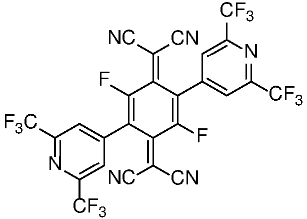 |
| I-1 | I-2 | I-3 |
| 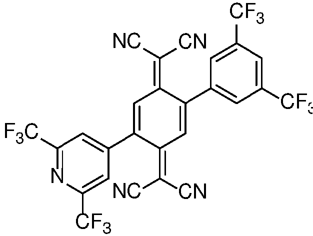 | 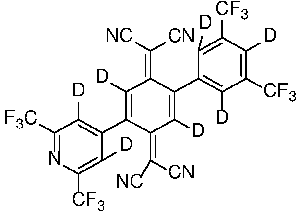 | 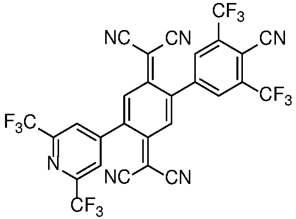 |
| I-4 | I-5 | I-6 |
| 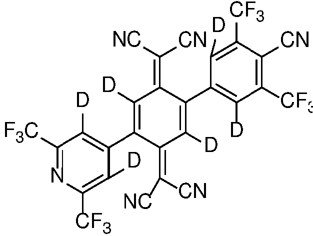 | 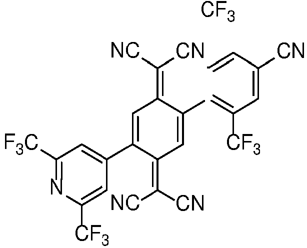 | 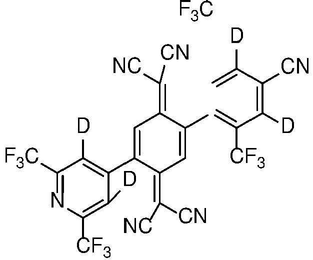 |
| I-7 | I-8 | I-9 |
| 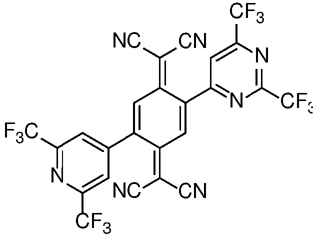 | 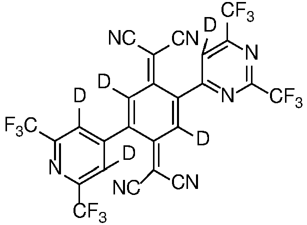 | 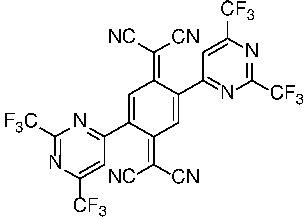 |
| I-10 | I-11 | I-12 |
| 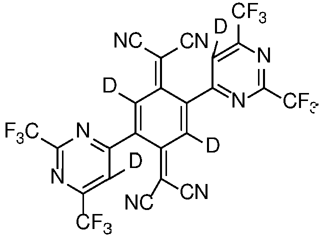 | 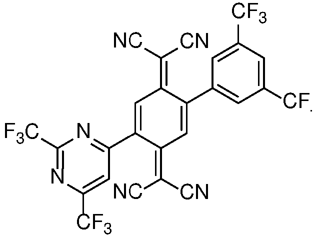 | 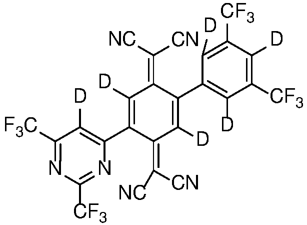 |
| I-13 | I-14 | I-15 |
| 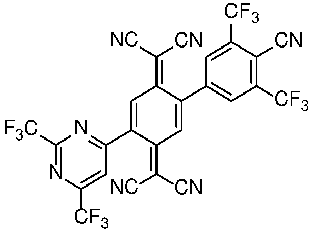 | 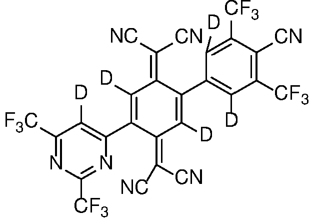 | 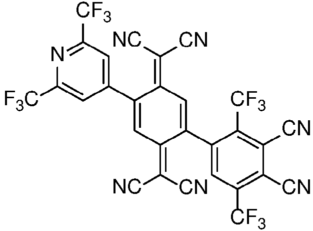 |
| I-16 | I-17 | I-18 |
| 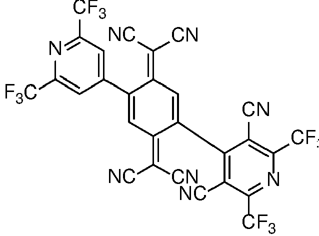 | 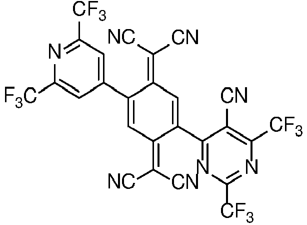 | 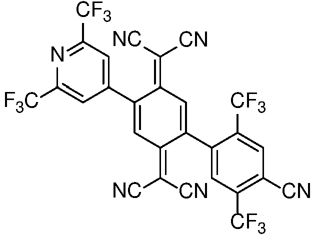 |
| I-19 | I-20 | I-21 |
| 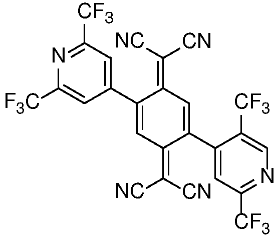 | 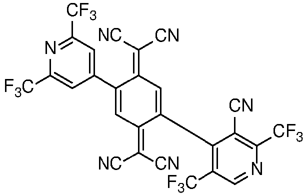 | 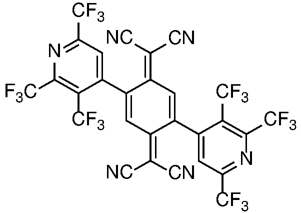 |
| I-22 | I-23 | I-24 |
| 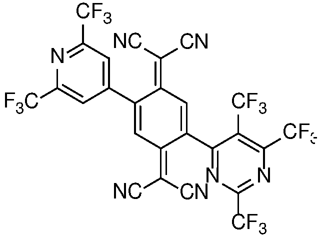 | 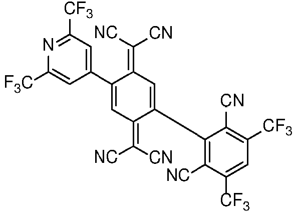 | 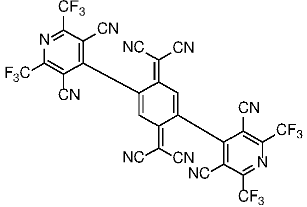 |
| I-25 | I-26 | I-27 |
| 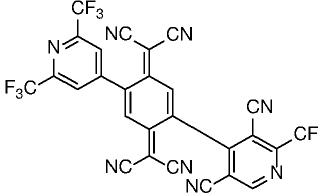 | 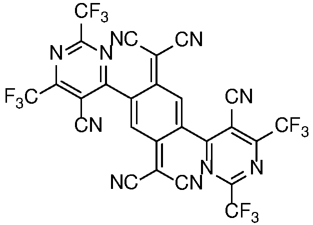 | 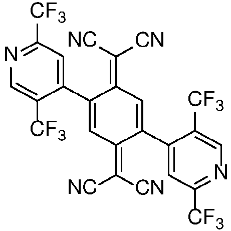 |
| I-28 | I-29 | I-30 |
| 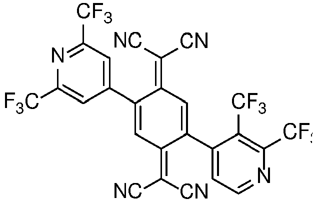 | 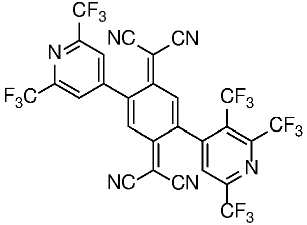 | 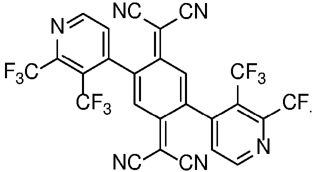 |
| I-32 | I-33 | I-34 |
| 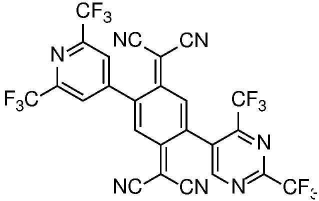 | 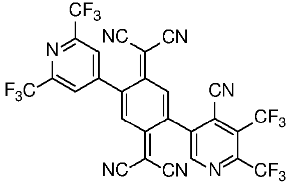 | 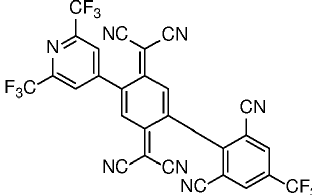 |
| I-35 | I-36 | I-37 |
| 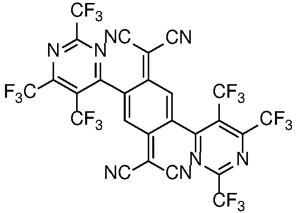 | 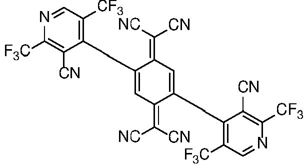 | 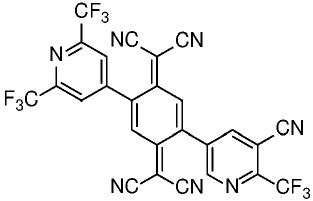 |
| I-38 | I-39 | I-40. |

25. An organic semiconductor layer, wherein the organic semiconductor layer comprises a compound of formula (I) according to claims 1 to 24, and preferably the compound of formula (VII).

26. The organic semiconductor layer according to claim 25, wherein the organic semiconductor layer comprises the compound of formula (I), preferably the compound of formula (VII), and a hole transport matrix compound.

27. The organic semiconductor layer according to claim 25 or 26, wherein the organic semiconductor layer is a hole injection layer or a p-type charge generation layer.

28. An organic electronic device, wherein the organic electronic device comprises an organic semiconductor layer according to claims 25 to 27.

29. An organic electronic device according to claim 28, wherein the organic electronic device comprises an anode layer, a cathode layer, a first photoactive layer, a second photoactive layer, a hole injection layer, a p-type charge generation layer, wherein the hole injection layer is arranged in direct contact to the anode layer, and wherein the p-type charge generation layer is arranged between the first photoactive layer and the second photoactive layer, wherein the hole injection layer is closer to the anode layer than the p-type charge generation layer, wherein the hole injection layer or the p-type charge generation layer is an organic semiconductor layer according to claims 25 to 27, wherein the first photoactive layer, the second photoactive layer, the hole injection layer, the p-type charge generation layer are arranged between the anode layer and cathode layer.

30. A display device comprising an organic electronic device according to claim 28 or 29.

## Patentansprüche

1. Verbindung der Formel (I): 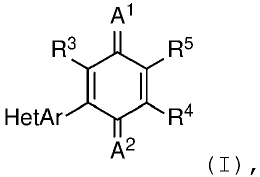 wobei
HetAr aus Formel (II) ausgewählt ist: 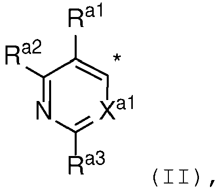
A¹ aus Formel (III) ausgewählt ist: 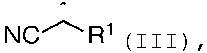
A² aus Formel (IV) ausgewählt ist: 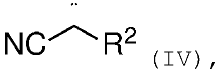
wobei das Sternchen "*" die Bindungsposition bezeichnet, R¹ und R² unabhängig aus CN, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, SF₅ ausgewählt sind,
wobei der eine oder die mehreren Substituenten an R¹ und R² unabhängig aus D, teilfluoriertem C₁- bis C₈-Alkyl,
perfluoriertem C₁- bis C₈-Alkyl, CF₃, Halogen, F, CN oder SF₅ ausgewählt sind;
R³ und R⁴ unabhängig aus H, D, Halogen, F, CN, teilfluoriertem C₁- bis C₄-Alkyl, perfluoriertem C₁- bis C₄-Alkyl, SF₅ oder CF₃ ausgewählt sind;
R⁵ unabhängig aus H, D, Halogen, F, CN, teilfluoriertem C₁- bis C₈-Alkyl, perfluoriertem C₁- bis C₈-Alkyl, CF₃, substituiertem oder unsubstituiertem C₆- bis C₃₀-Aryl oder substituiertem oder unsubstituiertem C₂- bis C₃₀-Heteroaryl, SF₅ ausgewählt ist,
wobei der eine oder die mehreren Substituenten an R⁵ unabhängig aus D, teilfluoriertem C₁- bis C₈-Alkyl oder perfluoriertem C₁- bis C₈-Alkyl, CF₃, Halogen, F, CN, SF₅ ausgewählt sind;
X^{a1} aus N oder CR^{a4} ausgewählt ist;
R^{a1}, R^{a2}, R^{a3} und R^{a4} unabhängig aus H, D, substituiertem oder unsubstituiertem C₁- bis C₈-Alkyl; teilfluoriertem C₁- bis C₈-Alkyl, perfluoriertem C₁- bis C₈-Alkyl, CF₃, substituiertem oder unsubstituiertem C₆-bis C₃₀-Aryl, substituiertem oder unsubstituiertem C₂- bis C₃₀-Heteroaryl, Halogen, F, CN, SF₅ ausgewählt sind,
wobei der eine oder die mehreren Substituenten an R^{a1}, R^{a2}, R^{a3} und R^{a4} unabhängig aus D, teilfluoriertem C¹- bis C₈-Alkyl oder perfluoriertem C₁- bis C₈-Alkyl, CF₃, Halogen, F, CN, SF₅ ausgewählt sind;
wobei mindestens eines von R^{a1}, R^{a2}, R^{a3} und R^{a4} aus teilfluoriertem C₁- bis C₈-Alkyl, perfluoriertem C₁- bis C₈-Alkyl, CF₃, Halogen, F, CN oder SF₃ ausgewählt ist.

2. Verbindung der Formel (I) nach Anspruch 1, wobei R¹ und R² unabhängig aus CN, teilfluoriertem C₁- bis C₄-Alkyl, perfluoriertem C₁- bis C₄-Alkyl, CF₃, substituiertem oder unsubstituiertem C₆- bis C₁₀-Aryl oder substituiertem oder unsubstituiertem C₃- bis C₉-Heteroaryl ausgewählt sind; vorzugsweise R¹ und R² unabhängig aus CN, teilfluoriertem C₁- bis C₄-Alkyl, perfluoriertem C₁- bis C₄-Alkyl, CF₃, substituiertem oder unsubstituiertem C₆-Aryl, substituiertem oder unsubstituiertem C₃- bis C₅-Heteroaryl ausgewählt sind;
und weiter bevorzugt R¹ und R² unabhängig aus CN, teilfluoriertem C₁- bis C₄-Alkyl, perfluoriertem C₁- bis C₄-Alkyl, CF₃, substituiertem oder unsubstituiertem C₆-Aryl, substituiertem oder unsubstituiertem C₁- bis C₅-Heteroaryl ausgewählt sind.

3. Verbindung der Formel (I) nach Anspruch 1 oder 2, wobei der eine oder die mehreren Substituenten an R¹ und R² unabhängig aus D, teilfluoriertem C₁- bis C₈-Alkyl, perfluoriertem C₁- bis C₈-Alkyl, CF₃ oder CN ausgewählt sind; vorzugsweise wobei der eine oder die mehreren Substituenten an R¹ und R² unabhängig aus D, teilfluoriertem C₁- bis C₄-Alkyl, perfluoriertem C₁- bis C₄-Alkyl, CF₃ oder CN ausgewählt sind; weiter bevorzugt der eine oder die mehreren Substituenten an R¹ und R² unabhängig aus D, CF₃, CN ausgewählt sind.

4. Verbindung der Formel (I) nach einem der vorhergehenden Ansprüche 1 bis 3, wobei A¹ und A² aus Formel (V) ausgewählt sind: 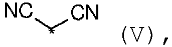 wobei das Sternchen "*" die Bindungsposition bezeichnet.

5. Verbindung der Formel (I) nach einem der vorhergehenden Ansprüche 1 bis 4, wobei R³ und R⁴ unabhängig aus H, D, CN, teilfluoriertem C₁- bis C₄-Alkyl, perfluoriertem C₁- bis C₄-Alkyl oder CF₃ ausgewählt sind, vorzugsweise R³ und R⁴ unabhängig aus H, D, CN, CF₃ ausgewählt sind und weiter bevorzugt R³ und R⁴ unabhängig aus H, D ausgewählt sind.

6. Verbindung der Formel (I) nach einem der vorhergehenden Ansprüche 1 bis 5, wobei R⁵ aus H, D, CN, CF₃, substituiertem oder unsubstituiertem C₆-Aryl, substituiertem oder unsubstituiertem C₄- bis C₅-Heteroaryl ausgewählt ist; vorzugsweise R⁵ aus H, D, CN, substituiertem oder unsubstituiertem C₆-Aryl oder substituiertem oder unsubstituiertem C₄- bis C₅-Heteroaryl ausgewählt ist; oder weiter bevorzugt R⁵ aus substituiertem oder unsubstituiertem C₆-Aryl oder substituiertem oder unsubstituiertem C₄- bis C₅-Heteroaryl ausgewählt ist.

7. Verbindung der Formel (I) nach einem der vorhergehenden Ansprüche 1 bis 6, wobei der eine oder die mehreren Substituenten an R⁵ unabhängig aus D, teilfluoriertem C₁- bis C₄-Alkyl, perfluoriertem C₁- bis C₄-Alkyl, CF₃, Halogen, F oder CN ausgewählt sind; vorzugsweise der eine oder die mehreren Substituenten an R⁵ unabhängig aus D, teilfluoriertem C₁- bis C₄-Alkyl, perfluoriertem C₁- bis C₄-Alkyl, CF₃ oder CN ausgewählt sind; und weiter bevorzugt der eine oder die mehreren Substituenten an R⁵ unabhängig aus D, CF₃ oder CN ausgewählt sind.

8. Verbindung der Formel (I) nach einem der vorhergehenden Ansprüche 1 bis 7, wobei eine CN-Gruppe nicht an ein aromatisches Kohlenstoffatom gebunden ist, wenn der aromatische Kohlenstoff direkt an ein sp2-hybridisiertes Ringstickstoffatom gebunden ist.

9. Verbindung der Formel (I) nach einem der vorhergehenden Ansprüche 1 bis 8, wobei R⁵ aus Formel (VIa) ausgewählt ist: 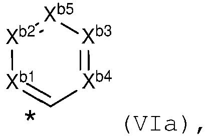 wobei das "*" die Bindungsposition bezeichnet, und
wobei
X^{b1} aus N oder CR^{b1} ausgewählt ist,
X^{b2} aus N oder CR^{b2} ausgewählt ist,
X^{b3} aus N oder CR^{b3} ausgewählt ist,
X^{b4} aus N oder CR^{b4} ausgewählt ist,
X^{b5} aus N oder CR^{b5} ausgewählt ist,
R^{b1} bis R^{b5} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F oder CN ausgewählt sind,
wobei der eine oder die mehreren Substituenten an R^{b1} bis R^{b5} unabhängig aus D, teilfluoriertem C₁- bis C₈-Alkyl, perfluoriertem C₁- bis C₈-Alkyl, CF₃, Halogen, F oder CN ausgewählt sind;
vorzugsweise mindestens eines von R^{b1} bis R^{b5} aus teilfluoriertem oder perfluoriertem C₁- bis C₈-Alkyl, CF₃, Halogen, F oder CN ausgewählt ist; und weiter bevorzugt mindestens eines von R^{b1} bis R^{b5} aus teilfluoriertem oder perfluoriertem C₁- bis C₈-Alkyl, CF₃ oder CN ausgewählt ist.

10. Verbindung der Formel (I) nach einem der vorhergehenden Ansprüche 1 bis 9, wobei dann, wenn R^{bn} für N mit n=1 bis 5 steht, das benachbarte R^{bm} mit m=1 bis 5 nicht für eine CN-Gruppe steht.

11. Verbindung der Formel (I) nach einem der vorhergehenden Ansprüche 1 bis 10, wobei R⁵ aus Formel (VIb) ausgewählt ist: 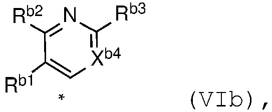 wobei das "*" die Bindungsposition bezeichnet, und
wobei
X^{b4} aus N oder CR^{b4} ausgewählt ist,
R^{b1} bis R^{b3} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt sind,
wobei der eine oder die mehreren Substituenten an R^{b1} bis R^{b3} unabhängig aus D, teilfluoriertem C₁- bis C₈-Alkyl, perfluoriertem C₁- bis C₈-Alkyl, CF₃, Halogen, F, CN ausgewählt sind,
vorzugsweise mindestens eines von R^{b1} bis R^{b3} aus teilfluoriertem oder perfluoriertem C₁- bis C₈-Alkyl, CF₃, Halogen, F oder CN ausgewählt ist; und weiter bevorzugt mindestens eines von R^{b1} bis R^{b3} aus teilfluoriertem oder perfluoriertem C₁- bis C₈-Alkyl, CF₃ oder CN ausgewählt ist.

12. Verbindung der Formel (I) nach einem der vorhergehenden Ansprüche 1 bis 11, wobei R⁵ aus Formel (VIc) ausgewählt ist: 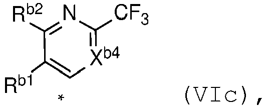 wobei das "*" die Bindungsposition bezeichnet, und
wobei
X^{b4} aus N oder CR^{b4} ausgewählt ist,
R^{b1} und R^{b2} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt sind,
wobei der eine oder die mehreren Substituenten an R^{b1} und R^{b2} unabhängig aus D, teilfluoriertem C₁- bis C₈-Alkyl, perfluoriertem C₁- bis C₈-Alkyl, CF₃, Halogen, F, CN ausgewählt sind,
vorzugsweise mindestens eines von R^{b1} und R^{b2} aus teilfluoriertem oder perfluoriertem C₁- bis C₈-Alkyl, CF₃, Halogen, F oder CN ausgewählt ist; und weiter bevorzugt mindestens eines von R^{b1} und R^{b2} aus teilfluoriertem oder perfluoriertem C₁- bis C₈-Alkyl, CF₃ oder CN ausgewählt ist.

13. Verbindung der Formel (I) nach einem der vorhergehenden Ansprüche 1 bis 12, wobei R⁵ aus Formel (VId) ausgewählt ist: 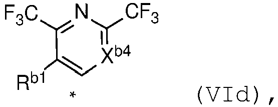 wobei das "*" die Bindungsposition bezeichnet, und
wobei
X^{b4} aus N oder CR^{b4} ausgewählt ist,
R^{b1} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt ist,
wobei der eine oder die mehreren Substituenten an R^{b1} unabhängig aus D, teilfluoriertem C₁- bis C₈-Alkyl, perfluoriertem C₁- bis C₈-Alkyl, CF₃, Halogen, F, CN ausgewählt sind,
vorzugsweise R^{b1} aus teilfluoriertem oder perfluoriertem C₁- bis C₈-Alkyl, CF₃, Halogen, F oder CN ausgewählt ist; und weiter bevorzugt R^{b1} aus teilfluoriertem oder perfluoriertem C₁- bis C₈-Alkyl, CF₃ oder CN ausgewählt ist.

14. Verbindung der Formel (I) nach einem der vorhergehenden Ansprüche 1 bis 13, wobei R⁵ aus Formel (VIe) ausgewählt ist: 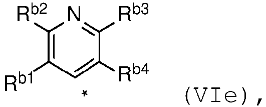 wobei das "*" die Bindungsposition bezeichnet, und
wobei
R^{b1} bis R^{b4} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt sind,
wobei der eine oder die mehreren Substituenten an R^{b1} bis R^{b4} unabhängig aus D, teilfluoriertem C₁- bis C₈-Alkyl, perfluoriertem C₁- bis C₈-Alkyl, CF₃, Halogen, F, CN ausgewählt sind,
vorzugsweise mindestens eines von R^{b1} bis R^{b4} aus teilfluoriertem oder perfluoriertem C₁- bis C₈-Alkyl, CF₃, Halogen, F oder CN ausgewählt sind; und weiter bevorzugt mindestens eines von R^{b1} bis R^{b4} aus teilfluoriertem oder perfluoriertem C₁- bis C₈-Alkyl, CF₃ oder CN ausgewählt sind.

15. Verbindung der Formel (I) nach einem der vorhergehenden Ansprüche 1 bis 14, wobei R⁵ aus Formel (VIf) ausgewählt ist: 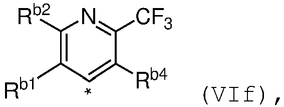 wobei das "*" die Bindungsposition bezeichnet, und
wobei
R^{b1}, R^{b2} und R^{b4} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt sind,
wobei der eine oder die mehreren Substituenten an R^{b1}, R^{b2} und R^{b4} unabhängig aus D, teilfluoriertem C₁- bis C₈-Alkyl, perfluoriertem C₁- bis C₈-Alkyl, CF₃, Halogen, F, CN ausgewählt sind,
vorzugsweise mindestens eines von R^{b1}, R^{b2} und R^{b4} aus teilfluoriertem oder perfluoriertem C₁- bis C₈-Alkyl, CF₃, Halogen, F oder CN ausgewählt sind; und weiter bevorzugt mindestens eines von R^{b1} bis R^{b4} aus teilfluoriertem oder perfluoriertem C₁- bis C₈-Alkyl, CF₃ oder CN ausgewählt sind.

16. Verbindung der Formel (I) nach einem der vorhergehenden Ansprüche 1 bis 15, wobei R⁵ aus Formel (VIg) ausgewählt ist: 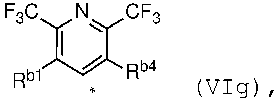 wobei das "*" die Bindungsposition bezeichnet, und
wobei
R^{b1} und R^{b4} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F, CN ausgewählt sind,
wobei der eine oder die mehreren Substituenten an R^{b1} und R^{b4} unabhängig aus D, teilfluoriertem C₁- bis C₈-Alkyl, perfluoriertem C₁- bis C₈-Alkyl, CF₃, Halogen, F, CN ausgewählt sind,
vorzugsweise mindestens eines von R^{b1} und R^{b4} aus teilfluoriertem oder perfluoriertem C₁- bis C₈-Alkyl, CF₃, Halogen, F oder CN ausgewählt sind; und weiter bevorzugt mindestens eines von R^{b1} und R^{b4} aus teilfluoriertem oder perfluoriertem C₁- bis C₈-Alkyl, CF₃ oder CN ausgewählt sind.

17. Verbindung der Formel (I) nach einem der vorhergehenden Ansprüche 1 bis 16, wobei R^{a1} bis R^{a4} in Formel I, R^{b1} bis R^{b5} in Formel (VIa), R^{b1} bis R^{b3} in Formel (VIb), R^{b1} bis R^{b2} in Formel (VIc), R^{b1} in Formel (VId), R^{b1} bis R^{b4} in Formel (VIe), R^{b1}, R^{b2} und R^{b4} in Formel (VIf), R^{b1} und R^{b4} in Formel (VIg) nicht als Halogen oder F ausgewählt sind; und vorzugsweise R^{a1} bis R^{a4} in Formel I, R^{b1} bis R^{b5} in Formel (VIa), R^{b1} bis R^{b3} in Formel (VIb), R^{b1} bis R^{b2} in Formel (VIc), R^{b1} in Formel (VId), R^{b1} bis R^{b4} in Formel (VIe), R^{b1}, R^{b2} und R^{b4} in Formel (VIf), R^{b1} und R^{b4} in Formel (VIg) nicht als Halogen oder F ausgewählt sind und der eine oder die mehreren Substituenten an R^{a1} bis R^{a4}, R^{b1} bis R^{b5} nicht aus Halogen oder F ausgewählt sind.

18. Verbindung der Formel (I) nach einem der vorhergehenden Ansprüche 1 bis 17, wobei:
R^{a2} bis R^{a3} in Formel (II), R^{b2} bis R^{b3} in Formel (VIa), (VIb), (VIe), und R^{b2} in Formel (VIc) und (VIf) nicht als CN ausgewählt sind.

19. Verbindung der Formel (I) nach einem der vorhergehenden Ansprüche 1 bis 18, wobei R⁵ aus einer durch B1 bis B160 wiedergegebenen Gruppe ausgewählt ist:
| | | | |
|---|---|---|---|
| 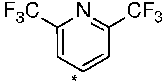 | 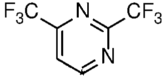 | 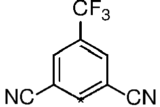 | 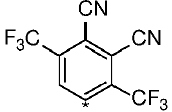 |
| B1 | B2 | B3 | B4 |
| 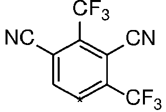 | 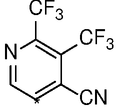 | 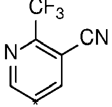 | 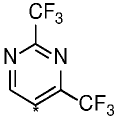 |
| B5 | B6 | B7 | B8 |
| 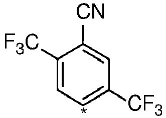 | 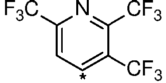 | 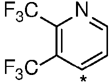 | 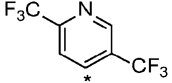 |
| B9 | B10 | B11 | B12 |
| 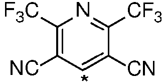 | 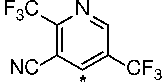 | 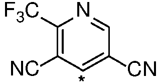 | 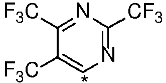 |
| B13 | B14 | B15 | B16 |
| 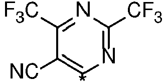 | 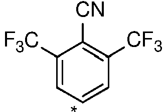 | 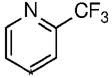 | 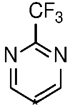 |
| B17 | B18 | B19 | B20 |
| 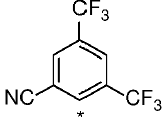 | 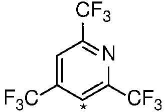 | 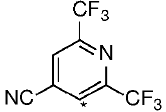 | 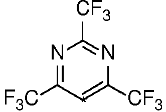 |
| B21 | B22 | B23 | B24 |
| 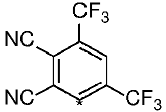 | 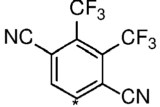 | 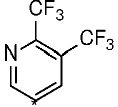 | 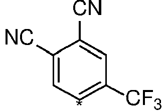 |
| B25 | B26 | B27 | B28 |
| 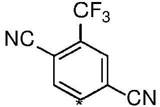 | 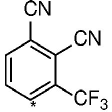 | 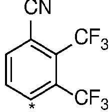 | 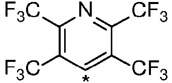 |
| B29 | B30 | B31 | B32 |
| 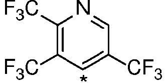 | 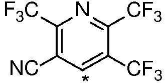 | 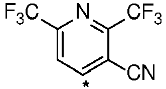 | 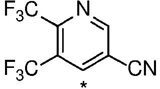 |
| B33 | B34 | B35 | B36 |
| 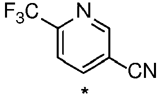 | 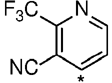 | 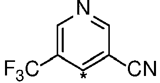 | 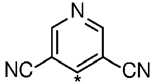 |
| B37 | B38 | B39 | B40 |
| 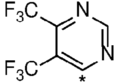 | 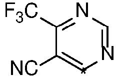 | 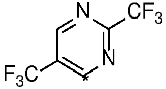 | 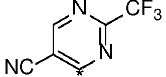 |
| B41 | B42 | B43 | B44 |
| 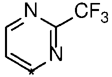 | 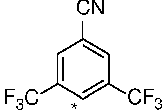 | 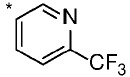 | 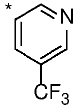 |
| B45 | B46 | B47 | B48 |
| 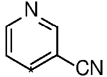 | 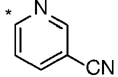 | 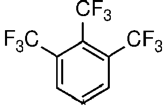 | 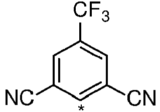 |
| B49 | B50 | B51 | B52 |
| 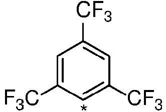 | 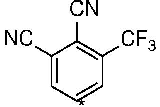 | 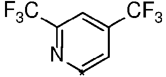 | 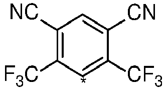 |
| B53 | B54 | B55 | B56 |
| 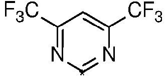 | 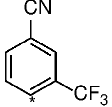 | 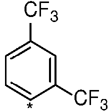 | 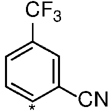 |
| B57 | B58 | B59 | B60 |
| 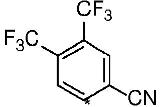 | 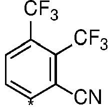 | 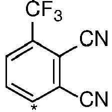 | 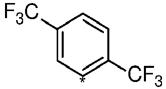 |
| B61 | B62 | B63 | B64 |
| 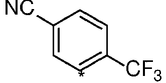 | 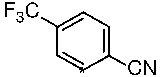 | 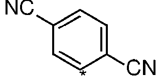 | 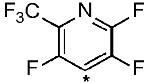 |
| B65 | B66 | B67 | B68 |
| 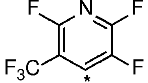 | 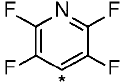 | 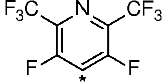 | 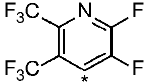 |
| B69 | B70 | B71 | B72 |
| 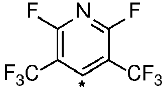 | 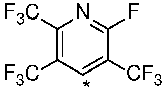 | 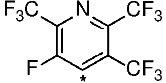 | 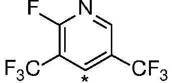 |
| B73 | B74 | B75 | B76 |
| 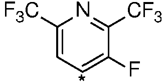 | 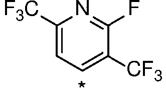 | 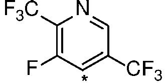 | 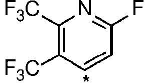 |
| B77 | B78 | B79 | B80 |
| 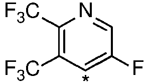 | 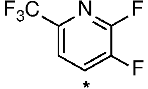 | 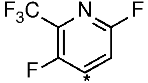 | 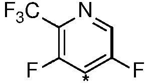 |
| B81 | B82 | B83 | B84 |
| 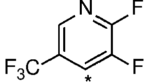 | 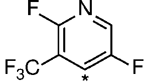 | 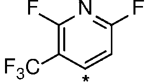 | 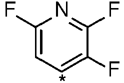 |
| B85 | B86 | B87 | B88 |
| 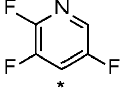 | 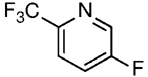 | 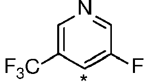 | 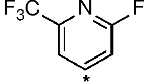 |
| B89 | B90 | B91 | B92 |
| 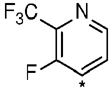 | 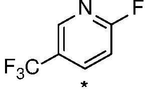 | 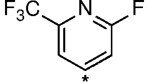 | 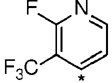 |
| B93 | B94 | B95 | B96 |
| 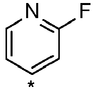 | 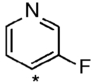 | 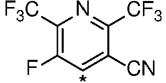 | 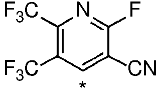 |
| B97 | B98 | B99 | B100 |
| 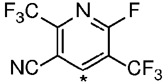 | 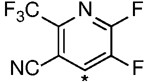 | 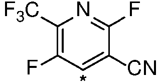 | 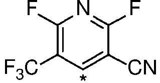 |
| B101 | B102 | B103 | B104 |
| 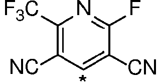 | 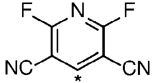 | 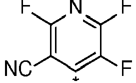 | 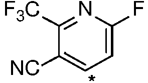 |
| B105 | B106 | B107 | B108 |
| 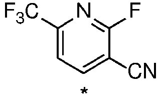 | 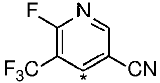 | 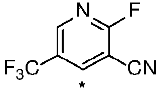 | 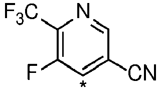 |
| B109 | B110 | B111 | B112 |
| 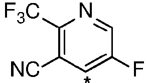 | 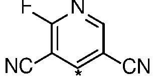 | 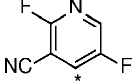 | 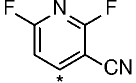 |
| B113 | B114 | B115 | B116 |
| 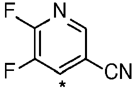 | 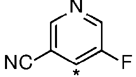 | 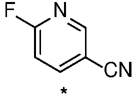 | 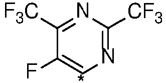 |
| B117 | B118 | B119 | B120 |
| 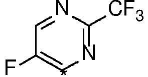 | 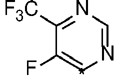 | 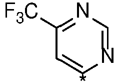 | 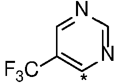 |
| B121 | B122 | B123 | B124 |
| 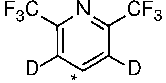 | 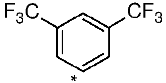 | 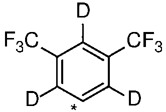 | 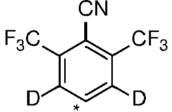 |
| B125 | B126 | B127 | B128 |
| 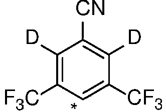 | 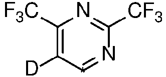 | 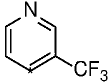 | 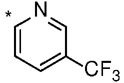 |
| B129 | B130 | B131 | B132 |
| 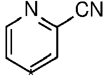 | 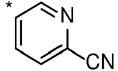 | 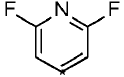 | 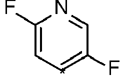 |
| B133 | B134 | B135 | B136 |
| 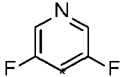 | 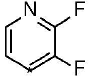 | 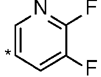 | 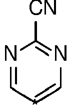 |
| B137 | B138 | B139 | B140 |
| 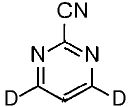 | 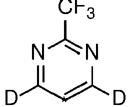 | 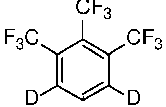 | 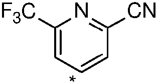 |
| B141 | B142 | B143 | B144 |
| 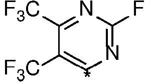 | 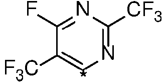 | 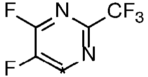 | 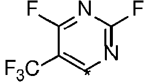 |
| B145 | B146 | B147 | B148 |
| 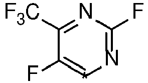 | 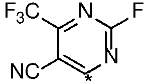 | 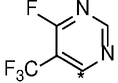 | 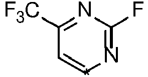 |
| B149 | B150 | B151 | B152 |
| 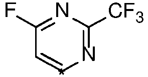 | 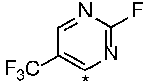 | 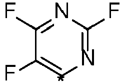 | 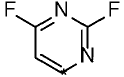 |
| B153 | B154 | B155 | B156 |
| 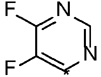 | 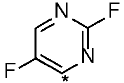 | 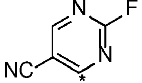 | 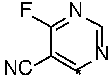 |
| B157 | B158 | B159 | B160 |
, wobei das "*" die Bindungsposition bezeichnet.

20. Verbindung der Formel (I) nach einem der vorhergehenden Ansprüche 1 bis 19, wobei Formel (II) aus den Formeln (IIa), (IIb), (IIc), (IId), (IIe) und (IIf) ausgewählt ist: 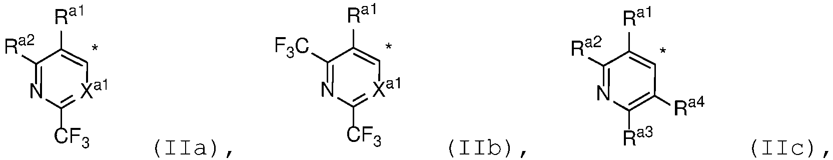 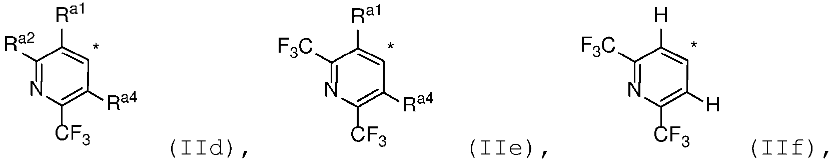 wobei das "*" die Bindungsposition bezeichnet, und vorzugsweise wobei die Formeln (IIa), (IIb), (IIc), (IId), (IIe) und (IIf) teilweise deuteriert oder vollständig deuteriert sind und weiter bevorzugt Formel (IIf) teilweise deuteriert oder vollständig deuteriert ist.

21. Verbindung der Formel (I) nach einem der vorhergehenden Ansprüche 1 bis 20, wobei HetAr aus einer durch C1 bis C109 wiedergegebenen Gruppe ausgewählt ist:
| | | | |
|---|---|---|---|
| 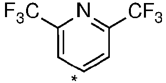 | 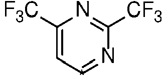 | 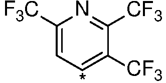 | 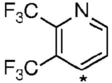 |
| C1 | C2 | C3 | C4 |
| 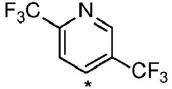 | 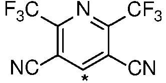 | 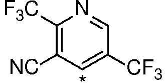 | 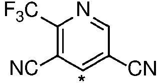 |
| C5 | C6 | C7 | C8 |
| 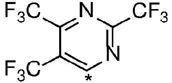 | 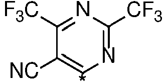 | 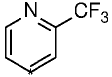 | 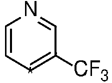 |
| C9 | C10 | C11 | C12 |
| 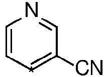 | 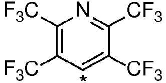 | 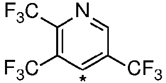 | 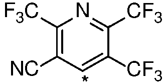 |
| C13 | C14 | C15 | C16 |
| 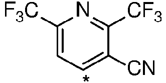 | 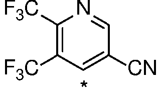 | 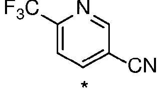 | 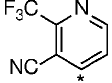 |
| C17 | C18 | C19 | C20 |
| 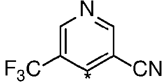 | 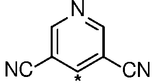 | 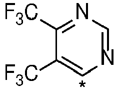 | 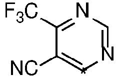 |
| C21 | C22 | C23 | C24 |
| 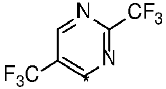 | 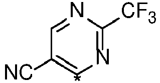 | 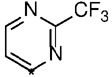 | 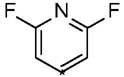 |
| C25 | C26 | C27 | C28 |
| 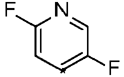 | 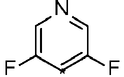 | 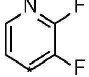 | 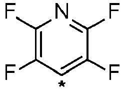 |
| C29 | C30 | C31 | C32 |
| 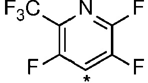 | 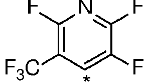 | 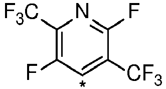 | 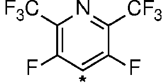 |
| C33 | C34 | C35 | C36 |
| 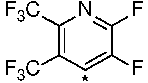 | 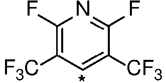 | 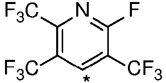 | 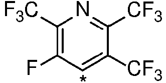 |
| C37 | C38 | C39 | C40 |
| 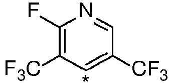 | 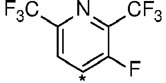 | 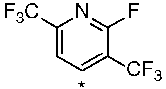 | 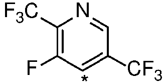 |
| C41 | C42 | C43 | C44 |
| 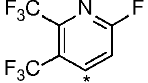 | 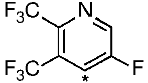 | 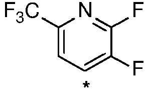 | 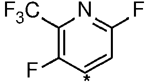 |
| C45 | C46 | C47 | C48 |
| 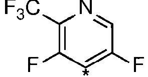 | 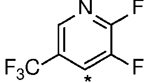 | 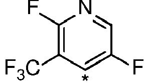 | 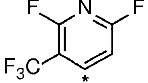 |
| C49 | C50 | C51 | C52 |
| 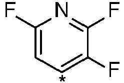 | 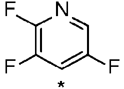 | 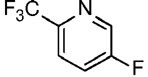 | 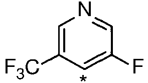 |
| C53 | C54 | C55 | C56 |
| 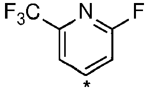 | 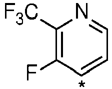 | 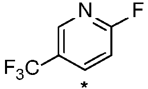 | 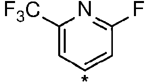 |
| C57 | C58 | C59 | C60 |
| 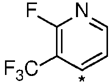 | 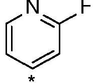 | 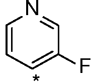 | 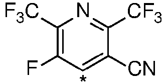 |
| C61 | C62 | C63 | C64 |
| 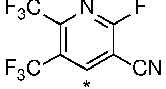 | 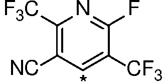 | 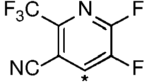 | 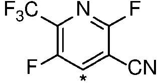 |
| C65 | C66 | C67 | C68 |
| 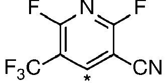 | 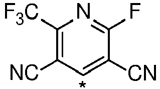 | 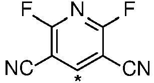 | 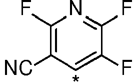 |
| C69 | C70 | C71 | C72 |
| 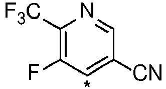 | 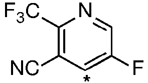 | 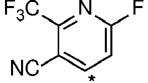 | 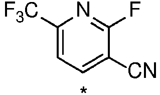 |
| C73 | C74 | C75 | C76 |
| 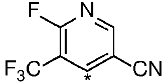 | 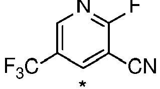 | 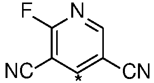 | 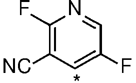 |
| C77 | C78 | C79 | C80 |
| 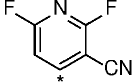 | 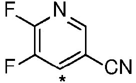 | 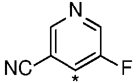 | 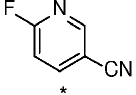 |
| C81 | C82 | C83 | C84 |
| 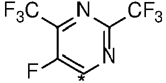 | 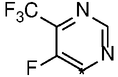 | 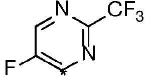 | 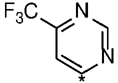 |
| C85 | C86 | C87 | C88 |
| 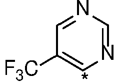 | 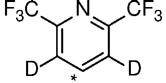 | 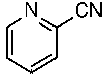 | 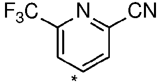 |
| C89 | C90 | C91 | C92 |
| 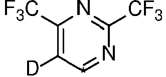 | 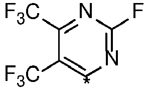 | 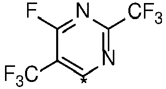 | 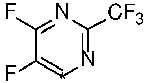 |
| C93 | C94 | C95 | C96 |
| 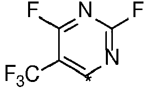 | 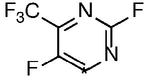 | 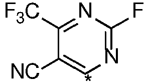 | 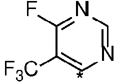 |
| C97 | C98 | C99 | C100 |
| 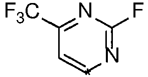 | 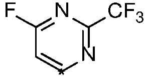 | 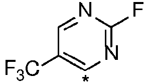 | 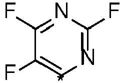 |
| C101 | C102 | C103 | C104 |
| 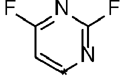 | 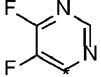 | 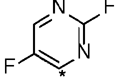 | 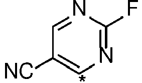 |
| C105 | C106 | C107 | C108 |
| 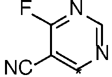 | | | |
| C109 | | | |
, wobei das "*" die Bindungsposition bezeichnet.

22. Verbindung der Formel (I) nach einem der vorhergehenden Ansprüche 1 bis 21, wobei die Verbindung der Formel (I) aus einer Verbindung der Formel (VII) ausgewählt ist: 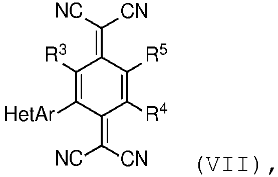 wobei
HetAr aus den Formeln (II), (IIa), (IIb), (IIc), (IId), (IIe) und (IIf) ausgewählt ist: 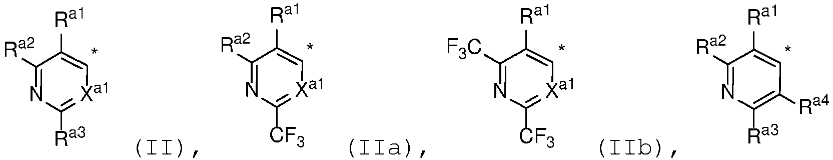 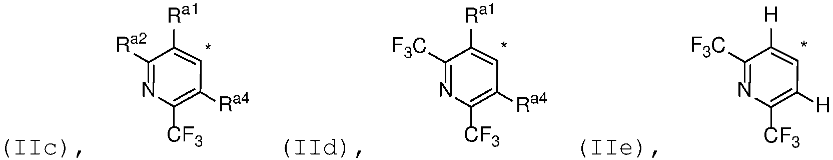
(IIf), wobei das Sternchen "*"
die Bindungsposition bezeichnet und
wobei
R³ und R⁴ aus H, D ausgewählt sind;
X^{a1} aus N oder CR^{a4} ausgewählt ist;
R^{a1} bis R^{a4} unabhängig aus H, D, substituiertem oder unsubstituiertem C₁- bis C₈-Alkyl; teilfluoriertem C₁- bis C₈-Alkyl oder perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F oder CN ausgewählt sind,
wobei der eine oder die mehreren Substituenten an R^{a1} bis R^{a4} unabhängig aus D, teilfluoriertem C₁- bis C₈-Alkyl oder perfluoriertem C₁- bis C₈-Alkyl, CF₃, CN ausgewählt sind;
mindestens eines von R^{a1} bis R^{a4} aus teilfluoriertem C₁-bis C₈-Alkyl oder perfluoriertem C₁- bis C₈-Alkyl, CF₃ oder CN ausgewählt ist;
R⁵ aus H, D, CN, teilfluoriertem C₁- bis C₈-Alkyl oder perfluoriertem C₁- bis C₈-Alkyl, CF₃, substituiertem oder unsubstituiertem C₆- bis C₃₀-Aryl oder substituiertem oder unsubstituiertem C₂- bis C₃₀-Heteroaryl ausgewählt ist,
wobei der eine oder die mehreren Substituenten an R⁵ unabhängig aus D, teilfluoriertem C₁- bis C₈-Alkyl oder perfluoriertem C₁- bis C₈-Alkyl, CF₃ oder CN ausgewählt sind.

23. Verbindung der Formel (I) nach einem der vorhergehenden Ansprüche 1 bis 22, wobei die Verbindung der Formel (I) aus einer Verbindung der Formel (VII) ausgewählt ist: 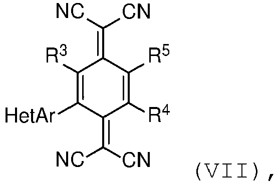 wobei
HetAr aus den Formeln (II), (IIa), (IIb), (IIc), (IId), (IIe) und (IIf) ausgewählt ist: 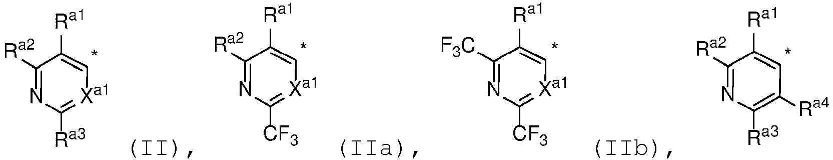 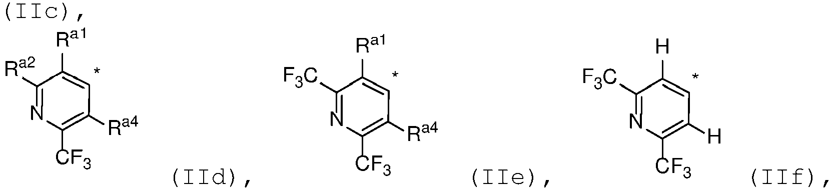
wobei das Sternchen "*" die Bindungsposition bezeichnet, und
wobei
R³ und R⁴ aus H, D ausgewählt sind;
X^{a1} aus N oder CR^{a4} ausgewählt ist;
R^{a1} bis R^{a4} unabhängig aus H, D, substituiertem oder unsubstituiertem C₁- bis C₈-Alkyl; teilfluoriertem C₁- bis C₈-Alkyl oder perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl, Halogen, F oder CN ausgewählt sind,
wobei der eine oder die mehreren Substituenten an R^{a1} bis R^{a4} unabhängig aus D, teilfluoriertem C₁- bis C₈-Alkyl oder perfluoriertem C₁- bis C₈-Alkyl, CF₃, CN ausgewählt sind;
mindestens eines von R^{a1} bis R^{a4} aus teilfluoriertem C₁-bis C₈-Alkyl oder perfluoriertem C₁- bis C₈-Alkyl, CF₃ oder CN ausgewählt ist;
R⁵ aus Formel (VIa) ausgewählt ist: 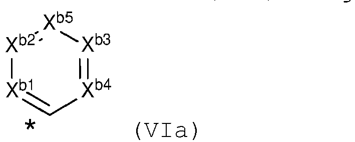
wobei
X^{b1} aus N oder CR^{b1} ausgewählt ist,
X^{b2} aus N oder CR^{b2} ausgewählt ist,
X^{b3} aus N oder CR^{b3} ausgewählt ist,
X^{b4} aus N oder CR^{b4} ausgewählt ist,
X^{b5} aus N oder CR^{b5} ausgewählt ist,
R^{b1} bis R^{b5} unabhängig aus H, D, substituiertem oder unsubstituiertem C₁- bis C₈-Alkyl, teilfluoriertem C₁- bis C₈-Alkyl, perfluoriertem C₁- bis C₈-Alkyl, CF₃, substituiertem oder unsubstituiertem C₆- bis C₃₀-Aryl, substituiertem oder unsubstituiertem C₂- bis C₃₀-Heteroaryl oder CN ausgewählt sind,
wobei der eine oder die mehreren Substituenten an R^{b1} bis R^{b5} unabhängig aus D, teilfluoriertem C₁- bis C₈-Alkyl, perfluoriertem C₁- bis C₈-Alkyl, CF₃ oder CN ausgewählt sind,
und wobei das Sternchen "*" die Bindungsposition bezeichnet.

24. Verbindung der Formel (I) nach einem der vorhergehenden Ansprüche 1 bis 23, wobei die Verbindung der Formel (I) aus einer durch I-1 bis I-40 wiedergegebenen Verbindung ausgewählt ist:
| | | |
|---|---|---|
| 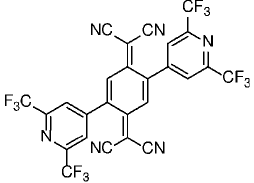 | 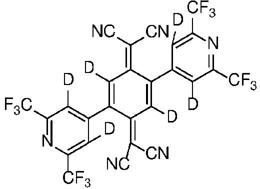 | 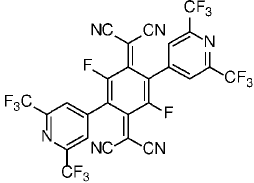 |
| I-1 | I-2 | I-3 |
| 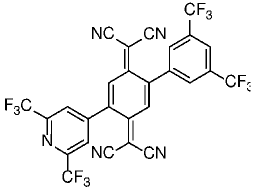 | 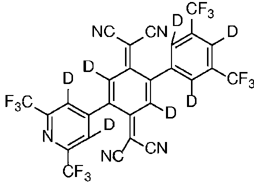 | 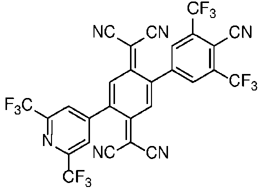 |
| I-4 | I-5 | I-6 |
| 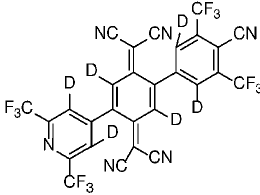 | 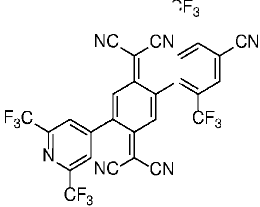 | 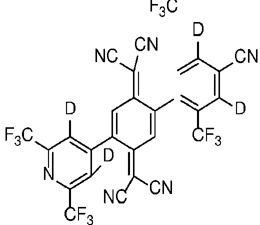 |
| I-7 | I-8 | I-9 |
| 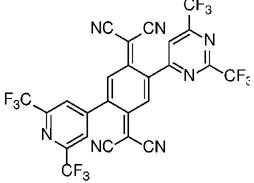 | 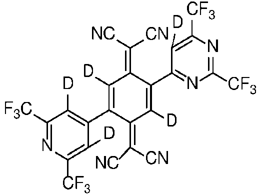 | 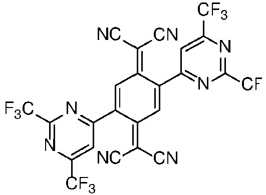 |
| I-10 | I-11 | I-12 |
| 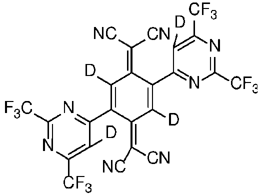 | 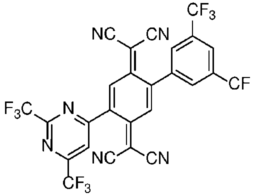 | 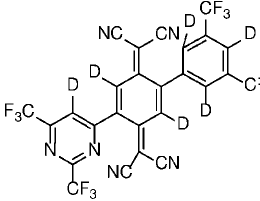 |
| I-13 | I-14 | I-15 |
| 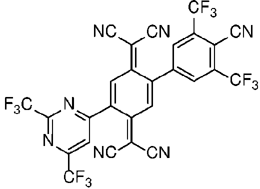 | 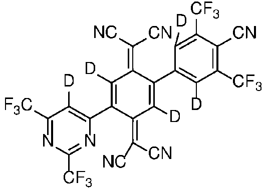 | 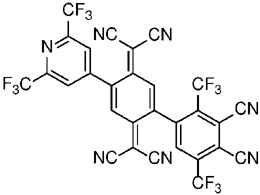 |
| I-16 | I-17 | I-18 |
| 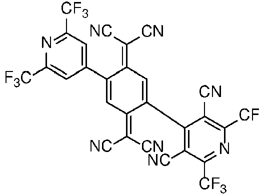 | 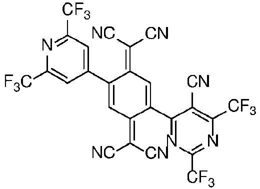 | 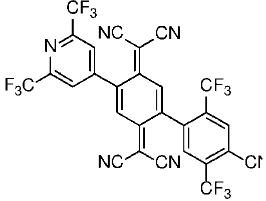 |
| I-19 | I-20 | I-21 |
| 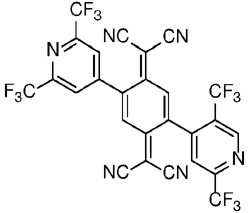 | 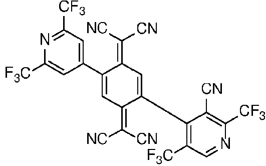 | 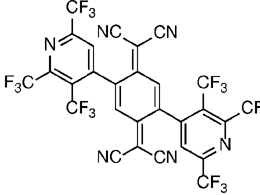 |
| I-22 | I-23 | I-24 |
| 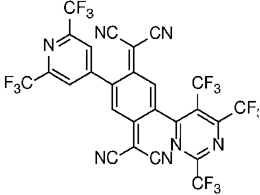 | 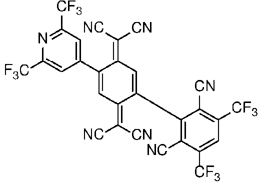 | 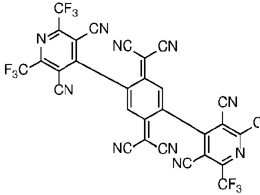 |
| I-25 | I-26 | I-27 |
| 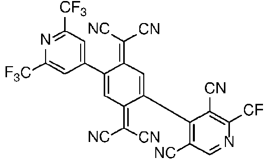 | 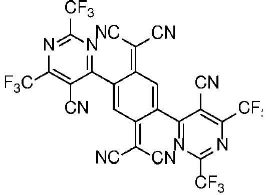 | 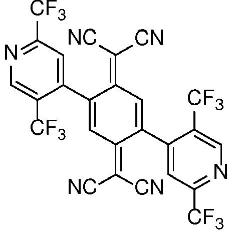 |
| I-28 | I-29 | I-30 |
| 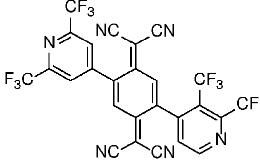 | 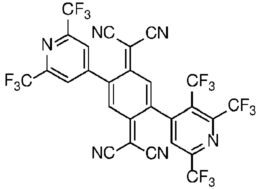 | 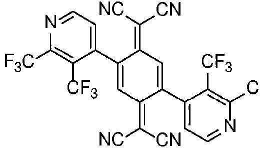 |
| I-32 | I-33 | I-34 |
| 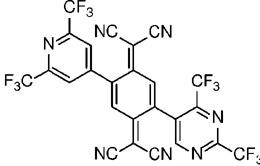 | 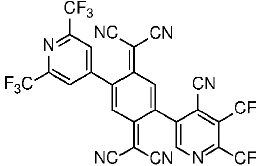 | 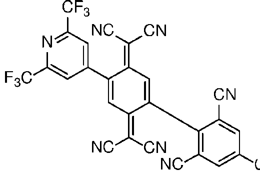 |
| I-35 | I-36 | I-37 |
| 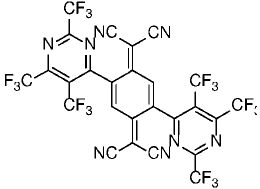 | 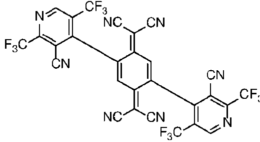 | 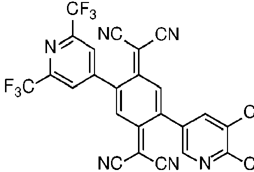 |
| I-38 | I-39 | I-40. |

25. Organische Halbleiterschicht, wobei die organische Halbleiterschicht eine Verbindung der Formel (I) nach den Ansprüchen 1 bis 24 und vorzugsweise die Verbindung der Formel (VII) umfasst.

26. Organische Halbleiterschicht nach Anspruch 25, wobei die organische Halbleiterschicht die Verbindung der Formel (I), vorzugsweise die Verbindung der Formel (VII) und eine Lochtransportmatrixverbindung umfasst.

27. Organische Halbleiterschicht nach Anspruch 25 oder 26, wobei es sich bei der organischen Halbleiterschicht um eine Lochinjektionsschicht oder eine Ladungserzeugungsschicht vom p-Typ handelt.

28. Organische elektronische Vorrichtung, wobei die organische elektronische Vorrichtung eine organische Halbleiterschicht nach den Ansprüchen 25 bis 27 umfasst.

29. Organische elektronische Vorrichtung nach Anspruch 28, wobei die organische elektronische Vorrichtung eine Anodenschicht, eine Kathodenschicht, eine erste photoaktive Schicht, eine zweite photoaktive Schicht, eine Lochinjektionsschicht, eine Ladungserzeugungsschicht vom p-Typ umfasst, wobei die Lochinjektionsschicht in direktem Kontakt mit der Anodenschicht angeordnet ist und wobei die Ladungserzeugungsschicht vom p-Typ zwischen der ersten photoaktiven Schicht und der zweiten photoaktiven Schicht angeordnet ist, wobei sich die Lochinjektionsschicht mehr an der Anodenschicht befindet als die Ladungserzeugungsschicht vom p-Typ, wobei es sich bei der Lochinjektionsschicht oder der Ladungserzeugungsschicht vom p-Typ um eine organische Halbleiterschicht nach den Ansprüchen 25 bis 27 handelt, wobei die erste photoaktive Schicht, die zweite photoaktive Schicht, die Lochinjektionsschicht und die Ladungserzeugungsschicht vom p-Typ zwischen der Anodenschicht und der Kathodenschicht angeordnet sind.

30. Anzeigevorrichtung, umfassend eine organische elektronische Vorrichtung nach Anspruch 28 oder 29.

## Revendications

1. Composé de formule (I) : 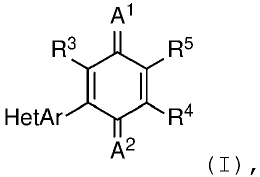
HetAr étant choisi parmi la formule (II) : 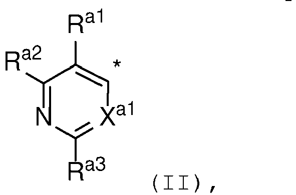
A¹ étant choisi parmi la formule (III) : 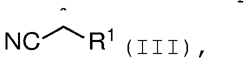
A² étant choisi parmi la formule (IV) 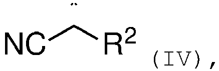
l'astérisque « * » désignant la position de liaison,
R¹ et R² étant indépendamment choisis parmi CN, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, SF₅,
le ou les substituants sur R¹ et R² étant indépendamment choisis parmi D, C₁ à C₈ alkyle partiellement fluoré, C₁ à C₈ alkyle perfluoré, CF₃, halogène, F, CN ou SF₅ ;
R³ et R⁴ étant indépendamment choisis parmi H, D, halogène, F, CN, C₁ à C₄ alkyle partiellement fluoré, C₁ à C₄ alkyle perfluoré, SF₅ ou CF₃ ;
R⁵ étant indépendamment choisi parmi H, D, halogène, F, CN, C₁ à C₈ alkyle partiellement fluoré, C₁ à C₈ alkyle perfluoré, CF₃, C₆ à C₃₀ aryle substitué ou non substitué ou C₂ à C₃₀ hétéroaryle substitué ou non substitué, SF₅, le ou les substituants sur R⁵ étant indépendamment choisis parmi D, C₁ à C₈ alkyle partiellement fluoré ou C₁ à C₈ alkyle perfluoré, CF₃, halogène, F, CN, SF₅ ;
X^{a1} étant choisi parmi N ou CR^{a4} ;
R^{a1}, R^{a2}, R^{a3} et R^{a4} étant indépendamment choisis parmi H, D, C₁ à C₈ alkyle substitué ou non substitué ; C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, CN, SF₃,
le ou les substituants sur R^{a1}, R^{a2}, R^{a3} et R^{a4} étant indépendamment choisis parmi D, C₁ à C₈ alkyle partiellement fluoré ou C₁ à C₈ alkyle perfluoré, CF₃, halogène, F, CN, SF₅ ;
au moins l'un parmi R^{a1}, R^{a2}, R^{a3} et R^{a4} étant choisi parmi C₁ à C₈ alkyle partiellement fluoré, C₁ à C₈ alkyle perfluoré, CF₃, halogène, F, CN, ou SF₅.

2. Composé de formule (I) selon la revendication 1, R¹ et R² étant indépendamment choisis parmi CN, C₁ à C₄ alkyle partiellement fluoré, C₁ à C₄ alkyle perfluoré, CF₃, C₆ à C₁₀ aryle substitué ou non substitué, ou C₃ à C₉ hétéroaryle substitué ou non substitué ; préférablement R¹ et R² étant indépendamment choisis parmi CN, C₁ à C₄ alkyle partiellement fluoré, C₁ à C₄ alkyle perfluoré, CF₃, C₆ aryle substitué ou non substitué, C₃ à C₅ hétéroaryle substitué ou non substitué ; et de manière en outre préférée R¹ et R² étant indépendamment choisis parmi CN, C₁ à C₄ alkyle partiellement fluoré, C₁ à C₄ alkyle perfluoré, CF₃, C₆ aryle substitué ou non substitué, C₄ à C₅ hétéroaryle substitué ou non substitué.

3. Composé de formule (I) selon la revendication 1 ou 2, le ou les substituants sur R¹ et R² étant indépendamment choisis parmi D, C₁ à C₈ alkyle partiellement fluoré, C₁ à C₈ alkyle perfluoré, CF₃, ou CN ; préférablement le ou les substituants sur R¹ et R² étant indépendamment choisis parmi D, C₁ à C₄ alkyle partiellement fluoré, C₁ à C₄ alkyle perfluoré, CF₃, ou CN ; de manière en outre préférée le ou les substituants sur R¹ et R² étant indépendamment choisis parmi D, CF₃, CN.

4. Composé de formule (I) selon l'une quelconque des revendications précédentes 1 à 3, A¹ et A² étant choisis parmi la formule (V) : 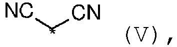 l'astérisque « * » désignant la position de liaison.

5. Composé de formule (I) selon l'une quelconque des revendications précédentes 1 à 4, R³ et R⁴ étant indépendamment choisis parmi H, D, CN, C₁ à C₄ alkyle partiellement fluoré, C₁ à C₄ alkyle perfluoré, ou CF₃, préférablement R³ et R⁴ étant indépendamment choisis parmi H, D, CN, CF₃, et de manière en outre préférée R³ et R⁴ étant indépendamment choisis parmi H, D.

6. Composé de formule (I) selon l'une quelconque des revendications précédentes 1 à 5, R⁵ étant choisi parmi H, D, CN, CF₃, C₆ aryle substitué ou non substitué, C₄ à C₅ hétéroaryle substitué ou non substitué ; préférablement R⁵ étant choisi parmi H, D, CN, C₆ aryle substitué ou non substitué ou C₄ à C₅ hétéroaryle substitué ou non substitué ; ou de manière en outre préférée R⁵ étant choisi parmi C₆ aryle substitué ou non substitué ou C₄ à C₅ hétéroaryle substitué ou non substitué.

7. Composé de formule (I) selon l'une quelconque des revendications précédentes 1 à 6, le ou les substituants sur R⁵ étant indépendamment choisis parmi D, C₁ à C₄ alkyle partiellement fluoré, C₁ à C₄ alkyle perfluoré, CF₃, halogène, F, ou CN ; préférablement le ou les substituants sur R⁵ étant indépendamment choisis parmi D, C₁ à C₄ alkyle partiellement fluoré, C₁ à C₄ alkyle perfluoré, CF₃, ou CN ; et de manière en outre préférée le ou les substituants sur R⁵ étant indépendamment choisis parmi D, CF₃, ou CN.

8. Composé de formule (I) selon l'une quelconque des revendications précédentes 1 à 7, un groupe CN n'étant pas lié à un atome de carbone aromatique lorsque ledit carbone aromatique est lié directement à un atome d'azote de cycle hybridé sp2.

9. Composé de formule (I) selon l'une quelconque des revendications précédentes 1 à 8, R⁵ étant choisi parmi la formule (VIa) : 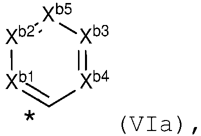 le « * » désignant la position de liaison, et
X^{b1} étant choisi parmi N ou CR^{b1},
X^{b2} étant choisi parmi N ou CR^{b2},
X^{b3} étant choisi parmi N ou CR^{b3},
X^{b4} étant choisi parmi N ou CR^{b4},
X^{b5} étant choisi parmi N ou CR^{b5},
R^{b1} à R^{b5} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, ou CN,
le ou les substituants sur R^{b1} à R^{b5} étant indépendamment choisis parmi D, C₁ à C₈ alkyle partiellement fluoré, C₁ à C₈ alkyle perfluoré, CF₃, halogène, F, ou CN ;
préférablement au moins l'un parmi R^{b1} à R^{b5} étant choisi parmi C₁ à C₈ alkyle partiellement fluoré ou perfluoré, CF₃, halogène, F, ou CN ; et de manière plus préférée au moins l'un parmi R^{b1} à R^{b5} étant choisi parmi C₁ à C₈ alkyle partiellement fluoré ou perfluoré, CF₃, ou CN.

10. Composé de formule (I) selon l'une quelconque des revendications précédentes 1 à 9, lorsque R^{bn} est N avec n=1 à 5 alors le R^{bm} adjacent avec m=1 à 5 n'est pas un groupe CN.

11. Composé de formule (I) selon l'une quelconque des revendications précédentes 1 à 10, R⁵ étant choisi parmi la formule (VIb) : 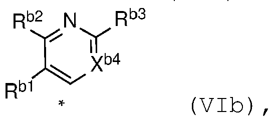 le « * » désignant la position de liaison, et
X^{b4} étant choisi parmi N ou CR^{b4},
R^{b1} à R^{b3} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué ou C₂ à C₃₀ hétéroaryle substitué ou non substitué, halogène, F, CN, le ou les substituants sur R^{b1} à R^{b3} étant indépendamment choisis parmi D, C₁ à C₈ alkyle partiellement fluoré, C₁ à C₈ alkyle perfluoré, CF₃, halogène, F, CN,
préférablement au moins l'un parmi R^{b1} à R^{b3} étant choisi parmi C₁ à C₈ alkyle partiellement fluoré ou perfluoré, CF₃, halogène, F, ou CN ; et plus préférablement au moins l'un parmi R^{b1} à R^{b3} étant choisi parmi C₁ à C₈ alkyle partiellement fluoré ou perfluoré, CF₃, ou CN.

12. Composé de formule (I) selon l'une quelconque des revendications précédentes 1 à 11, R⁵ étant choisi parmi la formule (VIc) : 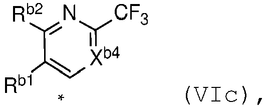 le « * » désignant la position de liaison, et
X^{b4} étant choisi parmi N ou CR^{b4},
R^{b1} et R^{b2} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, CN,
le ou les substituants sur R^{b1} et R^{b2} étant indépendamment choisis parmi D, C₁ à C₈ alkyle partiellement fluoré, C₁ à C₈ alkyle perfluoré, CF₃, halogène, F, CN,
préférablement au moins l'un parmi R^{b1} et R^{b2} étant choisi parmi C₁ à C₈ alkyle partiellement fluoré ou perfluoré, CF₃, halogène, F, ou CN ; et plus préférablement au moins l'un parmi R^{b1} et R^{b2} étant choisi parmi C₁ à C₈ alkyle partiellement fluoré ou perfluoré, CF₃, ou CN.

13. Composé de formule (I) selon l'une quelconque des revendications précédentes 1 à 12, R⁵ étant choisi parmi la formule (VId) : 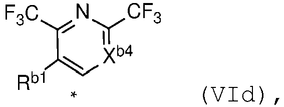 le « * » désignant la position de liaison, et
X^{b4} étant choisi parmi N ou CR^{b4},
R^{b1} étant indépendamment choisi parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, CN,
le ou les substituants sur R^{b1} étant indépendamment choisi parmi D, C₁ à C₈ alkyle partiellement fluoré, C₁ à C₈ alkyle perfluoré, CF₃, halogène, F, CN,
préférablement R^{b1} étant choisi parmi C₁ à C₈ alkyle partiellement fluoré ou perfluoré, CF₃, halogène, F, ou CN ; et plus préférablement R^{b1} étant choisi C₁ à C₈ alkyle partiellement fluoré ou perfluoré, CF₃, ou CN.

14. Composé de formule (I) selon l'une quelconque des revendications précédentes 1 à 13, R⁵ étant choisi parmi la formule (VIe) : 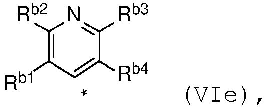 le « * » désignant la position de liaison, et
R^{b1} à R^{b4} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué ou C₂ à C₃₀ hétéroaryle substitué ou non substitué, halogène, F, CN, le ou les substituants sur R^{b1} à R^{b4} étant indépendamment choisis parmi D, C₁ à C₈ alkyle partiellement fluoré, C₁ à C₈ alkyle perfluoré, CF₃, halogène, F, CN,
préférablement au moins l'un parmi R^{b1} à R^{b4} étant choisis parmi C₁ à C₈ alkyle partiellement fluoré ou perfluoré, CF₃, halogène, F, ou CN ; et plus préférablement au moins l'un parmi R^{b1} à R^{b4} étant choisis parmi C₁ à C₈ alkyle partiellement fluoré ou perfluoré, CF₃, ou CN.

15. Composé de formule (I) selon l'une quelconque des revendications précédentes 1 à 14, R⁵ étant choisi parmi la formule (VIf) : 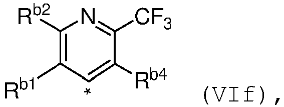 le « * » désignant la position de liaison, et
R^{b1}, R^{b2} et R^{b4} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, CN,
le ou les substituants sur R^{b1}, R^{b2} et R^{b4} étant indépendamment choisis parmi D, C₁ à C₈ alkyle partiellement fluoré, C₁ à C₈ alkyle perfluoré, CF₃, halogène, F, CN,
préférablement au moins l'un parmi R^{b1}, R^{b2} et R^{b4} étant choisis parmi C₁ à C₈ alkyle partiellement fluoré ou perfluoré, CF₃, halogène, F, ou CN ; et plus préférablement au moins l'un parmi R^{b1} à R^{b4} étant choisi parmi C₁ à C₈ alkyle partiellement fluoré ou perfluoré, CF₃, ou CN.

16. Composé de formule (I) selon l'une quelconque des revendications précédentes 1 à 15, R⁵ étant choisi parmi la formule (VIg) : 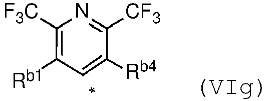 le « * » désignant la position de liaison, et
R^{b1} et R^{b4} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, halogène, F, CN,
le ou les substituants sur R^{b1} et R^{b4} étant indépendamment choisis parmi D, C₁ à C₈ alkyle partiellement fluoré, C₁ à C₈ alkyle perfluoré, CF₃, halogène, F, CN,
préférablement au moins l'un parmi R^{b1} et R^{b4} étant choisi parmi C₁ à C₈ alkyle partiellement fluoré ou perfluoré, CF₃, halogène, F, ou CN ; et plus préférablement au moins l'un parmi R^{b1} et R^{b4} étant choisis parmi C₁ à C₈ alkyle partiellement fluoré ou perfluoré, CF₃, ou CN.

17. Composé de formule (I) selon l'une quelconque des revendications précédentes 1 à 16, R^{a1} à R^{a4} dans la formule I, R^{b1} à R^{b5} dans la formule (VIa), R^{b1} à R^{b3} dans la formule (VIb), R^{b1} à R^{b2} dans la formule (VIc), R^{b1} dans la formule (VId), R^{b1} à R^{b4} dans la formule (Vie), R^{b1}, R^{b2} et R^{b4} dans la formule (VIf), R^{b1} et R^{b4} dans la formule (VIg) n'étant pas choisis parmi halogène ou F ; et préférablement R^{a1} à R^{a4} dans la formule I, R^{b1} à R^{b5} dans la formule (VIa), R^{b1} à R^{b3} dans la formule (VIb), R^{b1} à R^{b2} dans la formule (VIc), R^{b1} dans la formule (VId), R^{b1} à R^{b4} dans la formule (Vie), R^{b1}, R^{b2} et R^{b4} dans la formule (VIf), R^{b1} et R^{b4} dans la formule (VIg) n'étant pas choisis parmi halogène ou F, et le ou les substituants sur R^{a1} à R^{a4}, R^{b1} à R^{b5} n'étant pas choisis parmi halogène ou F.

18. Composé de formule (I) selon l'une quelconque des revendications précédentes 1 à 17 :
R^{a2} à R^{a3} dans la formule (II), R^{b2} à R^{b3} dans la formule (VIa), (VIb), (VIe), et R^{b2} dans la formule (VIc) et (VIf) n'étant pas choisis parmi CN.

19. Composé de formule (I) selon l'une quelconque des revendications précédentes 1 à 18, R⁵ étant choisi dans un groupe représenté par B1 à B160 :
| | | | |
|---|---|---|---|
| 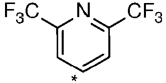 | 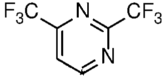 | 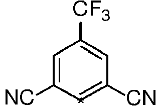 | 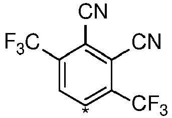 |
| B1 | B2 | B3 | B4 |
| 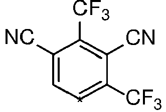 | 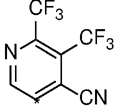 | 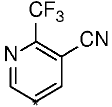 | 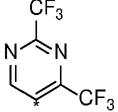 |
| B5 | B6 | B7 | B8 |
| 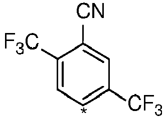 | 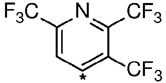 | 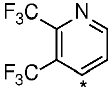 | 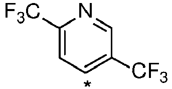 |
| B9 | B10 | B11 | B12 |
| 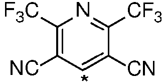 | 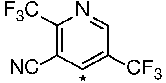 | 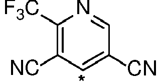 | 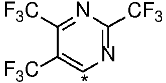 |
| B13 | B14 | B15 | B16 |
| 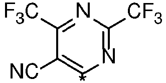 | 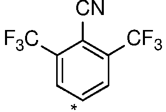 | 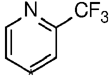 | 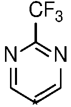 |
| B17 | B18 | B19 | B20 |
| 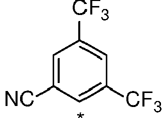 | 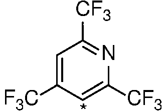 | 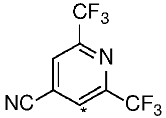 | 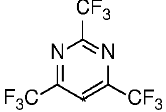 |
| B21 | B22 | B23 | B24 |
| 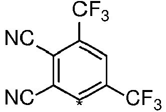 | 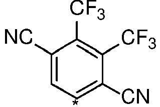 | 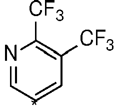 | 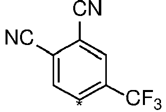 |
| B25 | B26 | B27 | B28 |
| 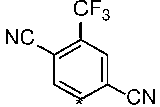 | 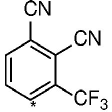 | 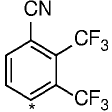 | 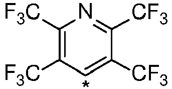 |
| B29 | B30 | B31 | B32 |
| 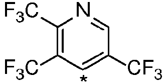 | 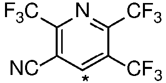 | 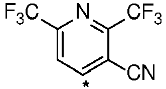 | 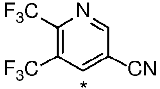 |
| B33 | B34 | B35 | B36 |
| 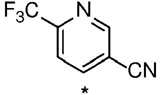 | 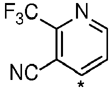 | 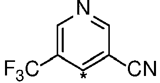 | 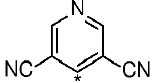 |
| B37 | B38 | B39 | B40 |
| 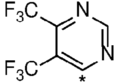 | 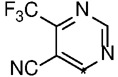 | 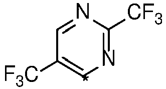 | 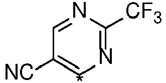 |
| B41 | B42 | B43 | B44 |
| 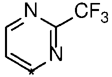 | 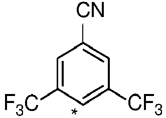 | 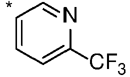 | 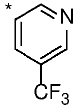 |
| B45 | B46 | B47 | B48 |
| 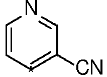 | 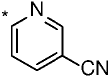 | 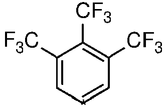 | 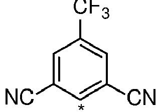 |
| B49 | B50 | B51 | B52 |
| 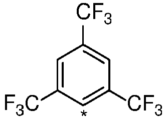 | 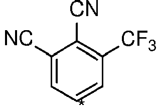 | 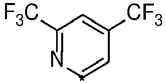 | 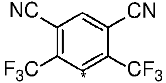 |
| B53 | B54 | B55 | B56 |
| 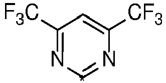 | 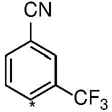 | 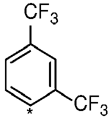 | 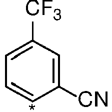 |
| B57 | B58 | B59 | B60 |
| 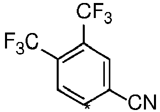 | 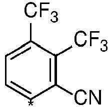 | 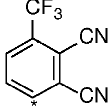 | 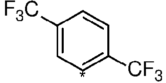 |
| B61 | B62 | B63 | B64 |
| 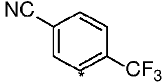 | 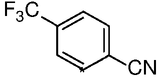 | 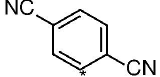 | 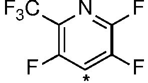 |
| B65 | B66 | B67 | B68 |
| 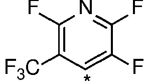 | 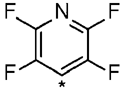 | 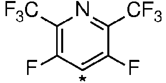 | 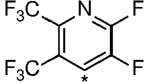 |
| B69 | B70 | N71 | N72 |
| 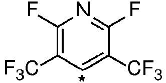 | 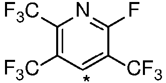 | 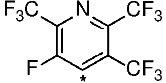 | 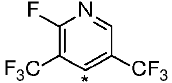 |
| B73 | B74 | B75 | B76 |
| 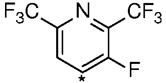 | 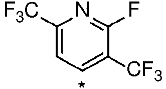 | 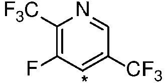 | 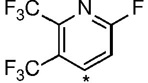 |
| B77 | B78 | B79 | B80 |
| 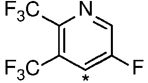 | 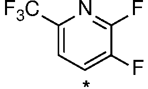 | 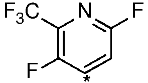 | 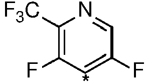 |
| B81 | B82 | B83 | B84 |
| 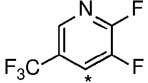 | 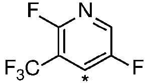 | 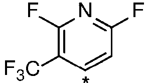 | 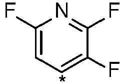 |
| B85 | B86 | B87 | B88 |
| 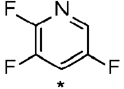 | 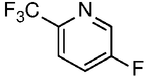 | 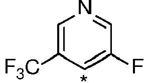 | 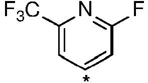 |
| B89 | B90 | B91 | B92 |
| 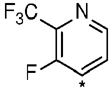 | 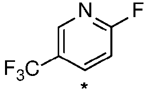 | 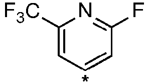 | 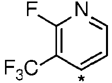 |
| B93 | B94 | B95 | B96 |
| 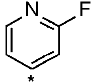 | 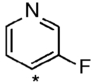 | 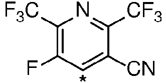 | 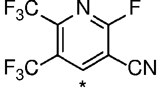 |
| B97 | B98 | B99 | B100 |
| 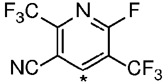 | 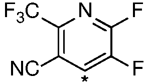 | 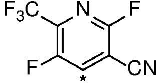 | 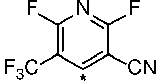 |
| B101 | B102 | B103 | B104 |
| 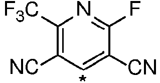 | 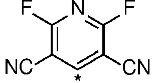 | 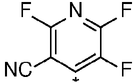 | 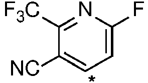 |
| B105 | B106 | B107 | B108 |
| 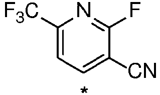 | 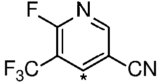 | 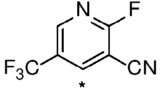 | 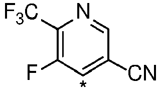 |
| B109 | B110 | B111 | B112 |
| 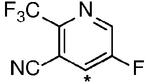 | 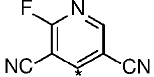 | 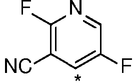 | 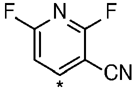 |
| B113 | B114 | B115 | B116 |
| 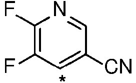 | 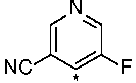 | 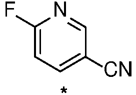 | 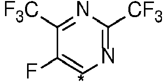 |
| B117 | B118 | B119 | B120 |
| 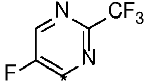 | 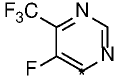 | 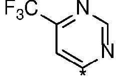 | 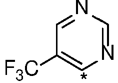 |
| B121 | B122 | B123 | B124 |
| 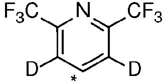 | 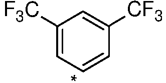 | 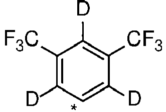 | 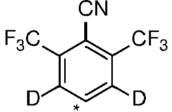 |
| B125 | B126 | B127 | B128 |
| 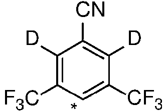 | 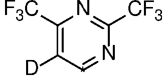 | 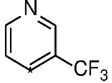 | 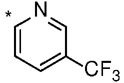 |
| B129 | B130 | B131 | B132 |
| 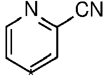 | 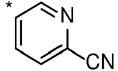 | 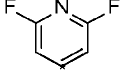 | 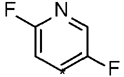 |
| B133 | B134 | B135 | B136 |
| 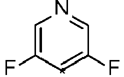 | 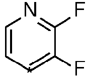 | 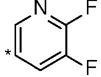 | 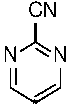 |
| B137 | B138 | B139 | B140 |
| 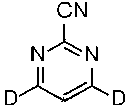 | 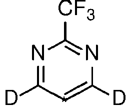 | 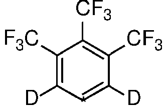 | 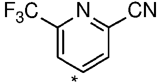 |
| B141 | B142 | B143 | B144 |
| 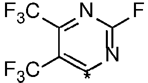 | 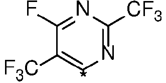 | 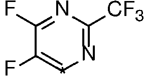 | 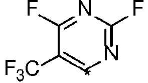 |
| B145 | B146 | B147 | B148 |
| 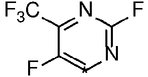 | 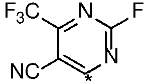 | 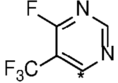 | 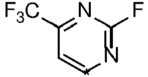 |
| B149 | B150 | B151 | B152 |
| 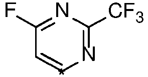 | 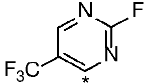 | 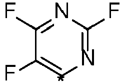 | 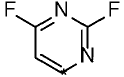 |
| B153 | B154 | B155 | B156 |
| 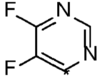 | 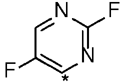 | 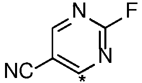 | 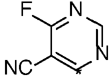 |
| B157 | B158 | B159 | B160 |
, le « * » désignant la position de liaison.

20. Composé de formule (I) selon l'une quelconque des revendications précédentes 1 à 19, la formule (II) étant choisie parmi les formules (IIa), (IIb), (IIe), (IId), (IIe), et (IIf) : 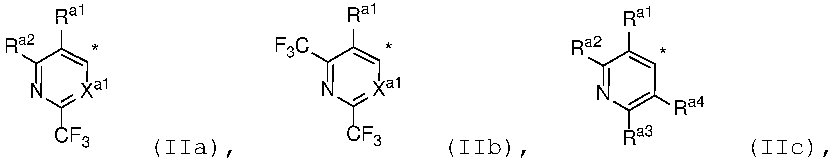 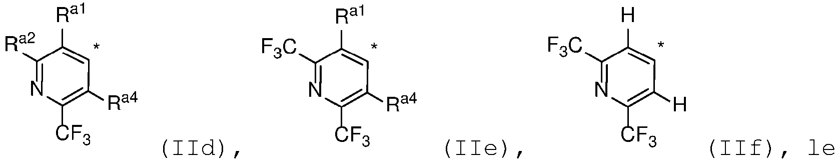 « * » désignant la position de liaison, et préférablement les formules (IIa), (IIb), (IIc), (IId), (IIe), et (IIf) étant partiellement deutérées ou entièrement deutérées, et de manière en outre préférée la formule (IIf) étant partiellement deutérée ou entièrement deutérée.

21. Composé de formule (I) selon l'une quelconque des revendications précédentes 1 à 20, HetAr étant choisi dans un groupe représenté par C1 à C109 :
| | | | |
|---|---|---|---|
| 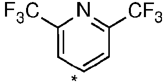 | 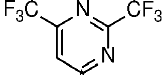 | 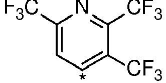 | 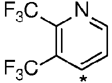 |
| C1 | C2 | C3 | C4 |
| 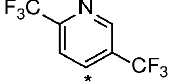 | 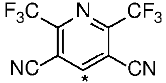 | 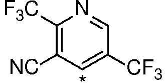 | 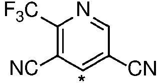 |
| 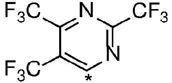 C5 | 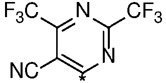 C6 | 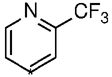 C7 | 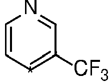 C8 |
| C9 | C10 | C11 | C12 |
| 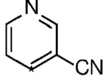 | 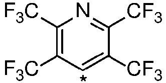 | 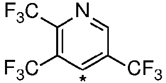 | 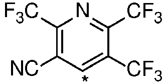 |
| C13 | C14 | C15 | C16 |
| 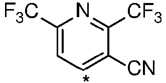 | 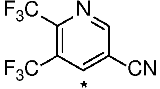 | 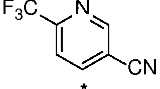 | 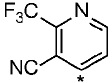 |
| C17 | C18 | C19 | C20 |
| 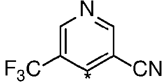 | 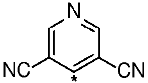 | 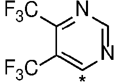 | 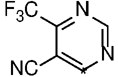 |
| C21 | C22 | C23 | C24 |
| 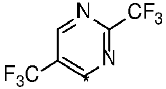 | 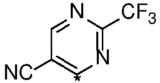 | 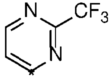 | 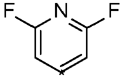 |
| C25 | C26 | C27 | C28 |
| 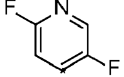 | 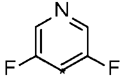 | 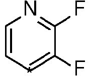 | 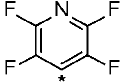 |
| C29 | C30 | C31 | C32 |
| 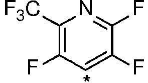 | 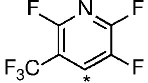 | 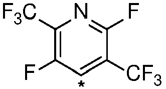 | 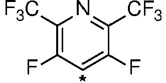 |
| C33 | C34 | C35 | C36 |
| 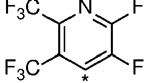 | 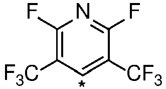 | 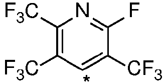 | 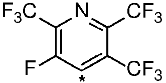 |
| C37 | C38 | C39 | C40 |
| 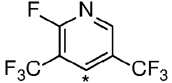 | 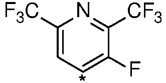 | 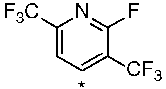 | 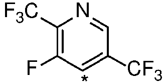 |
| C41 | C42 | C43 | C44 |
| 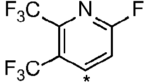 | 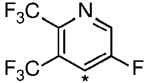 | 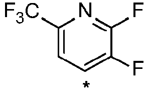 | 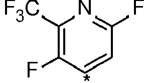 |
| C45 | C46 | C47 | C48 |
| 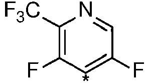 | 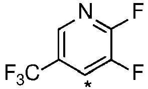 | 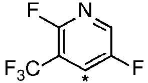 | 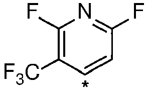 |
| C49 | C50 | C51 | C52 |
| 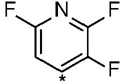 | 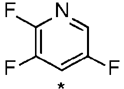 | 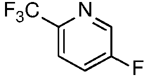 | 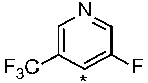 |
| C53 | C54 | C55 | C56 |
| 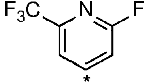 | 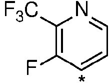 | 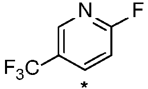 | 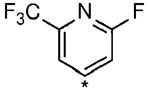 |
| C57 | C58 | C59 | C60 |
| 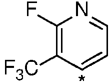 | 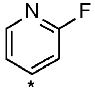 | 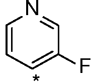 | 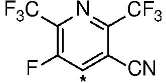 |
| C61 | C62 | C63 | C64 |
| 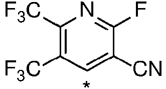 | 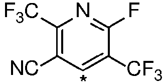 | 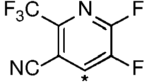 | 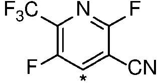 |
| C65 | C66 | C67 | C68 |
| 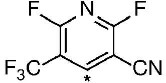 | 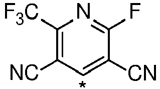 | 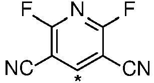 | 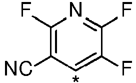 |
| C69 | C70 | C71 | C72 |
| 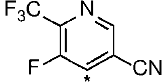 | 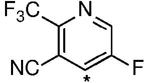 | 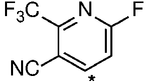 | 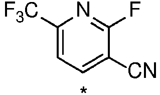 |
| C73 | C74 | C75 | C76 |
| 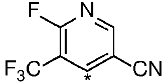 | 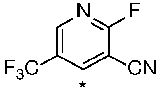 | 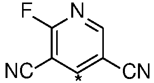 | 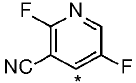 |
| C77 | C78 | C79 | 80C |
| 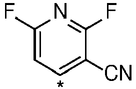 | 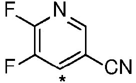 | 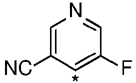 | 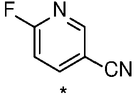 |
| C81 | C82 | C83 | C84 |
| 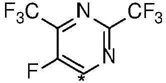 | 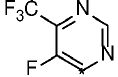 | 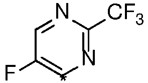 | 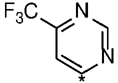 |
| C85 | C86 | C87 | C88 |
| 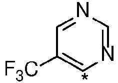 | 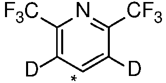 | 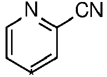 | 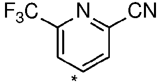 |
| C89 | C90 | C91 | C92 |
| 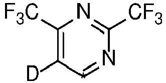 | 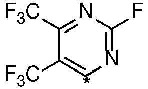 | 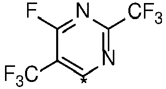 | 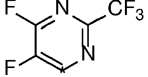 |
| C93 | C94 | C95 | C96 |
| 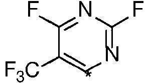 | 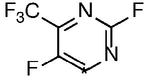 | 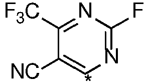 | 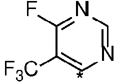 |
| C97 | C98 | C99 | C100 |
| 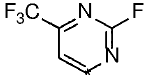 | 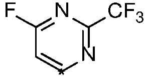 | 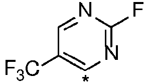 | 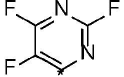 |
| C101 | C102 | C103 | C104 |
| 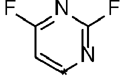 | 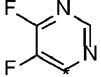 | 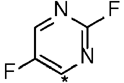 | 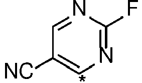 |
| C105 | C106 | C107 | C108 |
| 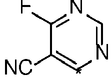 | | | |
| C109 | | | |
, le « * » désignant la position de liaison.

22. Composé de formule (I) selon l'une quelconque des revendications précédentes 1 à 21, le composé de formule (I) étant choisi parmi un composé de formule (VII) : 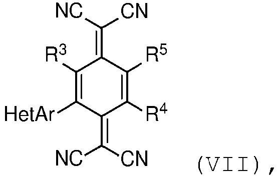
HetAr étant choisi parmi les formules (II), (IIa), (IIb), (IIc), (IId), (IIe), et (IIf) : 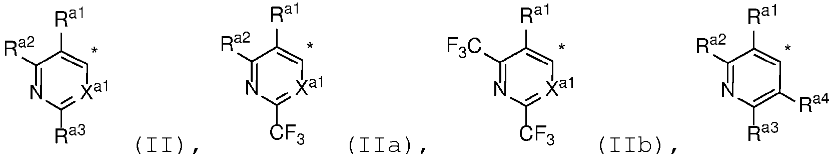 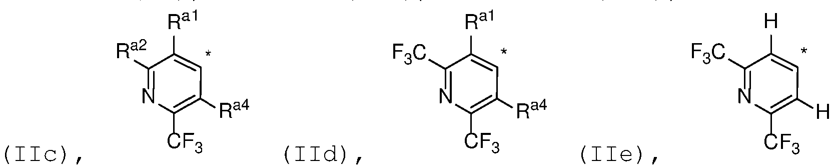
(IIf), l'astérisque « * »
désignant la position de liaison, et
R³ et R⁴ étant choisis parmi H, D ;
X^{a1} étant choisi parmi N ou CR^{a4} ;
R^{a1} à R^{a4} étant indépendamment choisis parmi H, D, C₁ à C₈ alkyle substitué ou non substitué ; C₁ à C₈ alkyle partiellement fluoré ou C₁ à C₈ alkyle perfluoré, CF₃, C₆ à C₃₀ aryle substitué ou non substitué, C₂ à C₃₀ hétéroaryle substitué ou non substitué, halogène, F, ou CN,
le ou les substituants sur R^{a1} à R^{a4} étant indépendamment choisis parmi D, C₁ à C₈ alkyle partiellement fluoré ou C₁ à C₈ alkyle perfluoré, CF₃, CN ;
au moins l'un parmi R^{a1} à R^{a4} étant choisi parmi C₁ à C₈ alkyle partiellement fluoré ou C₁ à C₈ alkyle perfluoré, CF₃, ou CN ;
R⁵ étant choisi parmi H, D, CN, C₁ à C₈ alkyle partiellement fluoré ou C₁ à C₈ alkyle perfluoré, CF₃, C₆ à C₃₀ aryle substitué ou non substitué, C₂ à C₃₀ hétéroaryle substitué ou non substitué,
le ou les substituants sur R⁵ étant indépendamment choisis parmi D, C₁ à C₈ alkyle partiellement fluoré ou C₁ à C₈ alkyle perfluoré, CF₃, ou CN.

23. Composé de formule (I) selon l'une quelconque des revendications précédentes 1 à 22, le composé de formule (I) étant choisi parmi un composé de formule (VII) : 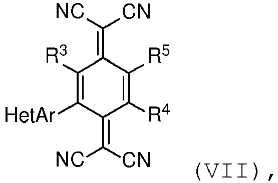
HetAr étant choisi parmi les formules (II), (IIa), (IIb), (IIc), (IId), (IIe), et (IIf) : 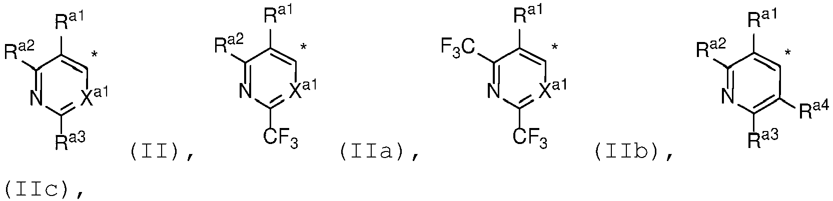 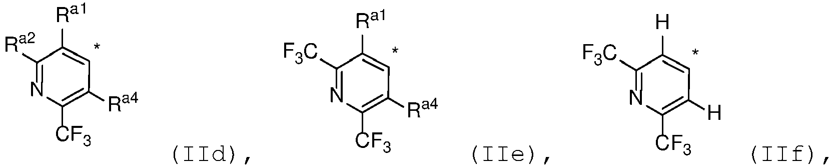
l'astérisque « * » désignant la position de liaison, et R³ et R⁴ étant choisis parmi H, D ;
X^{a1} étant choisi parmi N ou CR^{a4} ;
R^{a1} à R^{a4} étant indépendamment choisis parmi H, D, C₁ à C₈ alkyle substitué ou non substitué ; C₁ à C₈ alkyle partiellement fluoré ou C₁ à C₈ alkyle perfluoré, CF₃, C₆ à C₃₀ aryle substitué ou non substitué, C₂ à C₃₀ hétéroaryle substitué ou non substitué, halogène, F, ou CN,
le ou les substituants sur R^{a1} à R^{a4} étant indépendamment choisis parmi D, C₁ à C₈ alkyle partiellement fluoré ou C₁ à C₈ alkyle perfluoré, CF₃, CN ;
au moins l'un parmi R^{a1} à R^{a4} étant choisi parmi C₁ à C₈ alkyle partiellement fluoré ou C₁ à C₈ alkyle perfluoré, CF₃, ou CN ;
R⁵ étant choisi parmi la formule (VIa) 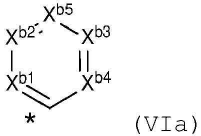
X^{b1} étant choisi parmi N ou CR^{b1},
X^{b2} étant choisi parmi N ou CR^{b2},
X^{b3} étant choisi parmi N ou CR^{b3},
X^{b4} étant choisi parmi N ou CR^{b4},
X^{b5} étant choisi parmi N ou CR^{b5},
R^{b1} à R^{b5} étant indépendamment choisis parmi H, D, C₁ à C₈ alkyle substitué ou non substitué, C₁ à C₈ alkyle partiellement fluoré, C₁ à C₈ alkyle perfluoré, CF₃, C₆ à C₃₀ aryle substitué ou non substitué, C₂ à C₃₀ hétéroaryle substitué ou non substitué, ou CN,
le ou les substituants sur R^{b1} à R^{b5} étant indépendamment choisis parmi D, C₁ à C₈ alkyle partiellement fluoré, C₁ à C₈ alkyle perfluoré, CF₃, ou CN,
et l'astérisque « * » désignant la position de liaison.

24. Composé de formule (I) selon l'une quelconque des revendications précédentes 1 à 23, le composé de formule (I) étant choisi parmi un composé représenté par I-1 à I-40:
| | | |
|---|---|---|
| 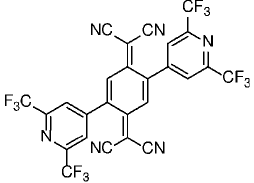 | 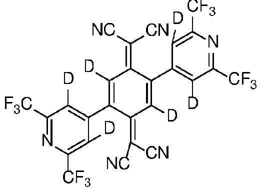 | 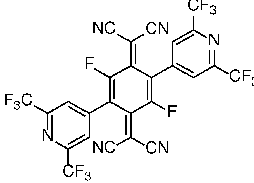 3 |
| I-1 | I-2 | I-3 |
| 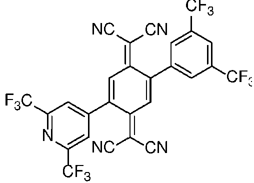 | 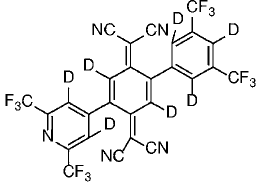 | 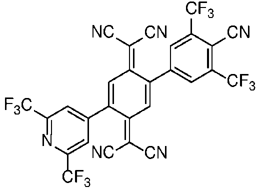 |
| I-4 | I-5 | I-6 |
| 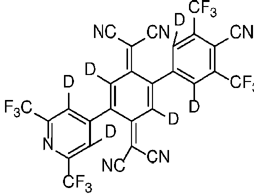 | 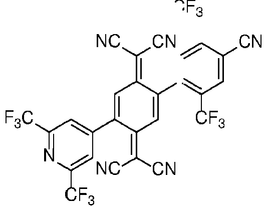 | 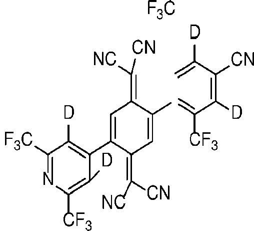 |
| I-7 | I-8 | I-9 |
| 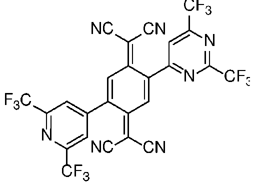 | 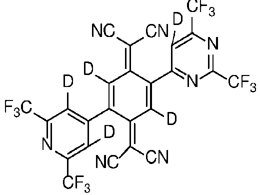 | 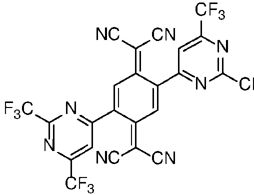 |
| I-10 | I-11 | I-12 |
| 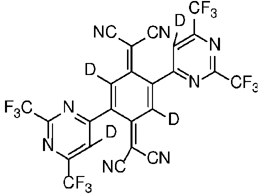 | 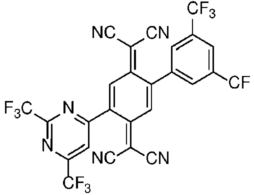 | 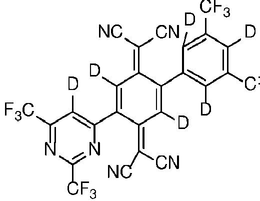 |
| I-13 | I-14 | I-15 |
| 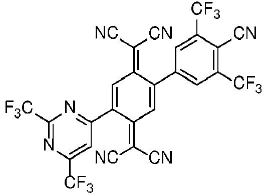 | 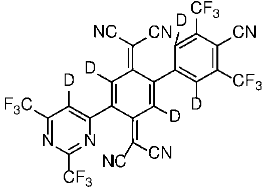 | 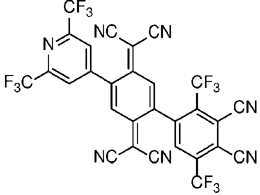 |
| I-16 | I-17 | I-18 |
| 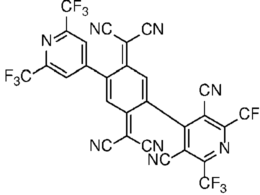 | 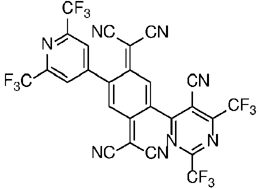 | 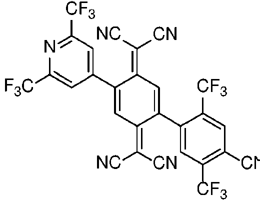 |
| I-19 | I-20 | I-21 |
| 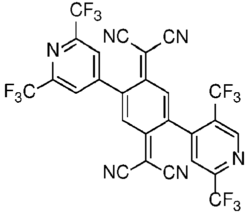 | 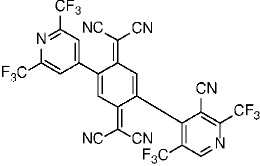 | 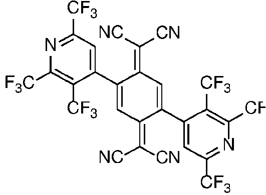 |
| I-22 | I-23 | I-24 |
| 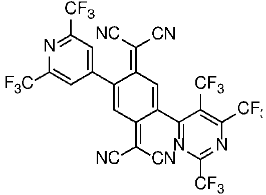 | 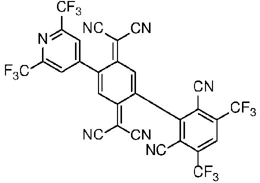 | 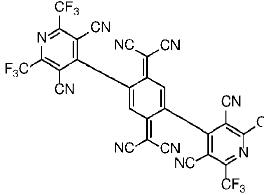 |
| I-25 | I-26 | I-27 |
| 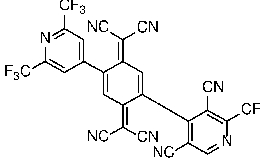 | 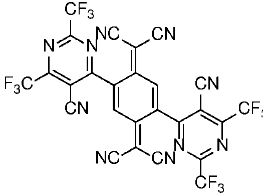 | 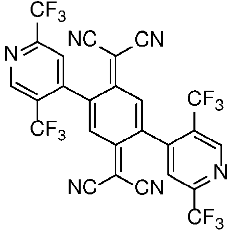 |
| I-28 | I-29 | I-30 |
| 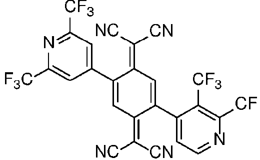 | 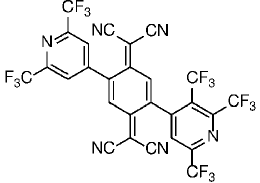 | 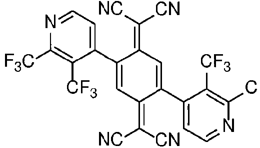 |
| I-32 | I-33 | I-34 |
| 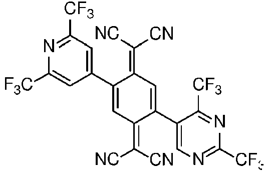 | 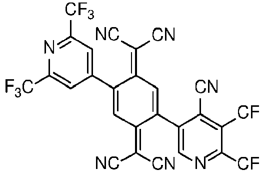 | 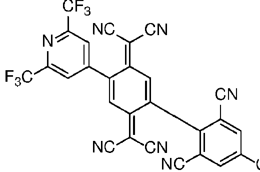 |
| I-35 | I-36 | I-37 |
| 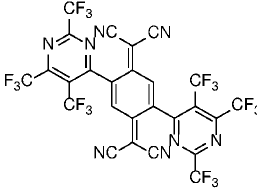 | 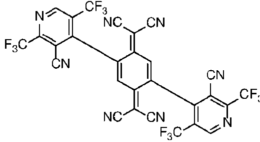 | 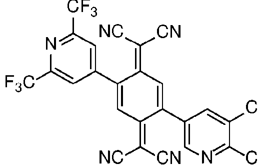 |
| I-38 | I-39 | 1-40. |

25. Couche de semi-conducteur organique, dans laquelle la couche de semi-conducteur organique comprend un composé de formule (I) selon les revendications 1 à 24, et de préférence le composé de formule (VII).

26. Couche de semi-conducteur organique selon la revendication 25, dans laquelle la couche de semi-conducteur organique comprend le composé de formule (I), de préférence le composé de formule (VII), et un composé de matrice de transport de trous.

27. Couche de semi-conducteur organique selon la revendication 25 ou 26, dans laquelle la couche de semi-conducteur organique est une couche d'injection de trous ou une couche de génération de charge de type p.

28. Dispositif électronique organique, dans lequel le dispositif électronique organique comprend une couche de semi-conducteur organique selon les revendications 25 à 27.

29. Dispositif électronique organique selon la revendication 28, dans lequel le dispositif électronique organique comprend une couche d'anode, une couche de cathode, une première couche photoactive, une seconde couche photoactive, une couche d'injection de trous, une couche de génération de charge de type p, dans lequel la couche d'injection de trous est agencée en contact direct avec la couche d'anode, et dans lequel la couche de génération de charge de type p est agencée entre la première couche photoactive et la seconde couche photoactive, dans lequel la couche d'injection de trous est plus proche de la couche d'anode que la couche de génération de charge de type p, dans lequel la couche d'injection de trous ou la couche de génération de charge de type p est une couche de semi-conducteur organique selon les revendications 25 à 27, dans lequel la première couche photoactive, la seconde couche photoactive, la couche d'injection de trous, la couche de génération de charge de type p sont agencées entre la couche d'anode et la couche de cathode.

30. Dispositif d'affichage comprenant un dispositif électronique organique selon la revendication 28 ou 29.
